# EUROPEAN PATENT APPLICATION

(11) **EP 0 976 703 A1**
(43) Date of publication of application: **02.02.2000**
(21) Application number: 98900685.3
(22) Date of filing: 21.01.1998
(51) Int. Cl.: C07C 13/28, C07C 23/18, C07D 309/04, C07D 317/42, C07D 319/06, C07D 339/08, C07D 405/04, C07D 405/06, C07D 405/08, C07D 405/10, C07D 405/12, C07D 405/14, C07D 407/04, C07D 407/10, C07D 409/04, C07D 411/04, C09K 19/34, C09K 19/42, G02F 1/13

(54) **LIQUID-CRYSTAL COMPOUNDS, LIQUID-CRYSTAL COMPOSITION CONTAINING THE SAME, AND LIQUID-CRYSTAL DISPLAY ELEMENT CONTAINING THE SAME**

(30) Priority: 22.01.1997 JP 963797
(71) Applicant: CHISSO CORPORATION, Osaka-shi Osaka 530-6591 (JP)
(72) Inventor: HASEBA, Yasuhiro, Matsudo-shi,Chiba 270 (JP); MATSUI, Shuichi, Ichihara-shi,Chiba 290 (JP); MIYAZAWA, Kazutoshi, Ichihara-shi,Chiba 290-01 (JP); HACHIYA, Norihisa, Ichihara-shi,Chiba 299-01 (JP); NAKAGAWA, Etsuo, Ichihara-shi,Chiba 290 (JP)
(74) Representative: HOFFMANN - EITLE
(86) International application number: JP9800222
(87) International publication number: WO9832721

(57) **Abstract**

There can be provided a liquid crystalline compound expressed by the general formula (1): 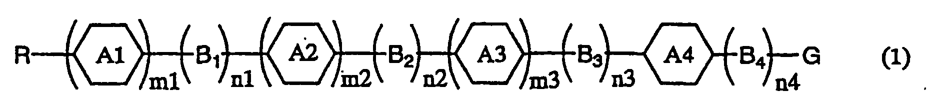 wherein R represents a hydrogen atom, a cyano group, a halogen atom, a C₁ to C₂₀ alkyl group or a halogenated alkyl group, B1, B2, B3 and B4 are independently a covalent bond or a C₁ to C₄ alkylene group, n1, n2, n3, n4, m1, m2 and m3 are independently 0 or 1, rings A1, A2, A3 and A4 are independently a benzene ring in which one to four hydrogen atoms may be replaced by halogen atoms, bicyclo[1.1.1]pentane ring, bicyclo[2.1.1]hexane ring, bicyclo[2.2.1]heptane ring, bicyclo[2.2.2]octane ring, naphthalene ring, 1,2,3,4-tetrahydronaphthalene ring, perhydronaphthalene ring, fluorene ring, phenanthrene ring, 9,10-dihydrophenanthrene ring, indan ring, indene ring, pyridine ring, pyrimidine ring, C₄ to C₂₀ cycloalkane ring or cycloalkene ring,
G represents any one of groups represented by the formulae (G1) to (G3): 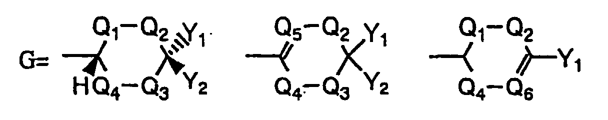 wherein Q₁, Q₂, Q₃ and Q₄ are independently a covalent bond, an oxygen atom, a sulfur atom, -CH₂-, -CH₂CH₂-, -CH=CH-, -CO-, -CF=CF- or -CF₂-, the number of covalent bonds is 2 or less, Q₁ and Q₄ are preferential, the oxygen atom and the sulfur atom are not adjacent to each other, Q₅ and Q₆ are independently =CH-, =CF- or =CCl-, Y₁ and Y₂ are independently a hydrogen atom, a halogen atom, a cyano group, a nitro group, an amino group, or a C₁ to C₂₀ alkyl group or halogenated alkyl group, as well as a liquid crystal composition comprising the same, and a liquid crystal display device fabricated by using the same, and these are used in various liquid crystal display devices including TFT type.

## Description

### TECHNICAL FIELD

The present invention relates to a liquid crystalline compound effective as a component in a liquid crystal composition, a liquid crystal composition comprising the same, and a liquid crystal display device fabricated by using said liquid crystal composition.

### BACKGROUND ART

The liquid crystal display device makes use of optical anisotropy and dielectric anisotropy possessed by liquid crystal substance. Depending on the display system, the liquid crystal display device is divided into various types such as TN type (twisted nematic type), DS type (dynamic scattering type), guest-host type, DAP type (deformation of aligned phase type), STN type (super twisted nematic type), and TFT type (thin film transistor type), but recently the 3 types of TN type, STN type and TFT type is mainly used. It is necessary that liquid crystal materials used in any display devices are stable to moisture, air, heat, light etc., show a liquid crystal phase in a temperature range as broad as possible around room temperature, are low viscous, have the optimum dielectric anisotropy value (Δε), have the optimum optical anisotropy value (Δn), and have the optimum elastic constant K and elastic constant ratio K33/K11 (K33: bend elastic constant, K11; splay elastic constant). At present, there is no substance which as a single compound, satisfies all such conditions, so under the present circumstances, a few to dozens of liquid crystalline compounds are mixed to meet prescribed properties. For preparation of liquid crystal composition, all these properties should be satisfied, so there are cases where the prescribed properties cannot be satisfied e.g. due to small Δε or large Δn.

At present, there is a demand for low power consumption in the liquid crystal display device. This demand can be solved by reducing Vth (threshold voltage) of liquid crystal material. At present, the effective means of reducing Vth is to develop a compound having larger Δε than that of the present-day conventional liquid crystalline compounds. As described on page 22 in the November Issue (1995) of a monthly magazine Display, fluorine type liquid crystalline compounds used most frequently in the present TFT type liquid crystal display are difluorobenzene derivatives having fluorine atoms introduced at the 3- and 4-positions in the benzene ring, and for example, mention can be made of the following compound (10) disclosed in Japanese Patent Publication No. 2-40048.

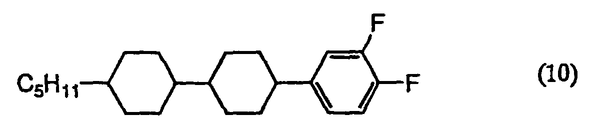

The Δε of this compound is about 5.

On the other hand, cyano type compounds are mainly used in the TN type and STN type liquid crystal displays. By way of example, mention can be made of compound (11) of the following formula:

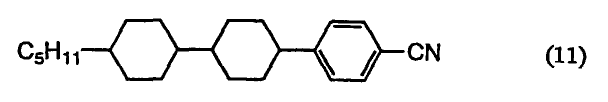

The Δε of this compound is about 9.

Accordingly, the development of partial skeletons capable of exhibiting larger Δε than that of the difluorophenyl group or cyanophenyl group is the point for solving the problem.

At present, the task of developing liquid crystalline compounds having large Δn can be solved by introducing benzene rings or a C≡C triple bonds into the compounds. However, in the case of the development of compounds having large absolute values of Δε, there are limits to the lower-limit values of Δn. This is because most compounds with large absolute values of As have electron attractive groups such as cyano group, fluorine atom, chlorine atom, trifluoromethyl group and trifluoromethoxy group bound to benzene rings inducing large Δn. Previously disclosed liquid crystalline compounds having electron attractive groups bound to a ring other than benzene ring are shown below.

Japanese Patent Publication Laid-open No. Hei 1-233239 discloses compounds (12) and (13) having chlorine atoms or fluorine atoms bound to a cyclohexane ring thereof.

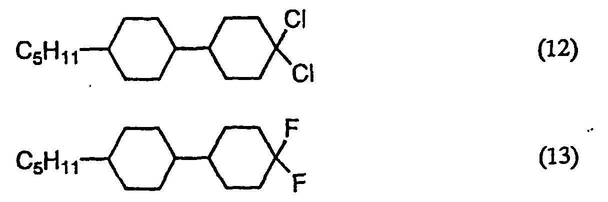

According to said Japanese patent publication laid-open, by merely adding 5 % by weight of compound (12) to 95 % by weight of the liquid crystal composition (A) having the following composition:

| | |
|---|---|
| 4-(4-propylcyclohexyl)benzonitrile | 24 % |
| 4-(4-pentylcyclohexyl)benzonitrile | 36 % |
| 4-(4-heptylcyclohexyl)benzonitrile | 25 % |
| 4-(4-pentylphenyl)benzonitrile | 15 % |

Δn is lowered but the clearing point (in this case, N-I transition paint) and Δε are greatly reduced while the viscosity is increased, or by merely adding 5 % by weight of compound (13) to 95 % by weight of the liquid crystal composition (A), the viscosity remains the same while Δn is lowered and the clearing point (N-I transition point) and Δε are greatly reduced.

As can be seen from the foregoing, compounds (12) and (13) lower not only Δn but also the clearing point (N-I transition point) and Δε.

At present, there is also a demand for low-temperature driving of liquid crystal display. Usually, cyclohexane rings are frequently used in compounds in low-Δn compositions, but such compositions are poor in miscibility at low temperature, thus easily precipitating the smectic phase or crystals.

From the foregoing, it has been desired to develop a liquid crystalline compound having small Δn and large Δε; a liquid crystalline compound having small Δn and a high clearing point; a liquid crystalline compound having small Δn and being superior in miscibility; or a liquid crystalline compound having middle Δn and large Δε.

### DISCLOSURE OF THE INVENTION

The object of the present invention is to develop a liquid crystalline compound having small Δn and large Δε, a liquid crystalline compound having small Δn and a high clearing point, a liquid crystalline compound haying small Δn and exhibiting the nematic phase even at low temperature, or a liquid crystalline compound having middle Δn and large Δε, which is necessary for solving the problem of the prior art described above, thereby providing a liquid crystal composition comprising the same as well as a liquid crystal display device fabricated by using the same.

To solve this problem, the inventions claimed in the present application are as follows:
(1) A liquid crystalline compound expressed by the general formula (1): 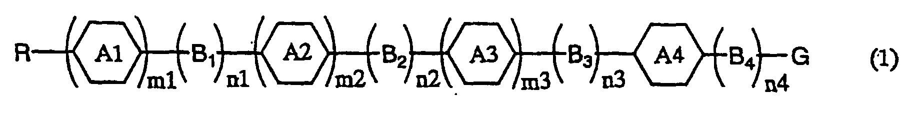 wherein R represents a hydrogen atom, a cyano group, a halogen atom, a C₁ to C₂₀ alkyl group or halogenated alkyl group, in which one or more -CH₂- groups may be replaced by an oxygen atom, a sulfur atom, -CH=CH-, -C≡C-, -CO-, -CF=CF-, -CF₂-, or a C₃ to C₅ cycloalkane ring or cycloalkene ring, provided that the oxygen atom and the sulfur atom are not adjacent to each other,
   B1, B2, B3 and B4 independently represent a covalent bond or a C₁ to C₄ alkylene group, in which one or more -CH₂- groups may be replaced by an oxygen atom, a sulfur atom, -CH=CH-, -C≡C-, -CO-, -CF=CF-, -CF₂-, or a C₃ to C₅ cycloalkane ring or cycloalkene ring, provided that the oxygen atom and the sulfur atom are not adjacent to each other,
   n1, n2, n3, n4, m1, m2 and m3 are independently 0 or 1,
   rings A1, A2, A3 and A4 independently represent a benzene ring in which one to four hydrogen atoms may be replaced by halogen atoms, bicyclo[1.1.1]pentane ring, bicyclo[2.1.1]hexane ring, bicyclo[2.2.1]heptane ring, bicyclo[2.2.2]octane ring, naphthalene ring, 1,2,3,4-tetrahydronaphthalene ring, perhydronaphthalene ring, fluorene ring, phenanthrene ring, 9,10-dihydrophenanthrene ring, indan ring, indene ring, pyridine ring, pyrimidine ring, C₄ to C₂₀ cycloalkane ring or cycloalkene ring, and -CH₂- in the rings may be replaced by an oxygen atom(s) or a sulfur atom,
   G represents any one of the groups represented by (G1) to (G3): 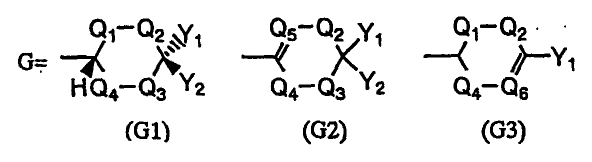
   Q₁, Q₂, Q₃ and Q₄ are independently a covalent bond, an oxygen atom, a sulfur atom, -CH₂-, -CH₂CH₂-, -CH=CH-, -CO-, -CF=CF- or -CF₂-, whereupon the number of the covalent bonds is 2 or less and Q₁ and Q₄ are preferential, the oxygen atom and the sulfur atom are not adjacent to each other, and Q₅ and Q₆ are independently =CH-, =CF- or =CCl-,
   Y₁ and Y₂ are independently a hydrogen atom, a halogen atom, a cyano group, a nitro group, an amino group, or a C₁ to C₂₀ alkyl group or halogenated alkyl group, in which one or more methylene groups may be replaced by an oxygen atom, a sulfur atom, -CH=CH-, -C≡C-, -CO-, -CF=CF-, -CF₂-, or a C₃ to C₅ cycloalkane ring or cycloalkene ring, provided that the oxygen atom and the sulfur atom are not adjacent to each other,
   when G is a group of the formula (G1), Y₁ and Y₂ are not simultaneously hydrogen atoms; both Q₁ and Q₄ are oxygen atoms, both Q₂ and Q₃ are -CH₂-, and Y₂ is a hydrogen atom, then n4 is 1 and B4 is -CH=CH-; it is not a case Q₂ and Q₃ are independently an oxygen atom or a sulfur atom, and Y₁ and Y₂ are a combination of a hydrogen atom and a saturated alkyl group, of a hydrogen atom and a trifluoromethyl group, or of a trifluoromethyl group and a trifluoromethyl group; and it is not a case that all Q₁ to Q₄ are -CH₂-,
   when G is a group of the formula (G2), Y₂ is not a hydrogen atom insofar as all Q₂ to Q₄ are -CH₂- and Q₅ is =CH-,
   when G is a group of the formula (G3), all it is not a case that Q₁, Q₂ and Q₄ are -CH₂-, and Q₆ is not =CH-.
(2) A liquid crystalline compound according to item (1) wherein G is a group represented by the formula (G1).
(3) A liquid crystalline compound according to item (1) wherein G is a group represented by the formula (G2).
(4) A liquid crystalline compound according to item (1) wherein G is a group represented by the formula (G3).
(5) A liquid crystalline compound according to item (2) wherein Q₂ and Q₃ are independently an oxygen atom or a sulfur atom.
(6) A liquid crystalline compound according to item (5), wherein both Q₁ and Q₄ are -CH₂-.
(7) A liquid crystalline compound according to item (5) wherein Y₁ is a halogen atom or a cyano group.
(8) A liquid crystalline compound according to item (5) wherein Y₁ and Y₂ are independently a fluorine atom or a chlorine atom.
(9) A liquid crystalline compound according to item (5) wherein Y₁ is a C₁ to C₂₀ alkyl group in which one or more -CH₂- groups are replaced by -CH=CH-, -C≡C-, -CO-, -CF=CF- or CF₂, Y₂ is a hydrogen atom and it is not a case that m3 is 1 and rings A3 and A4 are simultaneously cyclohexane rings.
(10) A liquid crystalline compound according to item (9) wherein Y₁ is a C₁ to C₂₀ alkyl group in which one or more -CH₂- groups are replaced by -CH=CH-.
(11) A liquid crystalline compound according to item (10) wherein Y₁ is -CH=CH₂.
(12) A liquid crystal composition comprising at least one liquid crystalline compound according to any of items (1) to (11).
(13) A liquid crystal composition comprising as a first component at least one liquid crystalline compound according to any of items (1) to (11) and as a second component at least one compound selected from the group of compounds expessed by the general formulae (2), (3) or (4): 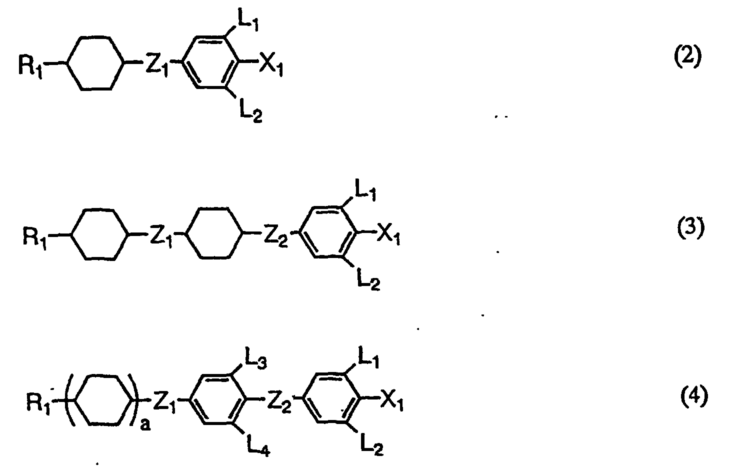 wherein R₁ represents a C₁ to C₁₀ alkyl group, X₁ represents F, Cl, -OCF₃, -OCF₂H, -CF₃, -CF₂H or -CFH₂, L₁, L₂, L₃ and L₄ independently represent H or F, Z₁ and Z₂ independently represent -(CH₂)₂-, -CH=CH- or a single bond, and a represents 1 or 2.
(14) A liquid crystal composition comprising as a first component at least one liquid crystalline compound according to any of items (1) to (11) and as a second component at least one compound selected from the group of compounds expressed by the general formulae (5), (6), (7), (8) or (9): 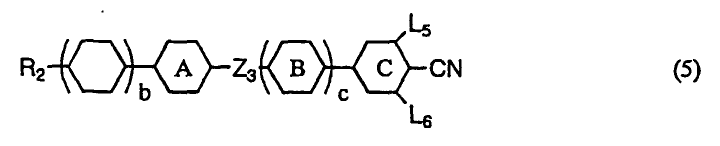 wherein R₂ represents F or a C₁ to C₁₀ alkyl group or C₂ to C₁₀ alkenyl group a nonadjacent and arbitrary methylene group(s) in the groups may be replaced by oxygen atoms, ring A represents a trans-1,4-cyclohexylene group, 1,4-phenylene group, or 1,3-dioxane-2,5-diyl group, ring B represents a trans-1,4-cyclohexylene group, 1,4-phenylene group, or pyrimidine-2,5-diyl group, ring C represents a trans-1,4-cyclohexylene group or 1,4-phenylene group, Z₃ represents -(CH₂)₂-, -COO- or a single bond, L₅ and L₆ independently represent H or F, and b and c independently one another represent 0 or 1. 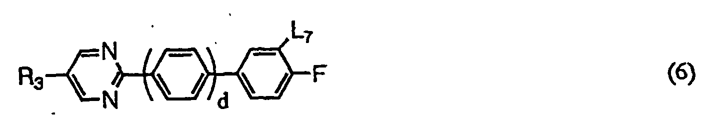 wherein R₃ represents a C₁ to C₁₀ alkyl group, L₇ represents H or F, and d represents 0 or 1. 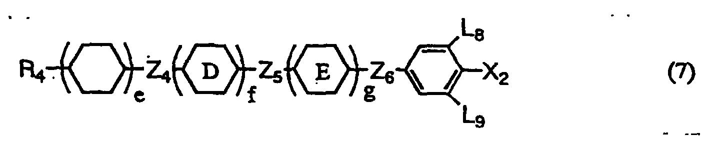 wherein R₄ represents a C₁ to C₁₀ alkyl group, rings D and E independently one another represent a trans-1,4-cyclohexylene group or 1,4-phenylene group, Z₄ and Z₅ independently one another represent -COO- or a single bond, Z₆ represents -COO-or -C≡C-, X₂ represents F, -OCF₃, -OCF₂H, -CF₃, -CF₂H or -CFH₂, and L₈ and L₉ independently one another represent H or F, and e, f and g independently one another represent 0 or 1. 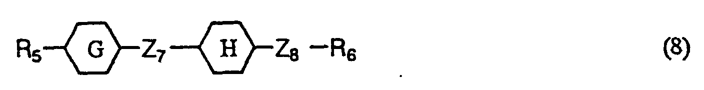 wherein R₅ and R₆ independently one another represent a C₁ to C₁₀ alkyl group or C₂ to C₁₀ alkenyl group a nonadjacent and arbitrary methylene groups in the rings may be replaced by oxygen atoms, ring G represents a trans-1,4-cyclohexylene group, 1,4-phenylene group, or pyrimidine-2,5-diyl group, ring H represents a trans-1,4-cyclohexylene group or 1,4-phenylene group, Z₇ represents -C≡C-, -COO-, -(CH₂)₂-, -CH=CH-C≡C- or a single bond, and Z₈ represents -COO- or a single bond, 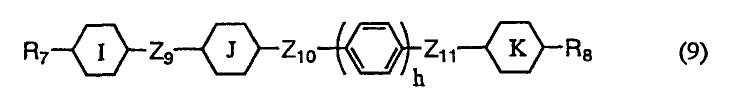 wherein R₇ and R₈ independently represent a C₁ to C₁₀ alkyl group or C₂ to C₁₀ alkenyl group in which arbitrary methylene groups not adjacent to each other may be replaced by oxygen atoms, ring I represents a trans-1,4-cyclohexylene group, 1,4-phenylene group, or pyrimidine-2,5-diyl group, ring J represents a trans-1,4-cyclohexylene group, a 1,4-phenylene group in which one or more hydrogen atoms may be replaced by F, or a pyrimidine-2,5-diyl group, ring K represents a trans-1,4-cyclohexylene group or 1,4-phenylene group, Z₉ and Z₁₁ independently one another represent -COO-, -(CH₂)₂- or a single bond, Z₁₀ represents -CH=CH-, -C≡C-, -COO- or a single bond, and h represents 0 or 1.
(15) A liquid crystal composition comprising as a first component at least one liquid crystalline compound according to any of items (1) to (11) and as a part of a second component at least one compound selected from the group consisting of the general formulae (2), (3) and (4) and as the other part of the second component at least one compound selected from the group consisting of the general formulae (5), (6), (7), (8) and (9).
(16) A liquid crystal display device fabricated by using a liquid crystal composition according to any of items (12) to (15).

The liquid crystalline compound of the general formula (1) according to the present invention is characterized that it has a partial skeleton in which a (mainly heterocyclic) ring other than benzene ring at the molecular terminal is replaced by a halogen atom, a cyano group, a nitro group, an amino group, or a C₁ to C₂₀ halogenated alkyl group. By making such a structure, the compound (1) of the present invention if having small Δn has large Δε or a high clearing point, or if having middle Δn, it has large Δε. The compounds of the invention having small Δn wherein said terminal group is replaced by an alkyl group in which -CH₂- may be replaced by -CH=CH- possess a high clearing point or are excellent in miscibility if the other terminal substituent group is an alkyl group in which -CH₂- may be replaced by -CH=CH-, or possess large Δε if the other terminal substituent group is the above-described electron withdrawing group. Accordingly, these compounds of the present invention are used as components in a liquid crystalline composition, a new liquid crystal composition having preferable characteristics can be provided.

The liquid crystalline compounds represented by the general formula (1) of the invention possess excellent characteristics as described above. The structures and characteristics thereof are described in more detail.

The side chain R represents a hydrogen atom, a cyano group, a halogen atom, or a C₁ to C₂₀ alkyl group and halogenated alkyl group in which one or more methylene groups may be replaced by an oxygen atom, a sulfur atom, -CH=CH-, -C≡C-, -CO-, -CF=CF-, -CF₂-, a C₃ to C₅ cycloalkane ring and cycloalkene ring, provided that the oxygen atom and the sulfur atom are not adjacent to each other, and in the general formula (1), the compound group (R-1) or (R-2) defined below possesses more preferable characteristics.

### Compound group (R-1):

Compounds of the general formula (1) wherein R is an alkyl group and halogenated alkyl group in which one or more -CH₂-groups may be replaced by an oxygen atom and -CH=CH-.

This compound group possesses a high clearing point and is low viscous, and if Y₁ and/or Y₂ is an electron withdrawing group such as fluorine atom or cyano group, this compound group has large Δε.

### Compound group (R-2):

Compounds of the general formula (1) wherein R is a cyano group, a halogen atom, a trifluoromethyl group, a trifluoromethoxy group or a C₁ to C₇ halogenated alkyl group in which one or more methylene groups may be replaced by oxygen atoms. This compound group has large Δε if Y₁ and Y₂ are not the electron withdrawing groups described above.

In the general formula (1), the number of rings is defined by m1 to m3, and m1 to m3 are independently 0 or 1, and the compound groups (m-1) to (m-3) possess preferable characteristics.

### Compound group (m-1):

Compounds of the general formula (1) wherein m1 = m2 = m3 = 0.

This compound group possesses superior characteristics such as very low viscosity and extremely good miscibility.

### Compound group (m-2):

Compounds of the general formula (1) wherein m1 = m2 = 0, m3 = 1.

This compound group possesses superior characteristics such as high clearing point, low viscosity and good miscibility. (m-3) Compounds of the general formula (1) wherein m1 = 0, m2 = m3 = 1.

This compound group possesses characteristics such as very high clearing point and the ability to significantly increase the upper limit of the clearing point of a composition upon incorporation in a small amount into the composition.

In the general formula (1), the number of bonding groups is defined by n1 to n4, and n1 to n4 are independently 0 or 1, and the compound group (n-1) or (n-2) has more preferable characteristics.

### Compound group (n-1):

Compounds of the general formula (1) wherein n1 = n2 = n3 = n4 = 0.

This compound group possesses a high clearing point and low viscosity.

### Compound group (n-2):

Compounds of the general formula (1) wherein n1 + n2 + n3 + n4 = 1 or 2.

This compound group possesses a high clearing point and excellent miscibility.

In the general formula (1), the bonding groups B₁ to B₄ are independently a covalent bond or a C₁ to C₄ alkylene group in which one or more -CH₂- groups may be replaced by an oxygen atom, a sulfur atom, -CH=CH-, -C≡C-, -CO-, -CF=CF-, -CF₂-, a C₃ to C₅ cycloalkane ring or cycloalkene ring, provided that the oxygen atom and the sulfur atom are not adjacent to each other, and the following compound groups (B-1) to (B-4) possess more preferable characteristics.

### Compound group (B-1):

Compounds of the general formula (1) wherein the corresponding n1 to n4 are 1, and at least one of B₁ to B₄ is a C₂ or C₄ alkylene group in which one -CH₂- group may be replaced by an oxygen atom.

This compound group possesses a high clearing point and excellent miscibility and is more stable to heat and light than compound group (B-2).

### Compound group (B-2):

Compounds of the general formula (1) wherein the corresponding n1 to n4 are 1, and at least one of B₁ to B₄ is -CH=CH- or -C=C-.

This compound group is inferior in stability to compound group (B-1), but possesses a high clearing point and a remarkably wide nematic range.

### Compound group (B-3):

Compounds of the general formula (1) wherein the corresponding n1 to n4 are 1, and at least one of B₁ to B₄ is -CF=CF-.

This compound group is inferior in stability to compound group (B-1), but has extremely low viscosity and a wide temperature range of liquid crystal.

### Compound group (B-4):

Compounds of the general formula (1) wherein the corresponding n1 to n4 are 1 and at least one of B₁ to B₄ is -COO- or -CF₂O-.

This compound group possesses large Δε.

In the general formula (1), rings A1, A2, A3 and A4 independently represent a benzene ring in which one to four hydrogen atoms may be replaced by halogen atoms, a pyridine ring and a pyrimidine ring, as well as bicyclo[1.1.1]pentane ring, bicyclo[2.1.1]hexane ring, bicyclo[2.2.1]heptane ring, bicyclo[2.2.2]octane ring, naphthalene ring, 1,2,3,4-tetrahydronaphthalene ring, perhydronaphthalene ring, fluorene ring, phenanthrene ring, 9,10-dihydrophenanthrene ring, indan ring, indene ring, or C₄ to C₂₀ cycloalkane ring or cycloalkene ring, a carbon atom(s) in the rings may be replaced by an oxygen atom(s) or sulfur atom(s), and the following compound groups (A-1) to (A-2) possess more preferable characteristics.

### Compound group (A-1):

Compounds of the general formula (1) wherein rings A1, A2, A3 and A4 are independently a benzene ring in which one to four hydrogen atoms may be replaced by a fluorine atom(s), a cyclohexane ring or a cyclohexene ring.

This compound group possesses low viscosity and is stable to heat and light.

### Compound group (A-2):

Compounds of the general formula (1) wherein ring A4 or the corresponding m1 to m3 are 1, and at least one of rings A1, A2 and A3 is a pyridine ring or a pyrimidine ring.

This compound group possesses large Δε and small elastic constant, so a liquid crystal composition containing this compound group is characterized in that it exhibits low threshold voltage in TN cells.

In the ring linking in the general formula (1), the following (A-3) to (A-15) possess particularly preferable characteristics. In the following description, the benzene ring in which one to four hydrogen atoms may be replaced by fluorine atoms is expressed as B'', the cyclohexane ring is expressed as H, the cyclohexane ring having a double bond at the R side is expressed as Ch, and the cyclohexane ring having a double bond at the G side is expressed as ch.

### Compound group (A-3):

Compounds (m-1) wherein ring A4 is H. This compound group possesses small Δn and extremely low viscosity.

### Compound group (A-4):

Compounds (m-1) wherein ring A4 is B''. This compound group possesses extremely low viscosity and very excellent miscibility.

### Compound group (A-5):

Compounds (m-1) wherein ring A4 is Ch or ch. This compound group possesses small Δn and extremely low viscosity.

### Compound group (A-6):

Compounds (m-2) wherein rings A3 and A4 are B'' and H respectively. This compound group possesses a high clearing point and excellent miscibility.

### Compound group (A-7):

Compounds (m-2) wherein rings A3 and A4 are B'' and B'' respectively. This compound group possesses excellent miscibility and large Δn.

### Compound group (A-8):

Compounds (m-2) wherein rings A3 and A4 are H and H respectively. This compound group possesses a high clearing point and small Δn.

### Compound group (A-9):

Compounds (m-2) wherein rings A3 and A4 are H and Ch or ch respectively. This compound group possesses excellent miscibility and small Δn.

### Compound group (A-10):

Compounds (m-2) wherein rings A3 and A4 are B'' and Ch or ch respectively. This compound group possesses very excellent miscibility.

### Compound group (A-11):

Compounds (m-2) wherein rings A3 and A4 are H and B'' respectively. This compound group possesses a high clearing point and excellent miscibility.

### Compound group (A-12):

Compounds (m-3) wherein rings A2, A3 and A4 are H, B'' and B'' respectively. This compound group possesses a very high clearing point and relatively low viscosity.

### Compound group (A-13):

Compounds (m-3) wherein rings A2, A3 and A4 are B'', B'' and H respectively.

### Compound group (A-14):

Compounds (m-3) wherein rings A2, A3 and A4 are B'', H and H respectively.

### Compound group (A-15):

Compounds (m-3) wherein rings A2, A3 and A4 are H, H and B'' respectively.

The compound groups (A-13) to (A-15) described above possess a very high clearing point.

By selection from the above compound groups and further selecting G in the compounds, the liquid crystalline compounds having desired characteristics can be obtained. Among these, compounds having specific groups from 2 or more compound groups described above are preferable.

Q₁ through Q₆ in G in the general formula (1) are suitably selected to be oxygen atoms or sulfur atoms so that the compounds having these atoms possess larger Δε. The partial skeleton G inducing such excellent characteristics is described in more detail.

In the case of compounds where R is the above-described alkyl group and halogenated alkyl group (excluding compounds wherein the core-bound carbon among carbons in R is substituted with a halogen atom), the following structure is superior in Δε, high clearing point and viscosity.

### Compounds wherein G = (G1)

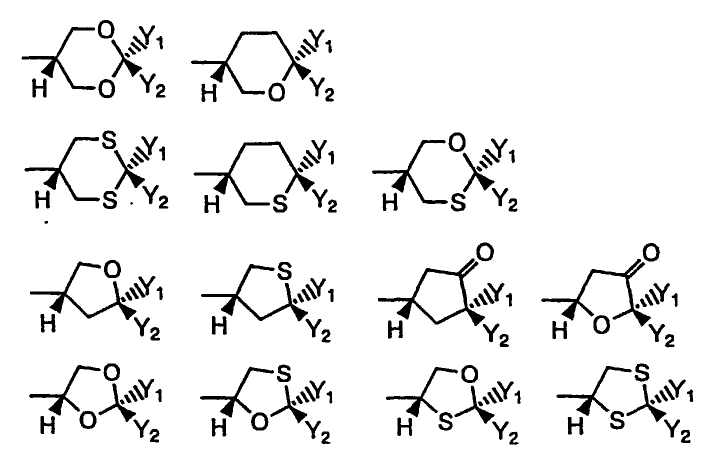

Among the above, the structure particularly having large Δε is the one wherein Q₂ and Q₃ are respectively an oxygen atom or a sulfur atom.

### Compounds wherein G = (G2)

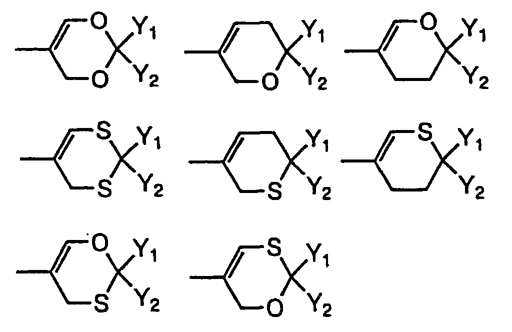

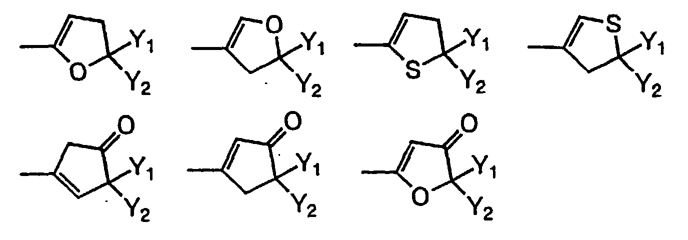

Among the above, the structure particularly having large Δε is the one wherein Q₂ and Q₃ respectively are an oxygen atom or a sulfur atom.

### Compounds wherein G = (G3)

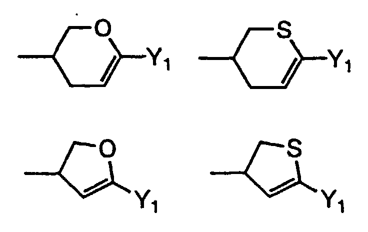

Among the above, the structure particularly having large Δε is the one wherein Q₂ is an oxygen atom or a sulfur atom.

If R is the halogenated alkyl group in which among carbons, carbon bound to the core (ring A1) is substituted with a halogen atom, a halogen atom or a cyano group, then the following structures in addition to those wherein (G-1) to (G-3) have the structures described above possess excellent characteristics with respect to Δε, high clearing point and viscosity.

### Compounds wherein G = (G1)

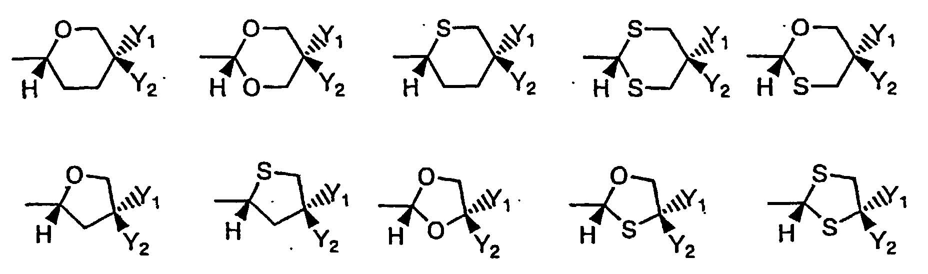

Among the above, the structure particularly having large Δε is the one wherein Q₁ and Q₄ in the six-membered ring or Q₁ and Q₂ in the five-membered ring are respectively an oxygen atom or a sulfur atom. The structure wherein n4 = 1 and B4 = ethenylene group exhibits particularly large Δε.

### Compounds wherein G = (G2)

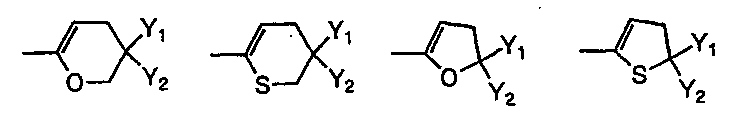

Among the above, the structure particularly having large Δε is the one wherein Q₄ is an oxygen atom or a sulfur atom.

### Compounds wherein G = (G3)

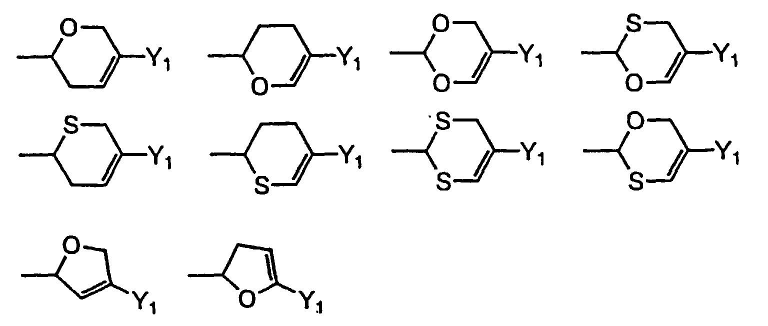

Among the above, the structure particularly having large Δε is the one wherein Q₁ and Q₄ in the six-membered ring or Q₁ and Q₂ in the five-membered ring is respectively an oxygen atom or a sulfur atom.

In the case where G is (G1) or (G2), any combination of Y₁ and Y₂ brings about superior characteristics, but combinations bringing about more superior characteristics are shown below.

In the case of the compounds wherein R is the above-described alkyl group and halogenated alkyl group (excluding compounds wherein the core-bound carbon among carbons in R is substituted with a halogen atom),
(1) compounds wherein Y₁ and Y₂ are independently a halogen atom are excellent compounds having large Δε, and more preferable compounds for low viscosity are particularly those wherein Y₁ and Y₂ are independently a fluorine atom or a chlorine atom, as well as those wherein Y₁ is a fluorine atom or a chlorine atom and Y₂ is a hydrogen atom;
(2) compounds wherein Y₁ is a cyano group and Y₂ is a hydrogen atom are those having very excellent characteristics such as very large Δε, high clearing points and low viscosity, and compounds wherein Y₁ is an alkenyl group and Y₂ is a hydrogen atom possess a very wide temperature range of nematic phase;
(3) compounds wherein Y₁ is the above-described halogenated alkyl group and Y₂ is a hydrogen atom are those having very large Δε and high clearing points, and particularly compounds wherein Y₁ is a C₁ to C₅ fluorinated alkyl group such as -CF₂H-, -CFH₂-, -CF₂CF₂H or -CF₂CF₃ and Y₂ is a hydrogen atom are preferable for low viscosity; and
(4) compounds wherein Y₁ is an alkyl group in which one or more -CH₂- groups may be replaced by -CH=CH- and Y₂ is a hydrogen atom are excellent in miscibility and low in viscosity.
   In the case of the compounds wherein R is the halogenated alkyl group in which among carbons, carbon bound to the core is substituted with a halogen atom, a halogen atom or a cyano group
(5) compounds wherein Y₁ is the above-described alkyl group or halogenated alkyl group (excluding compounds wherein the core-bound carbon among carbons in Y₁ is substituted with a halogen atom) and Y₂ is a hydrogen atom are preferable because they have large Δε.

The compounds represented by the general formula (1) according to the present invention can be produced in any methods known in the art or by suitable combination thereof. One example is shown below:

### (1) Method of constructing the partial skeleton G

### (1-1) In the case of G = (G1)

### (1-1-1) Method of synthesizing compounds represented by the general formula (14).

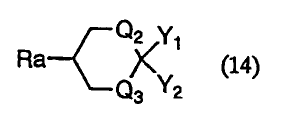

wherein Ra is the site indicated in the following formula. In the scheme, Ha1 indicates bromine or iodine.

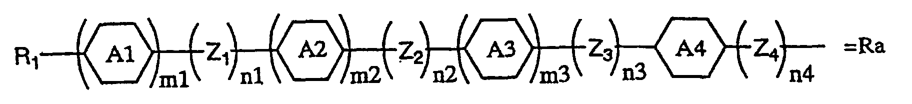

### (1-1-1-1) Method of synthesizing compounds wherein Q₂ and Q₃ are independently an oxygen atom or a sulfur atom.

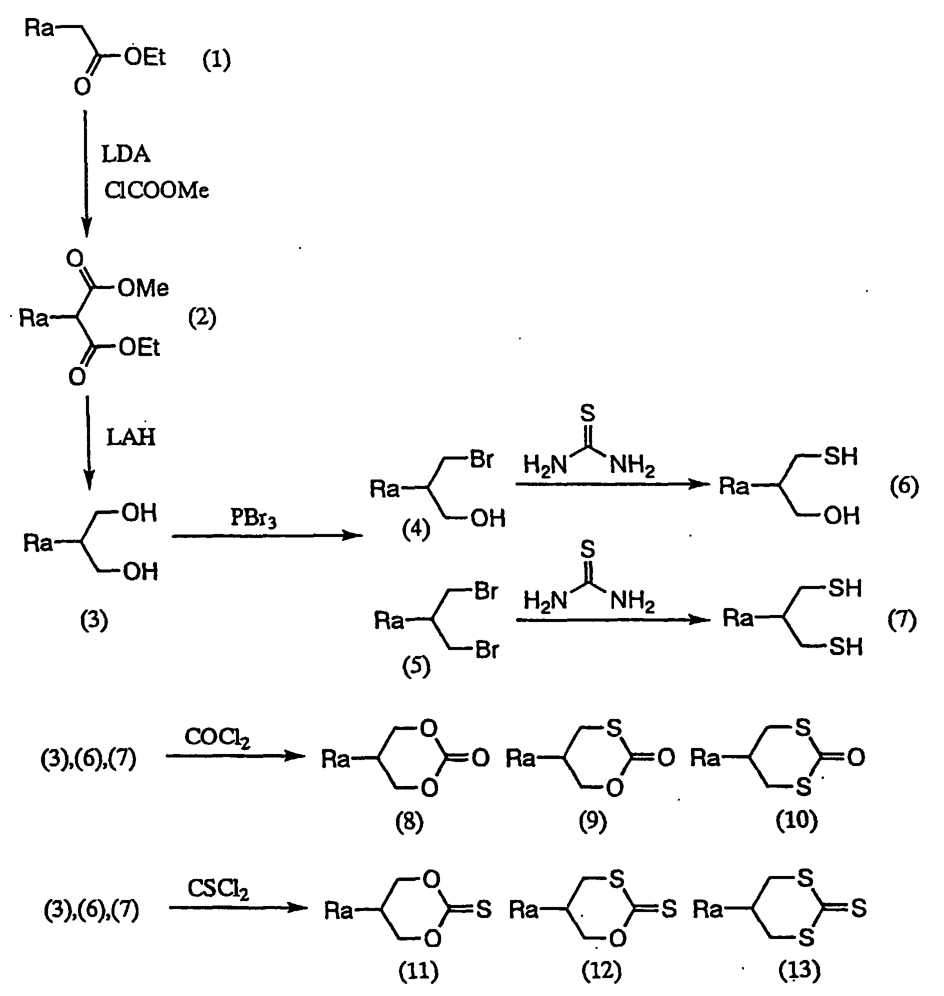

A base such as lithium diisopropylamide (abbreviated hereinafter to LDA) is allowed to act on ester derivative (1) in a solvent such as tetrahydrofuran (abbreviated hereinafter to THF) not participating in the reaction, followed by allowing methyl chlorobenzoate to act on it whereby diester derivative (2) can be obtained. A reducing agent such as lithium aluminum hydride (abbreviated hereinafter to LAH) is allowed to act on the compound (2) in a solvent such as THF not participating in the reaction, whereby the diol (3) can be obtained. A bromination reagent such as tribromophosphine is allowed to act on (3) whereby compound (4) or (5) can be obtained. Thiourea is allowed to act on the compound (4) or (5) whereby the compound (6) or (7) can be synthesized. (Tri)phosgene and pyridine are allowed to act on the compound (3), (6) or (7) in a solvent such as benzene or toluene not participating in the reaction, whereby the compound (8), (9) or (10) can be synthesized. Thiophosgene and pyridine are allowed to act on the compound (3), (6) or (7) in a solvent such as benzene or toluene not participating in the reaction, whereby the compound (11), (12) or (13) can be synthesized.

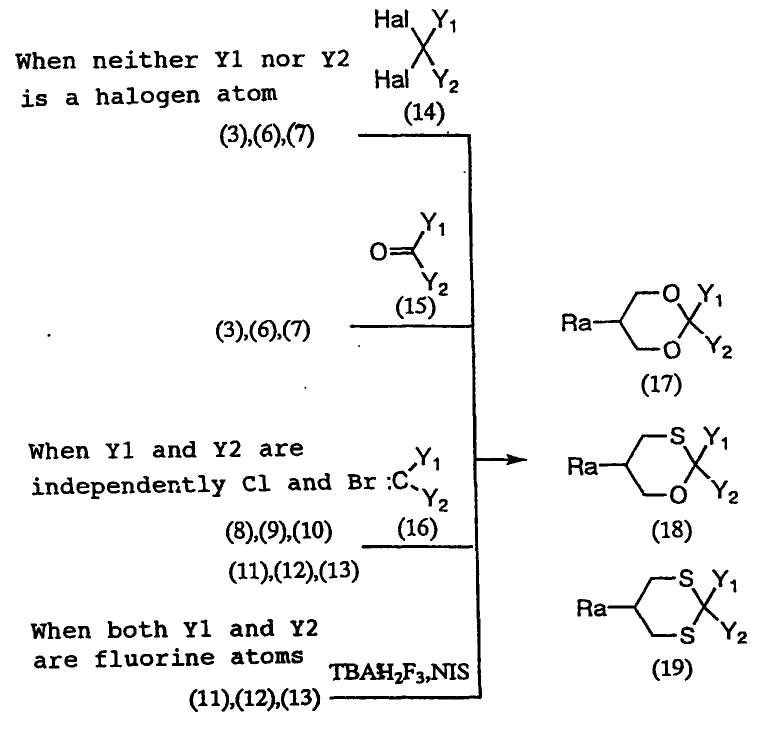

The method of synthesizing the compound (17), (18) or (19) is different depending on Y1 and Y2, so the synthesis method is shown for each case. If Y1 and Y2 are independently the above-described alkyl group, halogenated alkyl group or cyano group, then the dihalogen compound (14) or the carbonyl compound (15) is allowed to act on the compound (3), (6) or (7) whereby the corresponding compound (17), (18) or (19) can be synthesized. If Y1 and Y2 are independently a chlorine atom or bromine atom, then the carbene (16) is allowed to act on the compound (8), (9), (10) or (11), (12), (13), whereby the corresponding compound (17), (18) or (19) can be synthesized. If both Y1 and Y2 are fluorine atoms, then dihydrotrifluorotetrabutyl ammonium (abbreviated hereinafter to TBAH₂F₃) and N-iodinated succinic acid imide (abbreviated hereinafter to NIS) are allowed to act on the thiocarbonate derivative (11), (12) or (13) in a solvent such as 1,2-dichloroethane, chloroform or dichloromethane not participating in the reaction as described in Japanese Patent Publication Laid-open No. Hei 6-263679, whereby the corresponding compound (17), (18) or (19) can be synthesized.

### (1-1-1-2) A method of synthesizing compounds wherein Q₂ is -CH₂- and Q₃ is an oxygen atom.

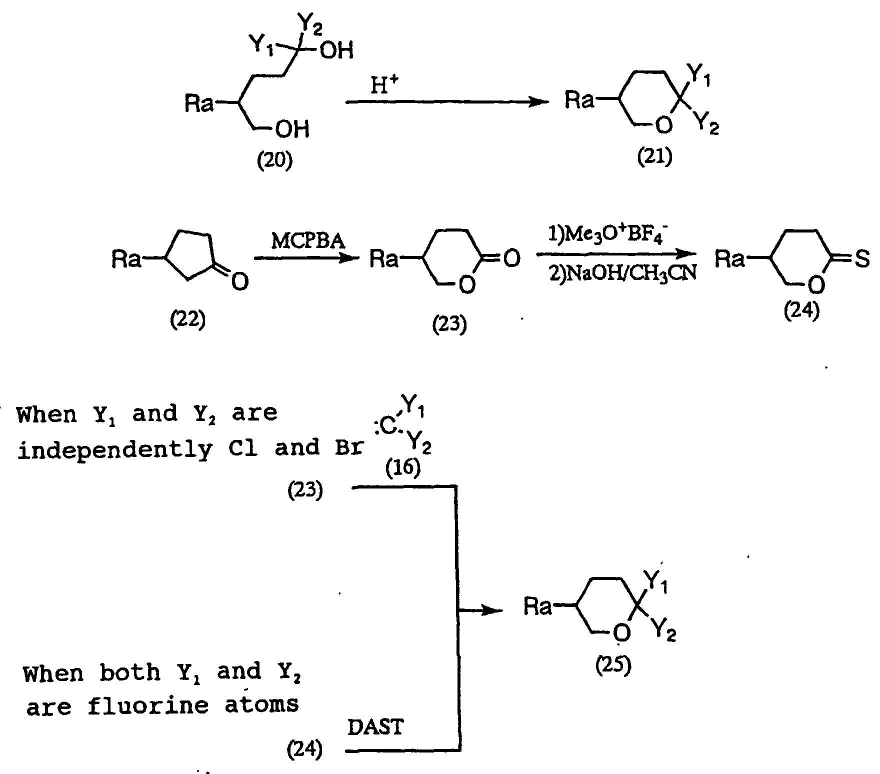

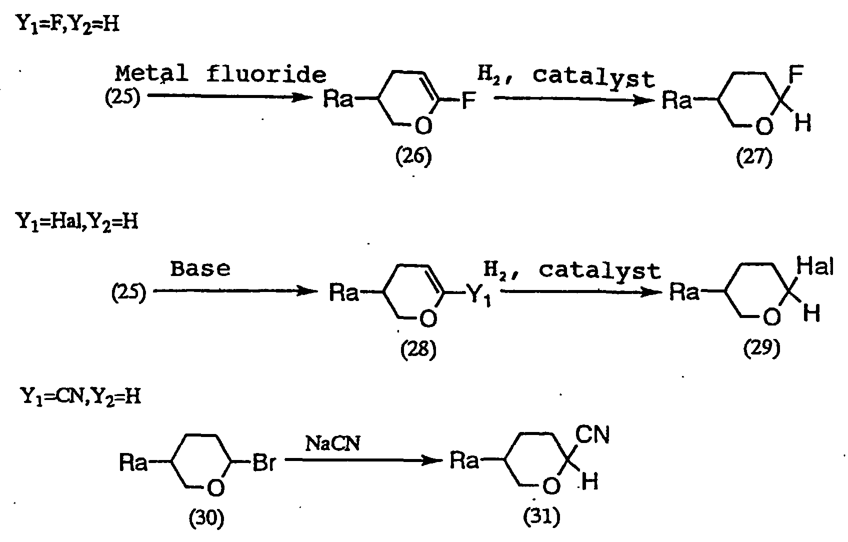

Compound (21) can be synthesized by allowing p-toluenesulfonic acid or cation-exchange resin to act on the compound (20) in a solvent such as toluene not participating in the reaction.

However, the compounds wherein Y1 and Y2 are independently a halogen atom, a cyano group or a hydrogen atom can be synthesized more preferably in the following manner. Compound (22) is subjected to Baeyer-Villiger reaction with a peroxide such as methachloroperbenzoic acid (abbreviated hereinafter to MCPBA) whereby lactone derivative (23) can be obtained. A Lawesson's reagent or a dimer of p-methoxyphenyl thionophosphine sulfide is allowed to act on the compound (23) in a solvent such as toluene not participating in the reaction, whereby it is converted into compound (24). Alternatively, the compound (24) can be synthesized by allowing a Meerwein Reagent and then sodium hydrosulfide to act on the compound (23), as described in Tetrahedron Letters Vol. 22, pp. 413-416 (1981). If Y₁ and Y₂ are halogen atoms other than fluorine atom, the carbene (16) is allowed to act on the lactone derivative (23) whereby the compound (25) can be synthesized. If both Y₁ and Y₂ are fluorine atoms, diethylaminosulfur trifluoride (abbreviated hereinafter to DAST) is allowed to act on the compound (24) in a solvent such as methylene chloride or dimethoxyethane not participating in the reaction, whereby the compound (25) can be synthesized. According to the method described in Japanese Patent Publication Laid-open No. 63-54332, a metal fluoride such as aluminum fluoride is allowed to act on the compound (25) (provided that both Y₁ and Y₂ are fluorine atoms) to synthesize compound (26) which is then hydrogenated in the presence of Pd/C as a catalyst whereby compound (27) can be synthesized. A base such as potassium t-butoxide and potassium hydroxide is allowed to act on the compound (25) (provided that Y₁ is a halogen atom other than fluorine atom and Y2 is a hydrogen atom) to synthesize compound (28) which is then hydrogenated by use of a Wilkinson's catalyst or a Pd/C catalyst, whereby compound (29) can be synthesized. For the compounds wherein Y₁ is a cyano group and Y₂ is a hydrogen atom, compound (30) is synthesized by the method of synthesizing the compound (29), followed by allowing a cyaniding reagent such as sodium cyanide to act on it whereby compound (31) can be synthesized.

### (1-1-2) A method of synthesizing compounds expressed by the general formula (15).

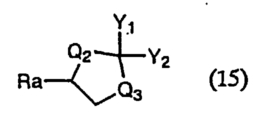

### (1-1-2-1) A method of synthesizing the compounds wherein Q₂ and Q₃ are independently an oxygen atom or a sulfur atom.

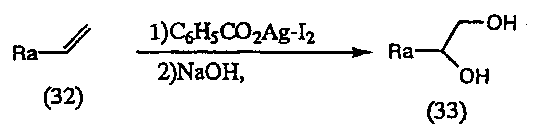

The compounds represented by the general formula (15) wherein Q₂ and Q₃ are independently an oxygen atom or a sulfur atom can be synthesized in the same manner as in synthesis of compounds (17), (18) and (19) except that, as described in "Synthesis and Reaction (I) of Organic Compounds" on pp. 492-493 in Shin Jikken Kagaku Kouza 14 (New Course of Chemical Experiment 14) (Maruzen), compound (32) is hydrolyzed by action of a reagent prepared from benzoic acid and iodine in e.g. benzene, and the resulting diol (33) is used as the starting material which is used in place of the compound (3).

### (1-1-2-2) A method of synthesizing the compounds wherein Q₂ is -CH₂- and Q₃ is an oxygen atom.

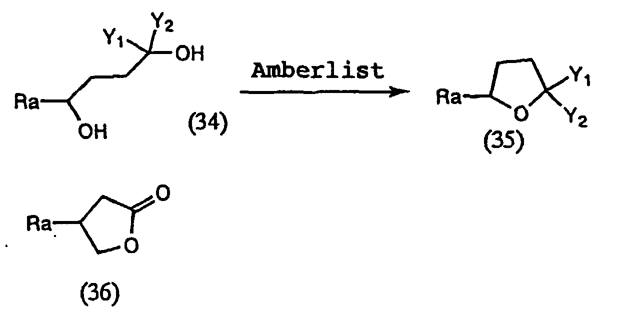

Compound (35) can be synthesized by allowing p-toluene sulfonic acid or cation exchange resin to act on compound (34) in a solvent such as toluene not participating in the reaction.

However, for the compounds wherein Y₁ and Y₂ are independently a halogen atom, a cyano group or a hydrogen atom, the desired compounds are synthesized more preferably in the same manner as in synthesis of the compounds (25), (27) and (29) except that the compound (23) is replaced by the compound (36).

### (1-1-2-3) A method of synthesizing the compounds wherein Q₂ is an oxygen atom and Q₃ is -CH₂-.

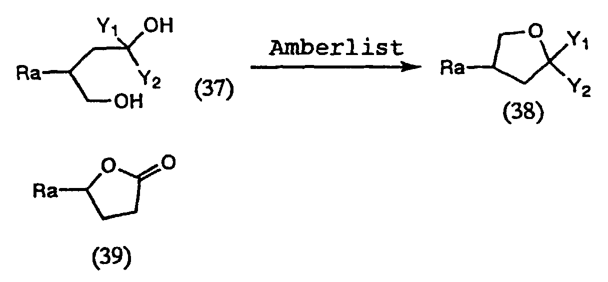

Compound (38) can be synthesized by allowing Amberlist to act on compound (37) in a solvent such as toluene not participating in the reaction.

However, for the compounds wherein Y₁ and Y₃ are independently a halogen atom, a cyano group or a hydrogen atom, the desired compounds are synthesized more preferably in the same manner as in synthesis of compounds (25), (27) and (29) except that the compound (23) is replaced by the compound (39).

### (1-2) In the case of G = (G2)

### (1-2-1) A method of synthesizing the compounds of the general formula (16) ((17)).

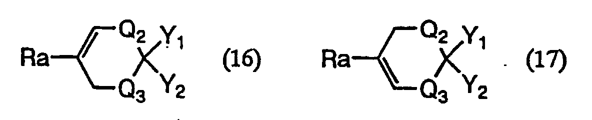

### (1-2-1-1) A method of synthesizing the compounds wherein Q₂ and Q₃ are independently an oxygen atom or a sulfur atom.

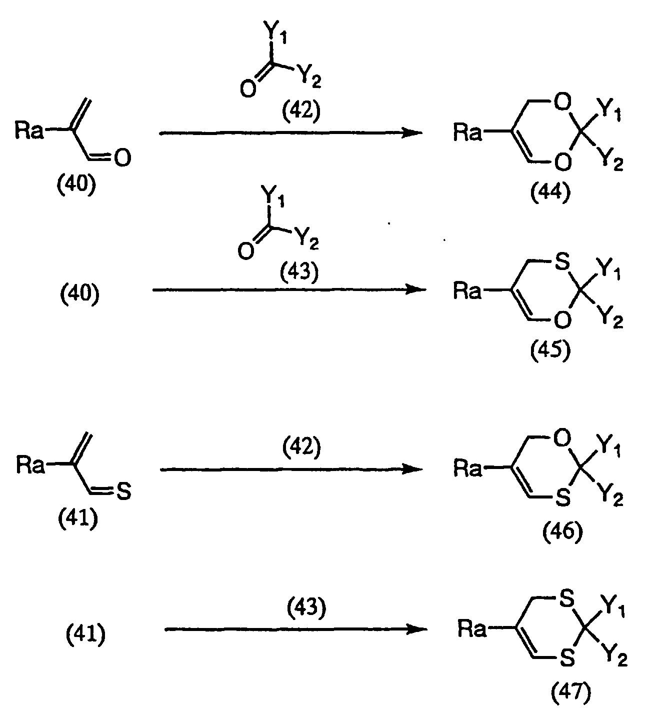

In the case where neither Y₂ nor Y₂ is a fluorine atom, compound (40) or (41) is subjected to cycloaddition reaction with compound (42) or (43) as described e.g. on pp. 74-75 in Guide 3 in Yuki Kagaku Jikken (Experiments in Organic Chemistry (Kagaku Dojin) whereby the desired compounds (44) to (47) can be synthesized. The compounds wherein Y₁ and/or Y₂ is a fluorine atom can be synthesized by converting a chlorine atom or bromine atom into a fluorine atom. A method for this conversion is described e.g. on pages 378-382 in Jikken Kagaku Kouza (Course of Chemical Experiment) (Fourth Edition).

### (1-2-1-2) A method of synthesizing the compounds wherein Q₂ is -CH₂- and Q₃ is an oxygen atom or a sulfur atom.

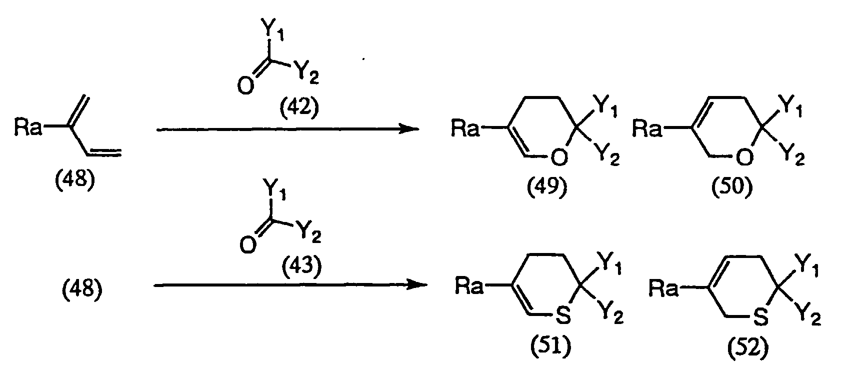

In the case where neither Y₁ nor Y₂ is a fluorine atom, compound (48) is subjected to cycloaddition reaction with compound (42) or (43) as described in e.g. on pp. 74-75 in Guide 3 in Yuki Kagaku Jikken (Experiments in Organic Chemistry (Kagaku Dojin) to synthesize (49) and (50) or (51) and (52), and these products are separated from each other whereby the desired compound can be obtained. The compounds wherein Y₁ and/or Y₂ is a fluorine atom can be synthesized by converting a chlorine atom or a bromine atom into a fluorine atom. A method for this conversion is described e.g. on pp. 378-382 in Jikken Kagaku Kouza (Course of Chemical Experiment) (Fourth Edition).

### (1-3) In the case of G = (G3)

### (1-3-1) A method of synthesizing compounds represented by the general formula (18).

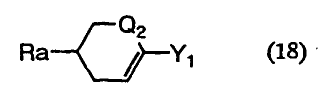

### (1-3-1-1) A method of synthesizing the compounds wherein Q₂ is an oxygen atom.

The compounds represented by the general formula (14) wherein X₂ is -CH₂-, X₃ is an oxygen atom and Y₂ is a halogen atom are synthesized, and then the resulting compounds wherein Y₂ is a fluorine atom are reacted with a metal fluoride such as aluminum fluoride according to the method described in Japanese Patent publication Laid-open No. 63-54332, or the resulting compounds wherein Y₂ is a halogen atom other than fluorine atom and Y2 is a hydrogen atom are reacted with a base such as potassium t-butoxide or potassium hydroxide, whereby the compounds represented by the general formula (18) can be synthesized. For the compounds wherein Y₁ is a cyano group, the desired compounds can be synthesized by allowing a cyaniding reagent such as sodium cyanide to act on (28).

The liquid crystal composition of the present invention preferably comprises 0.1 to 99.9 % by weight of one or more compounds of the general formula (1) in order to exhibit excellent characteristics.

Specifically, the liquid crystal composition provided by the present invention is completed by mixing the first component containing at least one compound (1) with compound(s) selected arbitrarily from other compounds of the general formula (1) or from compounds of the general formulae (2) to (9) depending on the object of the liquid crystal composition.

The liquid crystal composition provided by the present invention may comprise only the first component containing at least one of liquid crystalline compounds represented by the general formula (1), but preferably it further comprises at least one compound (referred to hereinafter as the second component A) selected from the group consisting of the general formulae (2), (3) and (4) and/or at least one compound (referred to hereinafter as the second component B) selected from the group consisting of the general formulae (5), (6), (7), (8) and (9). The liquid crystal composition may further comprise a known compound as a third component for the purpose of regulating threshold voltage, temperature range of liquid crystal phase, optical anisotropy, dielectric anisotropy, and viscosity.

Among the second component A described above, (2-1) to (2-15), (3-1) to (3-48) and (4-1) to (4-55) can be mentioned as preferable examples of compounds contained in the general formulae (2), (3) and (4). 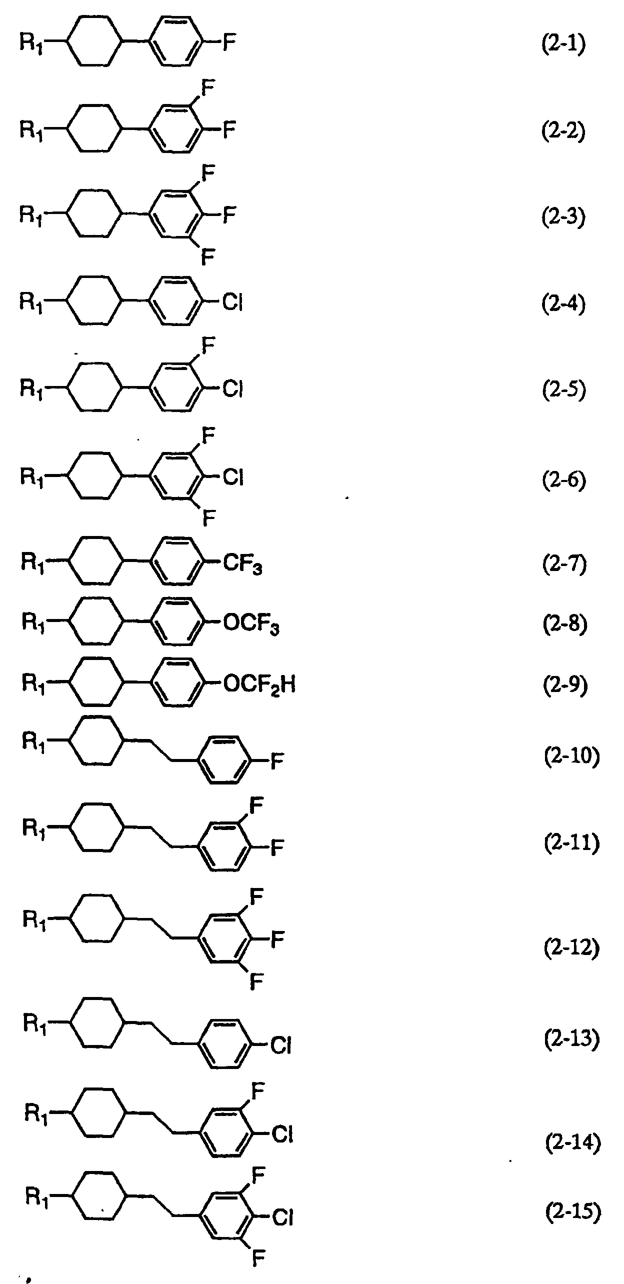 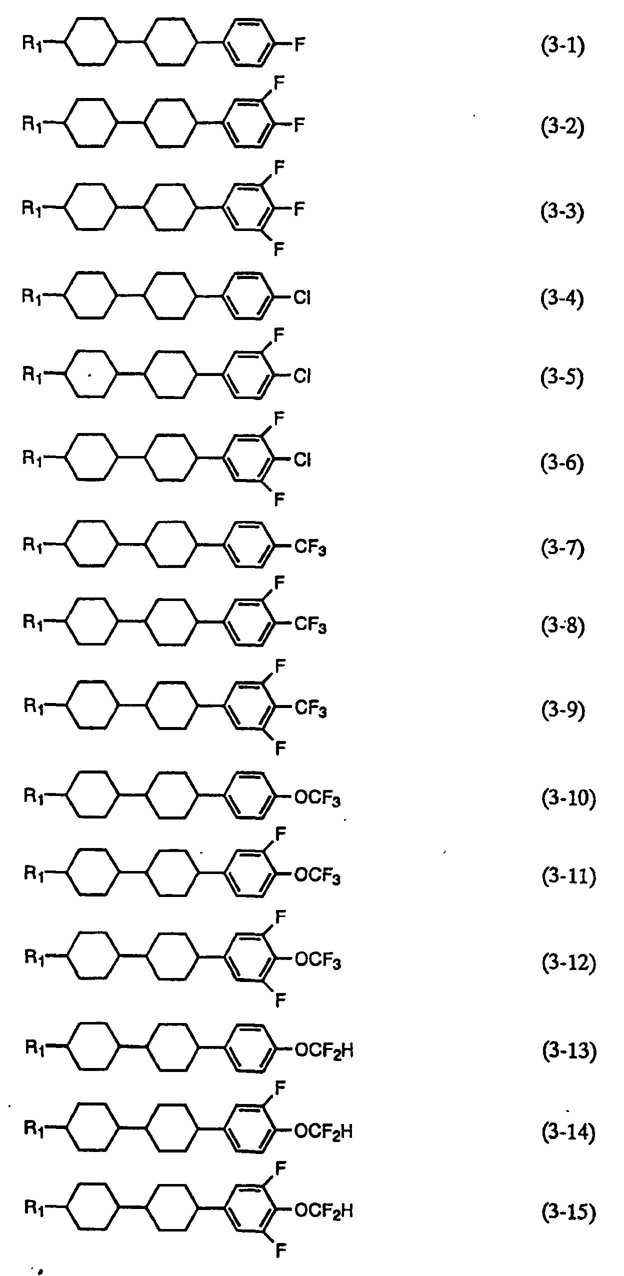 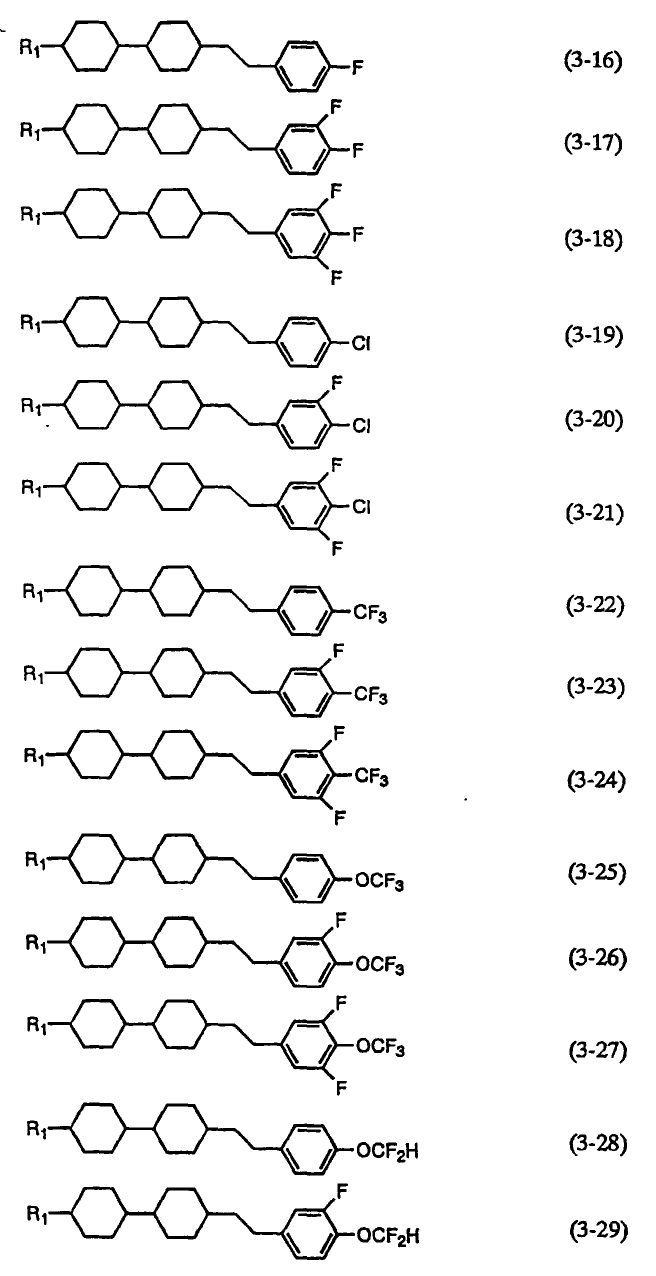 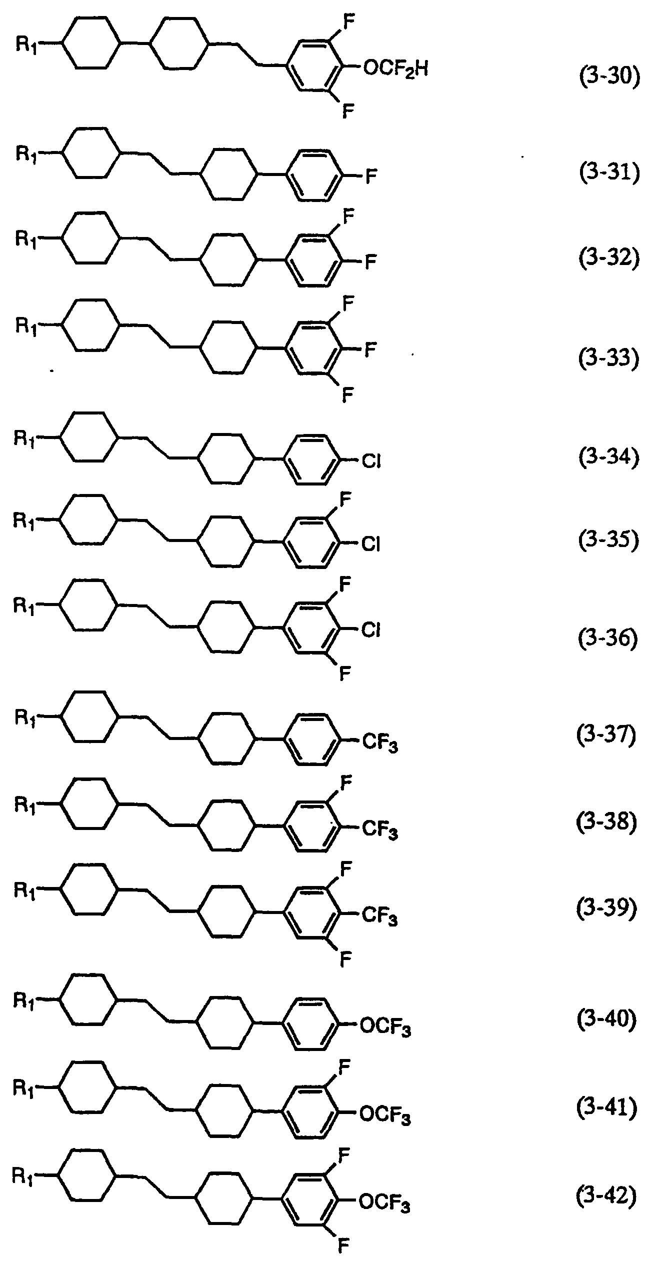 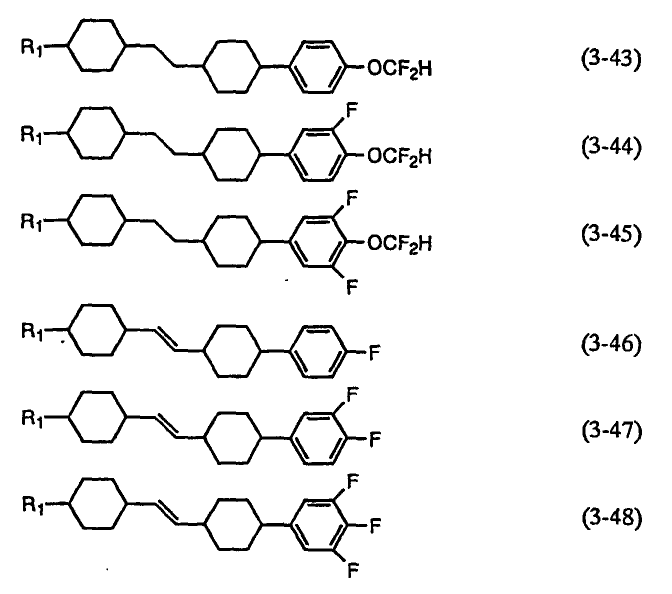 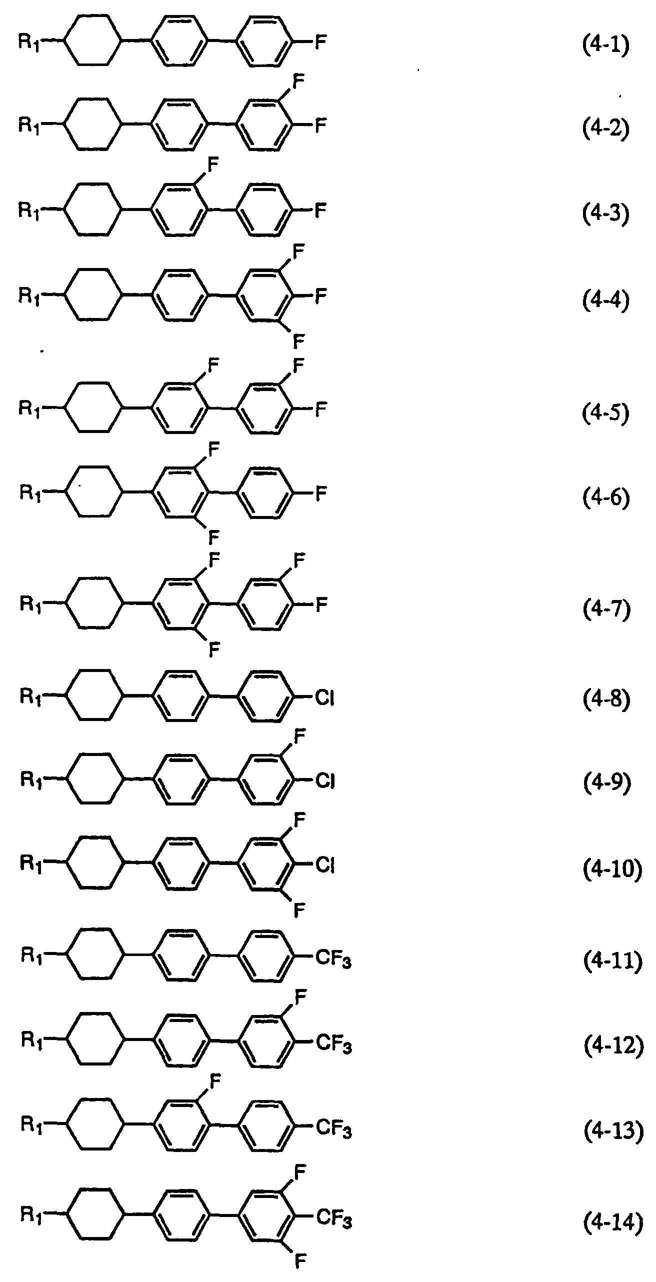 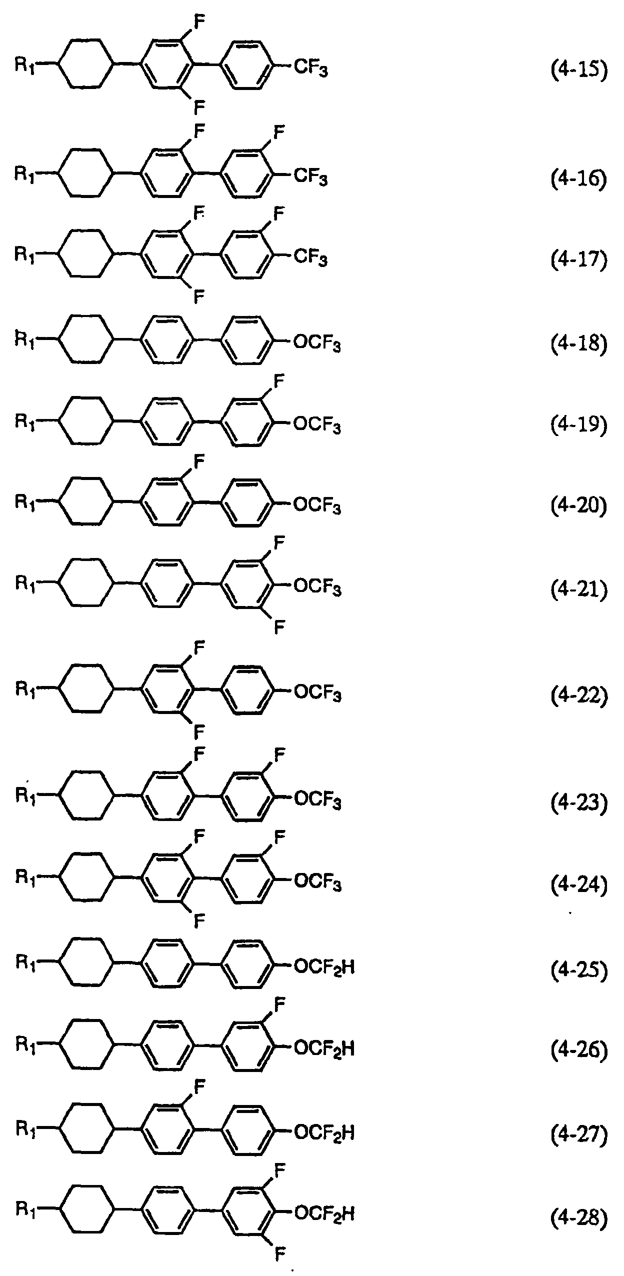 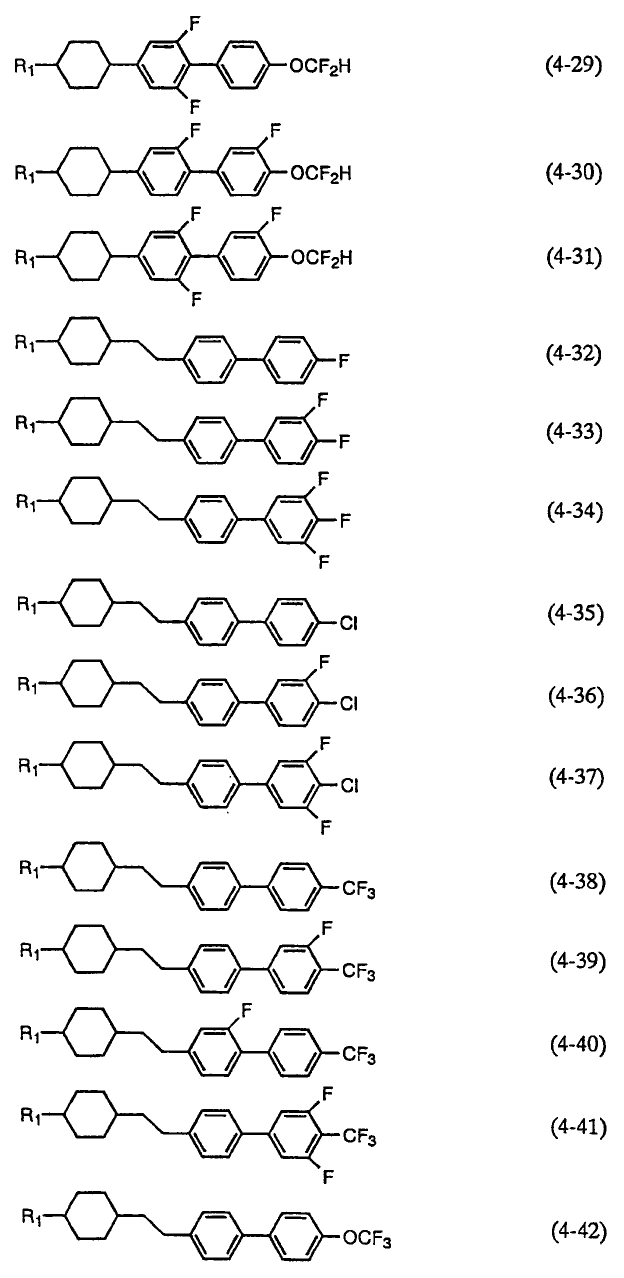 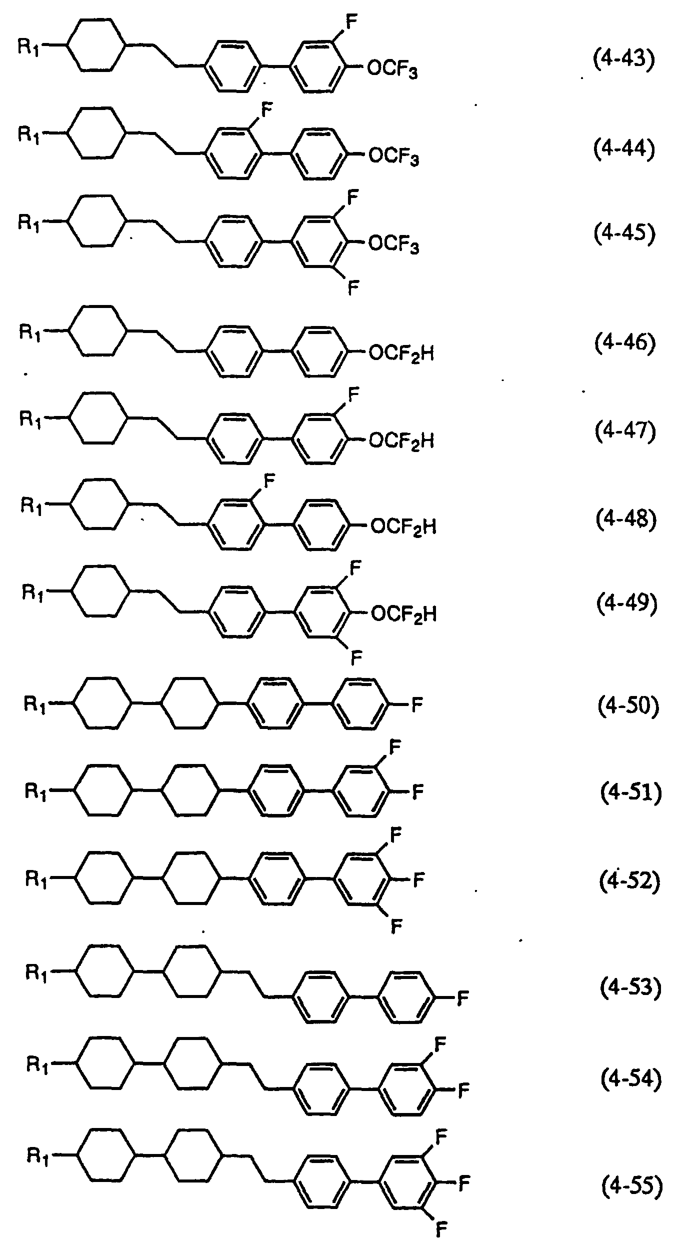 wherein R₁ has the same meanings as defined above.

Compounds represented the general formulae (2) to (4) show positive dielectric anisotropy values and are very superior in heat resistance and chemical stability.

The amount of these compounds used is preferably in the range of 1 to 99 % by weight, preferably 10 to 97 % by weight and more preferably 40 to 95 % by weight relative to the total weight of the liquid crystal composition.

Next, among the second component B described above, (5-1) to (5-24), (6-1) to (6-3) and (7-1) to (7-28) can be mentioned as preferable examples of compounds contained in the general formulae (5), (6) and (7). 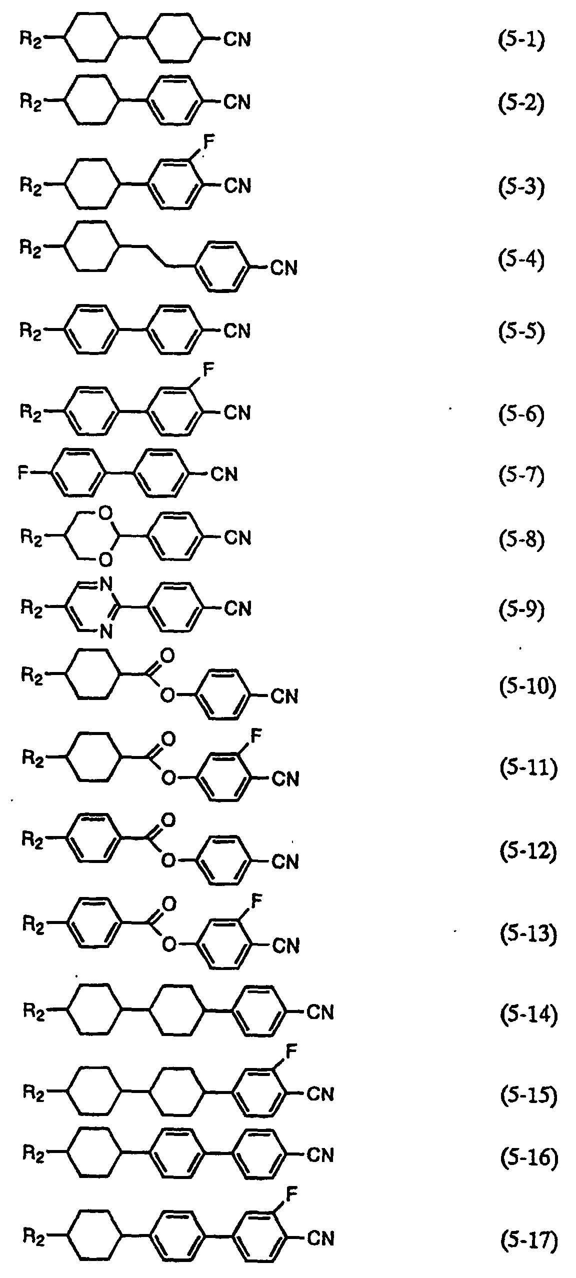 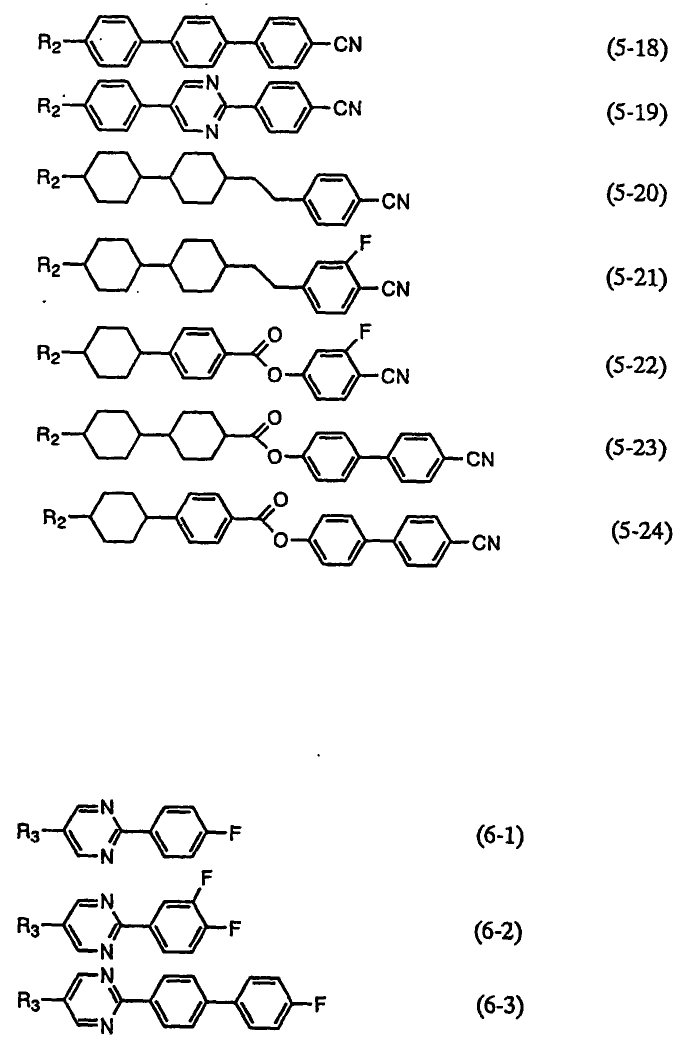 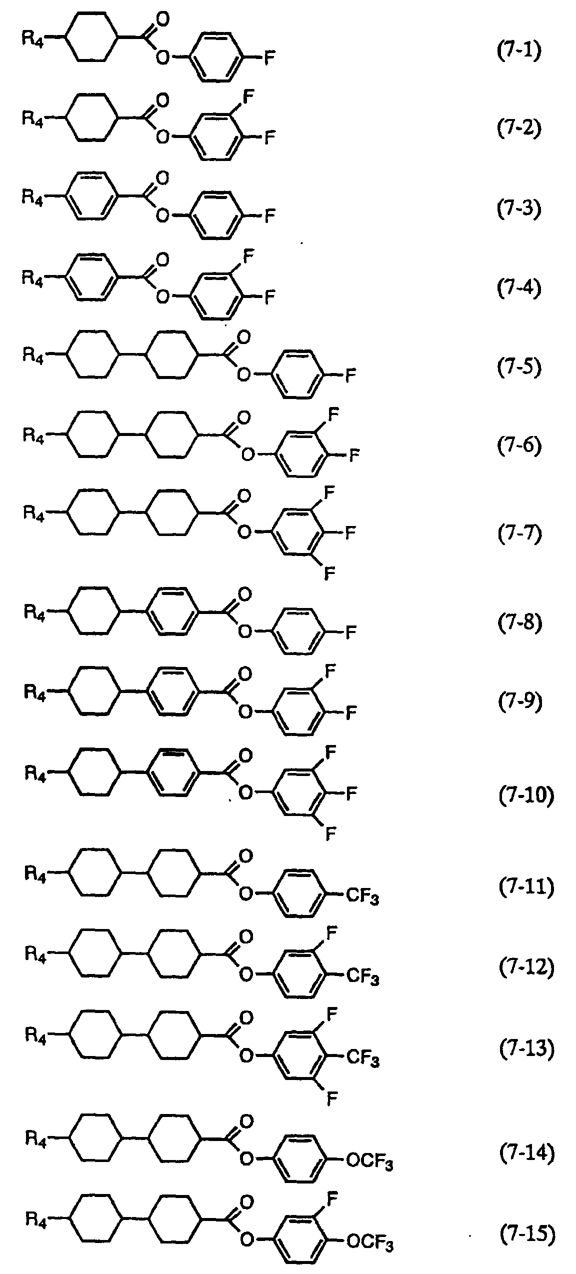 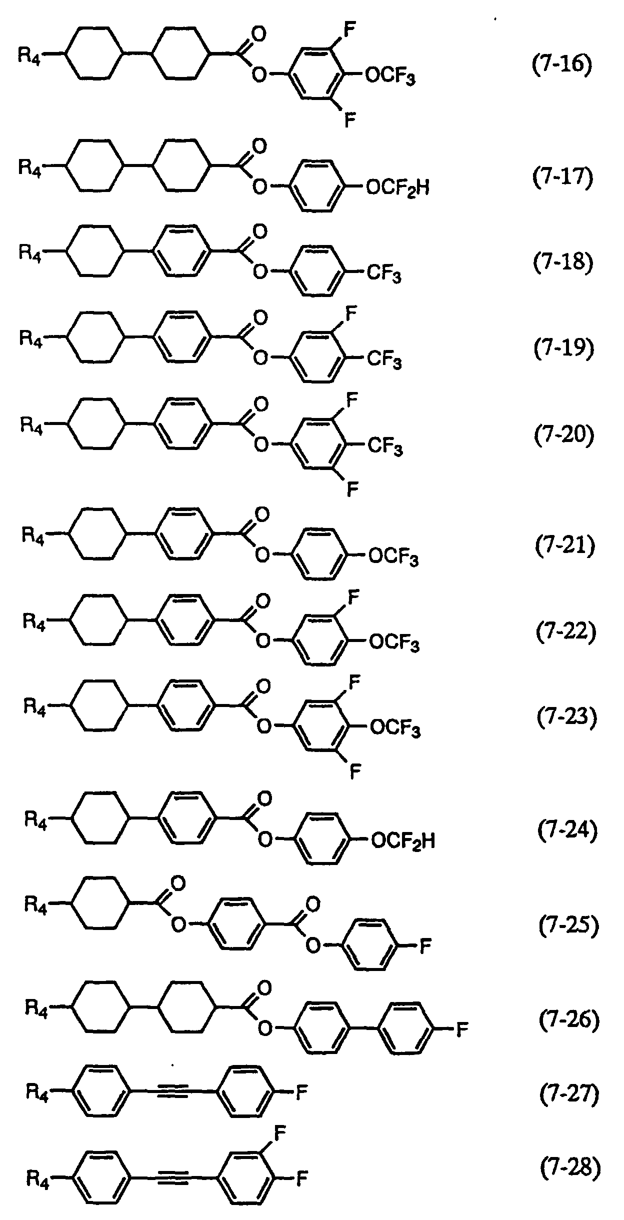 wherein R₂, R₃ and R₄ have the same meanings as defined above.

The compounds shown in the general formulae (5) to (7) have positive and large dielectric anisotropy values and are used as components in a composition for the purpose of particularly decreasing threshold voltage. Further, these compounds are also used for the purpose of regulating viscosity, regulating optical anisotropy, and widening the temperature range of liquid crystal phase or for the purpose of further improving steepness.

Among the second component B, (8-1) to (8-8) and (9-1) to (9-13) can be mentioned as preferable examples of compounds contained in the general formulae (8) and (9). 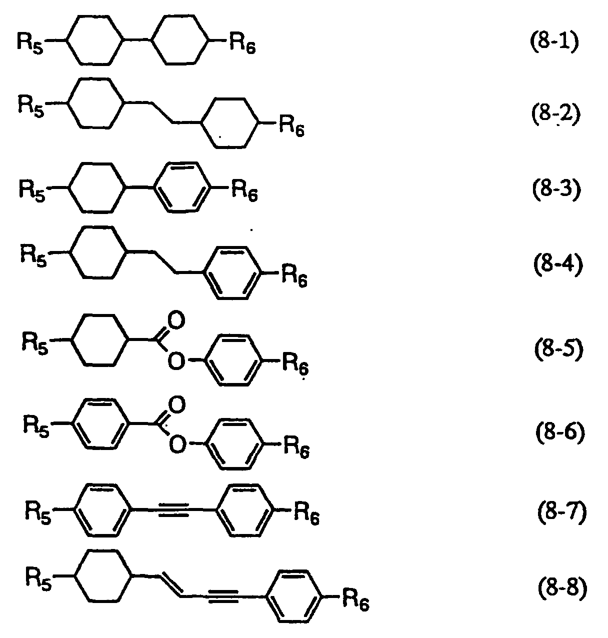 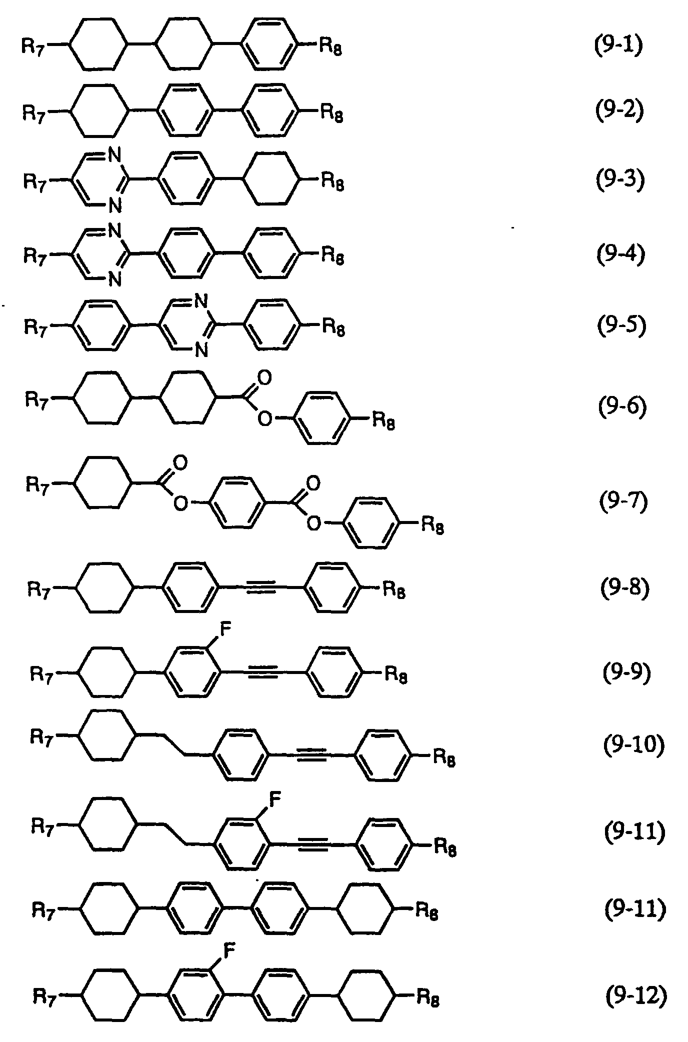 wherein R₅ and R₆ have the same meanings as defined above.

The compounds of the general formulae (8) and (9) are compounds having negative or slightly positive dielectric anisotropy values. The compounds of the general formula (8) are used mainly for the purpose of viscosity reduction and/or optical anisotropy control. Further, the compounds of the general formula (9) are used for the purpose of widening a nematic range such as raising clearing points and/or the purpose of regulating optical anisotropy values.

The compounds of the general formulae (5) to (9) are compounds essential for preparing liquid crystal compositions particularly for STN display system and conventional TN display system.

For preparing liquid crystal compositions for conventional TN display system and STN display system, the compounds of the general formulae (5) to (9) can be used in an arbitrary amount in the range of 1 to 99 % by weight, but these compounds are used preferably in the range of 10 to 97 % by weight, more preferably 40 to 95 % by weight. In this case, the compounds (2) to (4) may be partially used.

In the present invention, with the exception of the special case of liquid crystal compositions for OCB mode, optically active compounds are usually added for the purpose of inducing the helical structure of liquid crystal composition thus preparing necessary twisted nuclei and preventing reverse twist. For this purpose, any known optically active compounds can be used, but mention can be made of the following optically active compounds (Op-1) to (Op-8):

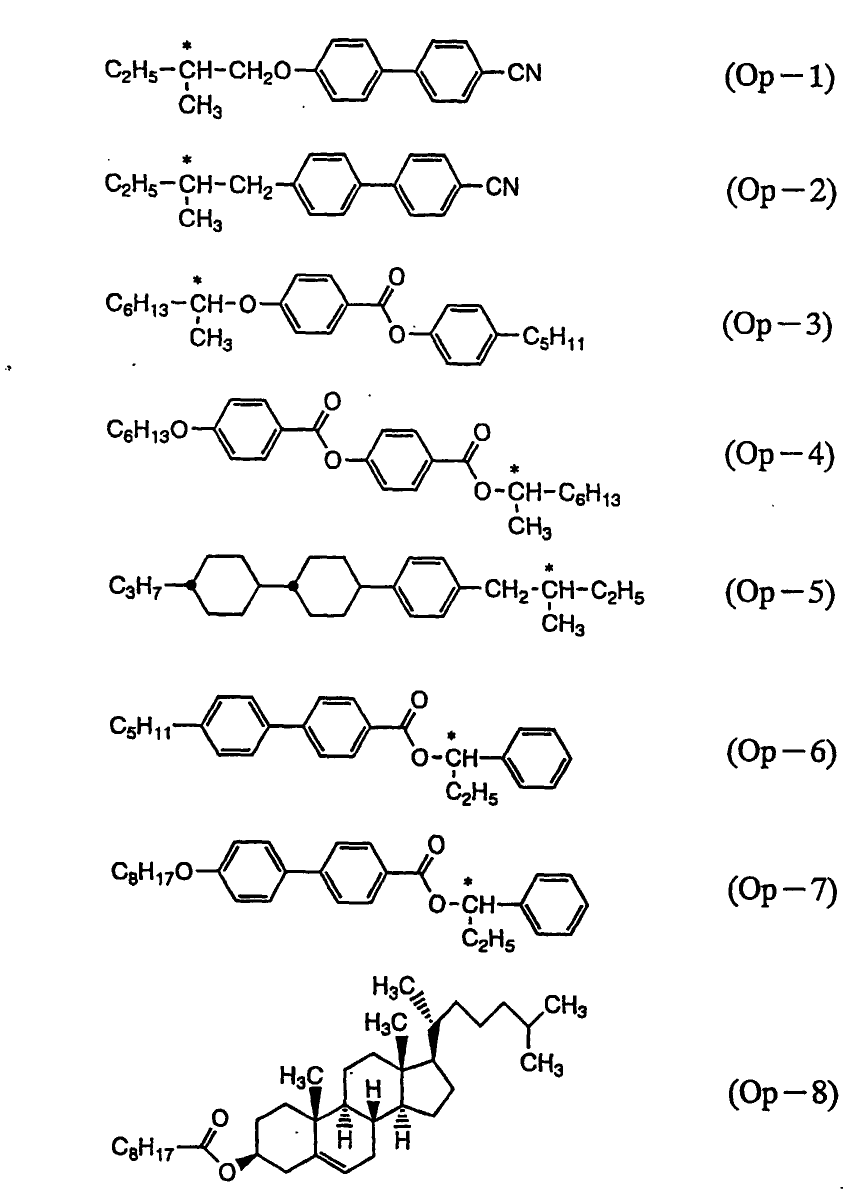

By using the liquid crystal compositions of the present invention in TFT liquid crystal display devices, steepness and viewing angles can be improved. Further, the compounds of the formula (1) are low viscous compounds, so the response rate of liquid crystal display devices using these compounds is improved and extremely high.

The liquid crystal compositions used in the present invention can be prepared in any method which is customary per se. Generally, a method of mutually dissolving various components at high temperature is used. Alternatively, the components may be mixed by dissolving them in an organic solvent in which liquid crystals are dissolved, and then the solvent may be distilled off under reduced pressure.

Further, the liquid crystal materials in the present invention are improved and optimized by using suitable additives depending on the intended use. Such additives are well-known to those skilled in the art and described in detail in literatures.

For example, the liquid crystal compositions of the present invention can be used as ones for guest-host (GH) mode by adding a dichroic dye such as merocyanine type, styryl type, azo type, azomethine type, azoxy type, quinophthalone type, anthraquinone type, and tetrazine type thereto. Alternatively, the liquid crystal compositions can be used as NCAP which is prepared by the microencapsulation of a nematic liquid crystal, or as liquid crystal compositions for polymer dispersed liquid crystal display devices (PDLCD) represented by polymer net work liquid crystal display devices (PNLCD) prepared by forming a polymer of three-dimensional reticulated structure in a liquid crystal. Still further, the liquid crystal compositions of the present invention can be used as ones for electrically controlled birefringence (ECB) mode or dynamic scattering (DS) mode.

As nematic liquid crystal compositions comprising the compound of the present invention, such composition examples (Composition Examples 1 through 25) as shown below can be mentioned. In each of the following Composition Examples, compounds are designated by abbreviations according to the definition shown in the following Table. Compound No. appended to the compounds of the present invention in the following Composition Examples means that the compounds are the same as those shown in Examples described below and having the same appended Compound No.

In the Composition Examples and the Examples, "%" means "% by weight" unless otherwise specified. Further, data of physical properties in Composition Examples are indicated in TNI (N-I phase transition temperature or clearing point: °C), η (viscosity: determined at 20.0 °C), Δn (optical anisotropy value: determined at 25.0 °C), Δε (dielectric anisotropy value: determined at 25.0 °C), and Vth (threshold voltage: determined at 25.0 °C).

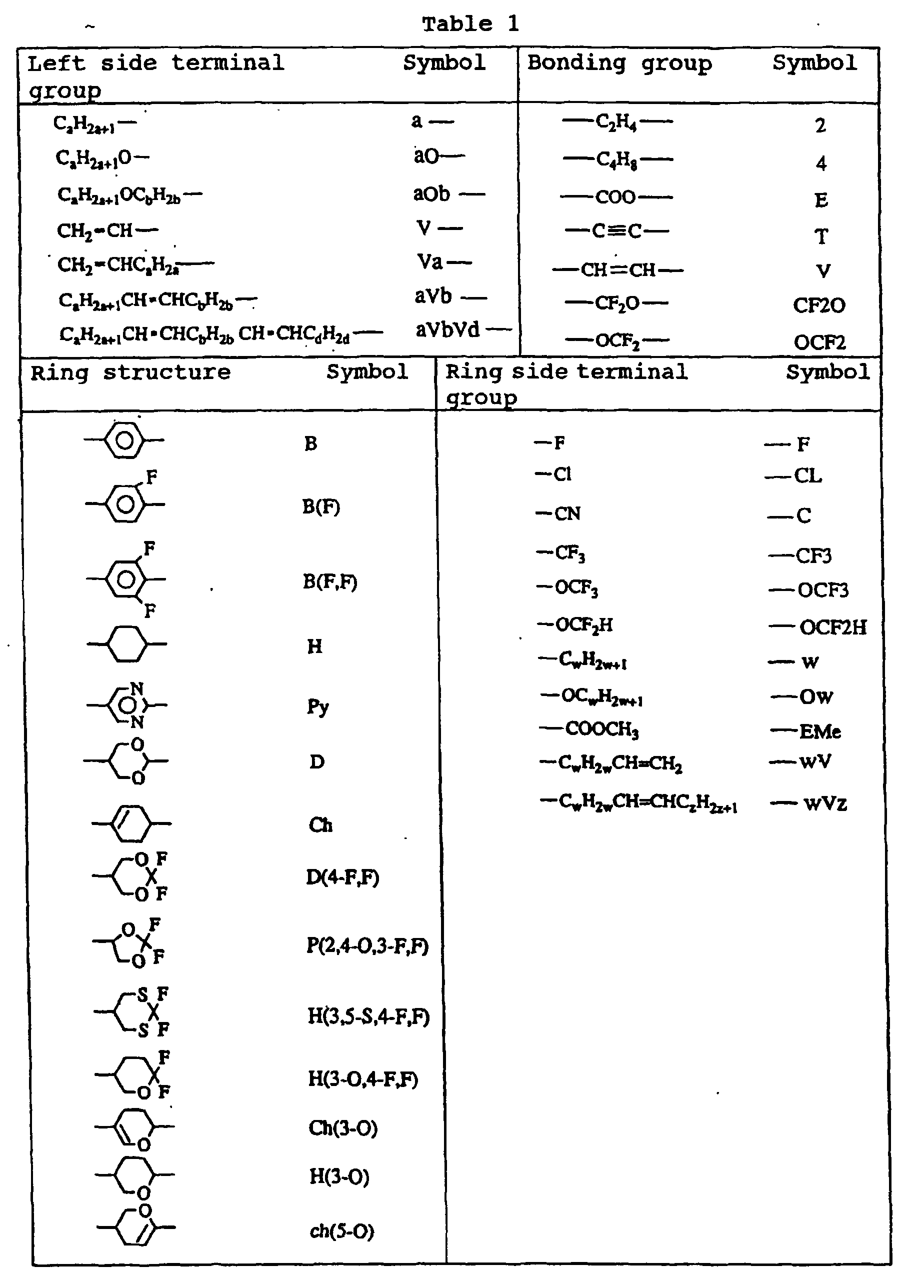

In the Table above, a, b, w and x represent an integer of 1 or more.

### Composition Example 1

| | |
|---|---|
| 5-HHD (4-F, F) (No. 31) | 4.0% |
| 1V2-BEB (F, F)-C | 12.0% |
| 3O1-BEB (F) -C | 12.0% |
| 2-HB-C | 12.0% |
| 3-HB-C | 19.0% |
| 2-HHB-C | 4.0% |
| 3-HHB-C | 5.0% |
| 4-HHB-C | 4.0% |
| 5-HHB-C | 4.0% |
| 3-HB-O2 | 7.0% |
| 3-HHB-1 | 7.0% |
| 3-H2BTB-2 | 4.0% |
| 3-H2BTB-3 | 3.0% |
| 3-H2BTB-4 | 3.0% |

### Composition Example 2

| | |
|---|---|
| 5-HHD (4-F, F) (No. 31) | 4.0% |
| 5-HHD-C (No. 251) | 4.0% |
| 3O1-BEB (F) -C | 12.0% |
| 1V2-BEB (F, F) -C | 15.0% |
| 3-HHEB-F | 5.0% |
| 5-HHEB-F | 5.0% |
| 3-HBEB-F | 6.0% |
| 3-HHB-F | 3.0% |
| 3-HB-O2 | 10.0% |
| 3-HH-EME | 5.0% |
| 3-HHB-1 | 6.0% |
| 3-HHB-3 | 8.0% |
| 3-HHB-O1 | 4.0% |
| 3-HB (F) VB-2 | 4.0% |
| 3-HB (F) VB-3 | 4.0% |
| 3-HB (F) VB-4 | 5.0% |

### Composition Example 3

| | |
|---|---|
| 3-DVHB-C (No. 383) | 7.0% |
| 5-DVHB-C (No. 384) | 7.0% |
| V2-HB-C | 9.0% |
| 1V2-HB-C | 9.0% |
| 3-HB-C | 10.0% |
| 1O1-HB-C | 8.0% |
| 2O1-HB-C | 4.0% |
| 3-HH-4 | 10.0% |
| 1O1-HH-5 | 8.0% |
| 2-BTB-O1 | 11.0% |
| 3-HHB-3 | 9.0% |
| 3-HB (F) TB-2 | 5.0% |
| 3-HB (F) TB-3 | 3.0% |

### Composition Example 4

| | |
|---|---|
| 5-HHD-C (No. 251) | 6.0% |
| 2-HB (F) -C | 12.0% |
| 3-HB (F) -C | 12.0% |
| 3-HHB (F) -C | 4.0% |
| 3-HB-O2 | 10.0% |
| 3-HH-4 | 10.0% |
| 2-BTB-O1 | 10.0% |
| 3-HHB-1 | 8.0% |
| 3-HHB-3 | 16.0% |
| 3-HHB-O1 | 4.0% |
| 3-H2BTB-2 | 4.0% |
| 3-H2BTB-3 | 4.0% |

### Composition Example 5

| | |
|---|---|
| 5-HHD-C (No. 251) | 5.0% |
| 3-DVHB-C (No. 383) | 5.0% |
| 3-DB-C | 10.0% |
| 4-DB-C | 10.0% |
| 2-BEB-C | 6.0% |
| 3-HHEBB-C | 3.0% |
| 3-HBEBB-C | 3.0% |
| 5-HBEBB-C | 3.0% |
| 3-PyB (F) -F | 6.0% |
| 3-HEB-O4 | 10.2% |
| 4-HEB-O2 | 7.7% |
| 5-HEB-O1 | 7.7% |
| 3-HEB-O2 | 6.4% |
| 5-HEB-O2 | 5.0% |
| 5-HEB-2 | 3.0% |
| 1O-BEB-2 | 3.0% |
| 3-HEBEB-1 | 3.0% |
| 3-HEBEB-F | 3.0% |

### Composition Example 6

| | |
|---|---|
| 3-H2P (2, 4-O, 3-F, F) (No. 86) | 3.0% |
| 5-HCh (3-O) -C (No. 181) | 3.0% |
| 5-HH (3-O) -C (No. 221) | 4.0% |
| 2-BB-C | 8.0% |
| 2-HB-C | 10.0% |
| 3-HB-C | 13.0% |
| 2-HHB-C | 4.0% |
| 3-HHB-C | 6.0% |
| 5-PyB-F | 6.0% |
| 3-PyBB-F | 6.0% |
| 2-BTB-1 | 3.0% |
| 3-HHB-1 | 8.0% |
| 3-HHB-3 | 15.0% |
| 3-HHB-O1 | 5.0% |
| 2-PyBH-3 | 3.0% |
| 4-PyBB-2 | 3.0% |

### Composition Example 7

| | |
|---|---|
| 5-HHD (4-F, F) (No. 31) | 4.0% |
| 3-DVHB-C (No. 383) | 6.0% |
| 3-HB-C | 10.0% |
| V2-HB-C | 10.0% |
| 1V2-HB-C | 10.0% |
| 1-BTB-3 | 7.0% |
| 2-BTB-1 | 7.0% |
| 3-PyB-2 | 3.0% |
| 2-PyB-O2 | 3.0% |
| 3-HH-4 | 4.0% |
| 3-H2BTB-2 | 4.0% |
| 3-H2BTB-3 | 4.0% |
| 3-H2BTB-4 | 4.0% |
| 3-HB (F) TB-2 | 8.0% |
| 3-HB (F) TB-3 | 5.0% |
| 3-HB (F) TB-4 | 8.0% |
| 1O1-HBBH-3 | 3.0% |

### Composition Example 8

| | |
|---|---|
| 5-HHD (4-F, F) (No. 31) | 5.0% |
| 7-HB (F, F) -F | 4.0% |
| 3-H2HB (F, F) -F | 12.0% |
| 4-H2HB (F, F) -F | 10.0% |
| 5-H2HB (F, F) -F | 10.0% |
| 3-HHB (F, F) -F | 10.0% |
| 3-HH2B (F, F) -F | 13.0% |
| 5-HH2B (F, F) -F | 10.0% |
| 3-HBB (F, F) -F | 10.0% |
| 5-HBB (F, F) -F | 10.0% |
| 3-HHBB (F, F) -F | 3.0% |
| 5-HH2BB (F, F) -F | 3.0% |

### Composition Example 9

| | |
|---|---|
| 2-HHD (4-F, F) (No. 33) | 4.0% |
| 3-HHD (4-F, F) (No. 32) | 4.0% |
| 5-HHD (4-F, F) (No. 31) | 4.0% |
| 7-HB (F, F) -F | 5.0% |
| 3-H2HB (F, F) -F | 5.0% |
| 5-H2HB (F, F) -F | 5.0% |
| 3-HH2B (F, F) -F | 5.0% |
| 3-HBB (F, F) -F | 20.0% |
| 5-HBB (F, F) -F | 20.0% |
| 2-HBEB (F, F) -F | 3.0% |
| 3-HBEB (F, F) -F | 5.0% |
| 5-HBEB (F, F) -F | 3.0% |
| 3-HHEB (F, F) -F | 10.0% |
| 4-HHEB (F, F) -F | 3.0% |
| 5-HHEB (F, F) -F | 4.0% |

### Composition Example 10

| | |
|---|---|
| 3-H2P (2, 4-O, 3-F, F) (No. 86) | 3.0% |
| 5-HHH (3, 5-S, 4-F, F) (No. 31) | 4.0% |
| 5-H2B (F) -F | 4.0% |
| 7-HB (F) -F | 4.0% |
| 2-HHB (F) -F | 12.0% |
| 3-HHB (F) -F | 12.0% |
| 5-HHB (F) -F | 12.0% |
| 2-H2HB (F) -F | 4.0% |
| 3-H2HB (F) -F | 2.0% |
| 5-H2HB (F) -F | 4.0% |
| 3-H2HB (F, F) -F | 6.0% |
| 4-H2HB (F, F) -F | 5.0% |
| 5-H2HB (F, F) -F | 5.0% |
| 3-HHB (F, F) -F | 8.0% |
| 3-HH2B (F, F) -F | 8.0% |
| 5-HH2B (F, F) -F | 7.0% |

### Composition Example 11

| | |
|---|---|
| 4-Hch (5-O) -CL (No. 314) | 4.0% |
| 5-BBH (3-O, 4-F, F) (No. 74) | 4.0% |
| 5-HB-CL | 4.0% |
| 4-H2BB (F) -F | 4.0% |
| 2-HBB (F) -F | 7.0% |
| 3-HBB (F) -F | 7.0% |
| 5-HBB (F) -F | 14.0% |
| 2-HHB-CL | 5.0% |
| 4-HHB-CL | 10.0% |
| 5-HHB-CL | 4.0% |
| 3-HBB (F, F) -F | 15.0% |
| 5-HBB (F, F) -F | 12.0% |
| 5-H2BB (F, F) -F | 5.0% |
| 3-HBB-F | 3.0% |
| 5-HHEBB-F | 2.0% |

### Composition Example 12

| | |
|---|---|
| 3-HHD (4-F, F) (No. 32) | 3.0% |
| 5-HHD (4-F, F) (No. 31) | 3.0% |
| 5-HEB-F | 5.0% |
| 7-HEB-F | 5.0% |
| 2-HHB (F) -F | 8.0% |
| 3-HHB- (F) -F | 8.0% |
| 5-HHB (F) -F | 8.0% |
| 3-H2HB (F, F) -F | 8.0% |
| 5-H2HB (F, F) -F | 8.0% |
| 3-HH2B (F, F) -F | 8.0% |
| 5-HH2B (F, F) -F | 5.0% |
| 3-HBB (F, F) -F | 8.0% |
| 3-HB-O2 | 10.0% |
| 3-HH-4 | 5.0% |
| 3-HHB-1 | 6.0% |
| 1O1-HBBH-3 | 2.0% |

### Composition Example 13

| | |
|---|---|
| 3-HDD (4-F, F) (No. 32) | 5.0% |
| 5-HHD (4-F, F) (No. 31) | 5.0% |
| 5-HB-F | 7.0% |
| 7-HB-F | 9.0% |
| 5-HB-3 | 5.0% |
| 3-HB-O1 | 5.0% |
| 3-HHB-OCF3 | 6.0% |
| 4-HHB-OCF3 | 5.0% |
| 5-HHB-OCF3 | 5.0% |
| 3-HHEB-OCF3 | 2.0% |
| 3-HH2B-OCF3 | 3.0% |
| 5-HH2B-OCF3 | 3.0% |
| 3-HH2B-F | 3.0% |
| 5-HH2B-F | 3.0% |
| 3-HBB (F) -F | 6.0% |
| 5-HBB (F) -F | 5.0% |
| 3-HH2B (F) -F | 7.0% |
| 5-HH2B (F) -F | 9.0% |
| 3-HB (F, F) B (F) -F | 2.0% |
| 3-HB (F) BH-3 | 3.0% |
| 5-HB (F) BH-3 | 2.0% |

### Composition Example 14

| | |
|---|---|
| 3-H2P (2, 4-O, 3-F, F) (No. 86) | 6.0% |
| 5-BBH (3-O, 4-F, F) (No. 74) | 3.0% |
| 5-HHH (3, 5-S, 4-F, F) (No. 111) | 3.0% |
| 7-HB-F | 6.0% |
| 3-HHB-OCF2H | 5.0% |
| 5-HHB-OCF2H | 4.0% |
| 3-HHB (F, F) -OCF2H | 7.0% |
| 5-HHB (F, F) -OCF2H | 13.0% |
| 2-HHB-OCF3 | 8.0% |
| 3-HHB-OCF3 | 9.0% |
| 5-HHB-OCF3 | 10.0% |
| 3-HH2B (F) -F | 8.0% |
| 5-HH2B (F) -F | 8.0% |
| 3-HHEB (F) -F | 5.0% |
| 5-HHEB (F) -F | 5.0% |

### Composition Example 15

| | |
|---|---|
| 5-HHD-C (No. 251) | 4.0% |
| 3-DVHB-C (No. 383) | 6.0% |
| V-HB-C | 10.0% |
| 1V-HB-C | 5.0% |
| 3-BB-C | 4.0% |
| 5-BB-C | 3.0% |
| 4-BB-3 | 3.0% |
| 3-H2B-O2 | 5.0% |
| 5-H2B-O2 | 8.0% |
| 3-BEB-C | 5.0% |
| 5-HEB-O1 | 8.0% |
| 5-HEB-O3 | 8.0% |
| 5-BBB-C | 5.0% |
| 4-BPyB-C | 4.0% |
| 4-BPyB-5 | 4.0% |
| 5-HB2B-4 | 5.0% |
| 5-HBB2B-3 | 3.0% |
| 1V-HH-1O1 | 5.0% |
| 1V2-HBB-3 | 5.0% |

### Composition Example 16

| | |
|---|---|
| 5-HHD (4-F, F) (No. 31) | 5.0% |
| 5-HCh (3-O) -C (No. 181) | 5.0% |
| 4-HEB (F) -F | 5.0% |
| 5-HEB (F) -F | 10.0% |
| 2-BEB (F) -C | 5.0% |
| 3-BEB (F) -C | 5.0% |
| 4-BEB (F) -C | 8.0% |
| 5-BEB (F) -C | 8.0% |
| 1O3-HB (F) -C | 6.0% |
| 3-HHEB (F) -F | 5.0% |
| 2-HBEB (F) -C | 5.0% |
| 3-HBEB (F) -C | 5.0% |
| 4-HBEB (F) -C | 5.0% |
| 5-HBEB (F) -C | 5.0% |
| 3-HBTB-2 | 8.0% |
| V2-HH-3 | 3.0% |
| V-HH-4 | 3.0% |
| V2-HHB-1 | 4.0% |

### Composition Example 17

| | |
|---|---|
| 2-HD-V (No. 374) | 8.0% |
| 3-HD-V (No. 373) | 8.0% |
| 4-HD-V (No. 372) | 8.0% |
| 5-HD-V (No. 371) | 10.0% |
| 5-HHB (F) -F | 16.0% |
| 2-H2HB (F) -F | 10.0% |
| 3-H2HB (F) -F | 5.0% |
| 5-H2HB (F) -F | 10.0% |
| 2-HBB (F) -F | 6.0% |
| 3-HBB (F) -F | 6.0% |
| 5-HBB (F) -F | 13.0% |
| TNI =85.4 (°C) | |
| η =22.0 (mPa·s) | |
| Δn =0.081 | |
| Vth =2.63 (V) | |

The pitch of a composition containing 0.3 part by weight of optically active compound (OP-8) added to 100 parts by weight of the above composition was 78.0 µm.

### Composition Example 18

| | |
|---|---|
| 3-HD-V (No. 373) | 5.0% |
| 4-HD-V (No. 372) | 5.0% |
| 5-HD-V (No. 371) | 4.0% |
| 7-HB-CL | 4.0% |
| 1O1-HH-5 | 5.0% |
| 2-HBB (F) -F | 8.0% |
| 3-HBB (F) -F | 8.0% |
| 5-HBB (F) -F | 14.0% |
| 4-HHB-CL | 8.0% |
| 5-HHB-CL | 8.0% |
| 3-H2HB (F) -CL | 4.0% |
| 3-HBB (F, F) -F | 10.0% |
| 5-H2BB (F, F) -F | 9.0% |
| 3-HB (F) VB-2 | 4.0% |
| 3-HB (F) VB-3 | 4.0% |
| TNI =99.2 (°C) | |
| η =24.6 (mPa·s) | |
| Δn =0.127 | |
| Vth=2.46 (V) | |

### Composition Example 19

| | |
|---|---|
| 2-HD-V (No. 374) | 4.0% |
| 3-HD-V (No. 373) | 4.0% |
| 4-HD-V (No. 372) | 4.0% |
| 5-HD-V (No. 371) | 4.0% |
| 3-HHB (F, F) -F | 9.0% |
| 3-H2HB (F, F) -F | 8.0% |
| 3-HBB (F, F) -F | 21.0% |
| 5-HBB (F, F) -F | 20.0% |
| 3-H2BB (F, F) -F | 10.0% |
| 5-HHBB (F, F) -F | 3.0% |
| 5-HHEBB-F | 2.0% |
| 3-HH2BB (F, F) -F | 3.0% |
| 1O1-HBBH-4 | 4.0% |
| 1O1-HBBH-5 | 4.0% |
| TNI =83.6 (°C) | |
| η =32.6 (mPa·s) | |
| Δn =0.114 | |
| Vth =1.89 (V) | |

### Composition Example 20

| | |
|---|---|
| 3-HD-V (No. 373) | 5.0% |
| 5-HD-V (No. 371) | 5.0% |
| 5-HB-F | 12.0% |
| 6-HB-F | 9.0% |
| 7-HB-F | 7.0% |
| 2-HHB-OCF3 | 7.0% |
| 3-HHB-OCF3 | 7.0% |
| 4-HHB-OCF3 | 7.0% |
| 5-HHB-OCF3 | 5.0% |
| 3-HH2B-OCF3 | 4.0% |
| 5-HH2B-OCF3 | 4.0% |
| 3-HHB (F, F) -OCF3 | 5.0% |
| 3-HBB (F) -F | 10.0% |
| 3-HH2B (F) -F | 3.0% |
| 3-HB (F) BH-3 | 3.0% |
| 5-HBBH-3 | 3.0% |
| 3-HHB (F, F) -OCF2H | 4.0% |
| TNI =80.4 (°C) | |
| η =13.8 (mPa·s) | |
| Δn =0.084 | |
| Vth =2.56 (V) | |

### Composition Example 21

| | |
|---|---|
| 3-HD-V (No. 373) | 7.0% |
| 5-H2HB (F, F) -F | 8.0% |
| 3-HHB (F, F) -F | 10.0% |
| 4-HHB (F, F) -F | 5.0% |
| 3-HH2B (F, F) -F | 9.0% |
| 5-HH2B (F, F) -F | 9.0% |
| 3-HBB (F, F) -F | 15.0% |
| 5-HBB (F, F) -F | 15.0% |
| 3-HBEB (F, F) -F | 2.0% |
| 4-HBEB (F, F) -F | 2.0% |
| 5-HBEB (F, F) -F | 2.0% |
| 3-HHEB (F, F) -F | 10.0% |
| 4-HHEB (F, F) -F | 3.0% |
| 5-HHEB (F, F) -F | 3.0% |
| TNI =79.5 (°C) | |
| η =30.9 (mPa·s) | |
| Δn =0.092 | |
| Vth =1.89 (V) | |

### Composition Example 22

| | |
|---|---|
| 3-HD-V (No. 373) | 5.0% |
| 5-HD-V (No. 371) | 5.0% |
| 3-HEB-F | 11.5% |
| 5-HEB-F | 11.5% |
| 3-HH-EMe | 25.0% |
| 3-HHEB (F, F) -F | 5.0% |
| 3-HHEB-F | 5.0% |
| 5-HHEB-F | 5.0% |
| 2-HBB (F) -F | 5.5% |
| 3-HBB (F) -F | 5.5% |
| 5-HBB (F) -F | 11.0% |
| 3-HBB (F, F) -F | 5.0% |
| TNI =71.2 (°C) | |
| η =20.6 (mPa·s) | |
| Δn =0.080 | |
| Vth =2.38 (V) | |

### Composition Example 23

| | |
|---|---|
| 3-HD-V (No. 373) | 10.0% |
| 1V2-BEB (F, F) -C | 5.0% |
| 3-HB-C | 25.0% |
| 1-BTB-3 | 5.0% |
| 3-HH-4 | 11.0% |
| 3-HHB-1 | 11.0% |
| 3-HHB-3 | 9.0% |
| 3-H2BTB-2 | 4.0% |
| 3-H2BTB-3 | 4.0% |
| 3-H2BTB-4 | 4.0% |
| 3-HB (F) TB-2 | 6.0% |
| 3-HB (F) TB-3 | 6.0% |
| TNI =95.2 (°C) | |
| η =17.6 (mPa·s) | |
| Δn =0.144 | |
| Vth =2.15 (V) | |

The pitch of a composition containing 0.8 part by weight of optically active compound (OP-4) added to 100 parts by weight of the above composition was 11.5 µm.

### Composition Example 24

| | |
|---|---|
| 3-HD-V (No. 373) | 10.0% |
| 2-HB-C | 5.0% |
| 3-HB-C | 12.0% |
| 3-HB-O2 | 5.0% |
| 2-BTB-1 | 3.0% |
| 3-HHB-1 | 8.0% |
| 3-HHB-F | 4.0% |
| 3-HHB-O1 | 5.0% |
| 3-HHB-3 | 14.0% |
| 3-HHEB-F | 4.0% |
| 5-HHEB-F | 4.0% |
| 2-HHB (F) -F | 7.0% |
| 3-HHB (F) -F | 7.0% |
| 5-HHB (F) -F | 7.0% |
| 3-HHB (F, F) -F | 5.0% |
| TNI =104.3 (°C) | |
| η =20.9 (mPa·s) | |
| Δn =0.099 | |
| Vth =2.64 (V) | |

### Composition Example 25

| | |
|---|---|
| 3-HD-V (No. 373) | 5.0% |
| 3-BEB (F) -C | 8.0% |
| 3-HB-C | 3.0% |
| V-HB-C | 8.0% |
| 1V-HB-C | 8.0% |
| 3-HB-O2 | 3.0% |
| 3-HH-2V | 14.0% |
| 3-HH-2V1 | 7.0% |
| V2-HHB-1 | 15.0% |
| 3-HHB-1 | 5.0% |
| 3-HHEB-F | 7.0% |
| 3-H2BTB-2 | 6.0% |
| 3-H2BTB-3 | 6.0% |
| 3-H2BTB-4 | 5.0% |
| TNI =99.4 (°C) | |
| η =15.8 (mPa·s) | |
| Δn =0.129 | |
| Vth =2.50 (V) | |

### BEST MODE FOR CARRYING OUT THE INVENTION

Hereinafter, the present invention is described in more detail by reference to the Examples. In the Examples, C is crystal, S is smectic phase, N is nematic phase, I is isotropic liquid phase, and phase transition point is expressed in °C.

### Example 1

Production of 5-(4-(4-pentylcyclohexyl)cyclohexyl)-2,2-difluoro-1,3-dioxane (the compound (No. 31) of formula (1) wherein R = pentyl group, m1 = m2 = 0, m3 = 1, n1 = n2 = n3 = n4 = 0, ring A3 = ring A4 = 1,4-cyclohexylene group, G = G1, Q₁ = Q₄ = -CH₂-, Q₂ = Q₃ = oxygen atom, Y₁ = Y₂ = fluorine atom).

### (First step)

14.2 g (355 mmol) of sodium hydride (60 %) was added to 560 ml dimethoxyethane, and 80.0 g (356 mmol) of diethylphosphonoacetic acid ethyl ester was added dropwise thereto, and the mixture was further stirred at room temperature. After evolution of hydrogen gas was finished, 300 ml solution of 70.6 g (282 mmol) of 4-(4-pentylcyclohexyl)cyclohexanone in dimethoxyethane was added dropwise thereto such that the liquid temperature did not exceed 30 °C, and the mixture was stirred for 5 hours at room temperature. The reaction mixture was added little by little to 500 ml water, and the product was extracted with 800 ml diethyl ether and then with 400 ml ethyl acetate, and the organic layer was washed with 3 N of 500 ml hydrochloric acid, 500 ml saturated aqueous sodium bicarbonate and 600 ml saline solution in this order and dried over magnesium sulfate, and the solvent was distilled off. 73 g of the residue was hydrogenated in 750 ml ethanol in the presence of 5 % Pd/C catalyst (3.0 g). After the reaction was finished, the catalyst was separated off, and the ethanol was distilled off under reduced pressure. The residue was purified by column chromatography with heptane/ethyl acetate = 3/1 as the eluent and then recrystallized from heptane to give 19.6 g (63.1 mmol) of a trans form of 4-(4-pentylcyclohexyl)cyclohexyl acetic acid ethyl ester. Its yield was 22.4 %.

### (Second step)

19.6 g (63.1 mmol) of the above 4-(4-pentylcyclohexyl)cyclohexyl acetic acid ethyl ester was dissolved in 100 ml tetrahydrofuran (abbreviated hereinafter to THF), and this solution was cooled to 0 °C, and 37.5 ml (75 mmol) of lithium diisopropylamide (abbreviated hereinafter to LDA) (2.0 mol/l) was added dropwise thereto such that the liquid temperature did not exceed 10 °C, and the mixture was stirred for 1 hour. A mixture of 6.80 g (37.9 mmol) of hexamethylphosphoramide and 7.56 g (80.0 mmol) of methyl chlorocarbonate was added dropwise thereto and stirred at room temperature for 1 hour. The reaction solution was added little by little to 100 ml of saturated aqueous ammonium chloride, and the product was extracted with ethyl acetate. Then, the organic layer was washed with saturated aqueous sodium bicarbonate and thenwith water and dried over magnesium sulfate. The solvent was distilled off, and the residue was purified by column chromatography with heptane/ethyl acetate = 3/1 with the eluent and recrystallized from heptane to give 13.4 g (35.3 mmol) of 4-(4-pentylcyclohexyl)cyclohexyl malonic acid methyl ester. Its yield was 55.9 %.

### (Third step)

30 ml THF was added dropwise to 2.68 g (70.6 mmol) of lithium aluminum hydride (abbreviated hereinafter to LAH) at 0 °C and then cooled to -20 °C, and 300 ml solution of 13.4 g (35.3 mmol) of the above 4-(4-pentylcyclohexyl)cyclohexyl malonic acid methyl ester in THF was added dropwise thereto such that the liquid temperature did not exceed -10 °C, and the mixture was stirred for 15 minutes, and the reaction solution was warmed to room temperature and further stirred for 1 hour. The reaction solution was cooled to 0 °C. After 100 ml of 1 N hydrochloric acid was added dropwise thereto, 100 ml of 6 N hydrochloric acid was added thereto, and the product was extracted with ethyl acetate, and the organic layer was washed with water and dried over magnesium sulfate, and the solvent was distilled off. The residue was recrystallized from heptane to give 10.0 g (32.3 mmol) of 2-(4-(4-pentylcyclohexyl)cyclohexyl)-1,3-propanediol. Its yield was 91.5 %.

### (Fourth step)

10.0 g (32.3 mmol) of the above 2-(4-(4-pentylcyclohexyl)cyclohexyl)-1,3-propanediol and 5.50 g (47.8 mmol) of thiophosgene were added to 200 ml benzene, and the mixture was stirred at 0 °C for 5 minutes, and 10 ml pyridine was added dropwise to this suspension at the same temperature and stirred at room temperature for 2 hours. The reaction solution was washed with water, and the organic layer was dried over magnesium sulfate, and the solvent and thiophosgene were distilled off under reduced pressure. The residue was purified by column chromatography with heptane/ethyl acetate = 1/1 as the eluent and then recrystallized from ethyl acetate to give 4.30 g (12.2 mmol) of 5-(4-(4-pentylcyclohexyl)cyclohexyl)-1,3-dioxane-2-thione. Its yield was 38.1 %.

### (Fifth step)

0.4 mol/l dihydrotrifluorotetrabutyl ammonium (abbreviated hereinafter to TBAH₂F₃) in 1,2-dichloroethane was prepared, and to 32.3 ml (12.9 mmol) of this solution was added 1.52 g (4.31 mmol) of the above 5-(4-(4-pentylcyclohexyl)cyclohexyl)-1,3-dioxane-2-thione at room temperature, and the mixture was stirred. 2.33 g (10.4 mmol) of N-iodinated succinic acid imide was added to this solution and stirred at the same temperature for 45 minutes, and the reaction mixture was decanted 3 times with 100 ml of a mixed solvent of heptane/diethyl ether = 1/1. The solvent was distilled off under reduced pressure, and the residue was purified by column chromatography with heptane/ethyl acetate = 8/1 as the eluent to give 0.12 g (0.33 mmol) of 5-(4-(4-pentylcyclohexyl)cyclohexyl)-2,2-difluoro-1,3-dioxane. Its yield was 7.6 %.
1H-NMR (CDCl3) δ (ppm): 4.41-3.95 (m, 4H), 1.99-0.90 (m, 32H).

### Example 2

Production of 5-(4-(4-pentylphenyl)phenyl)-2,2-difluorotetrahydropyrane (the compound (No. 74) of formula (1) wherein R = pentyl group, m1 = m2 = 0, m3 = 1, n1 = n2 = n3 = n4 = 0, ring A3 = ring A4 = 1,4-phenylene, G = G1, Q₁ = Q₂ = Q₄ = -CH₂-, Q₃ = oxygen atom, Y₁ = Y₂ = fluorine atom).

### (First step)

500 ml TNF solution containing 78.0 g (500 mmol) of 6,10-dioxaspiro[4.5]decane-3-one synthesized at room temperature from cyclopentane dione was added dropwise to 1 L of dry THF containing a Grignard reagent prepared at room temperature from magnesium and 167 g (551 mmol) of 4-(4-pentylphenyl)bromobenzene, and the mixture was stirred at room temperature for 5 hours. The reaction solution was added to 1 L of 6 N hydrochloric acid, and the product was extracted with ethyl acetate. The extract was washed with saturated aqueous sodium bicarbonate and then with water and dried over magnesium sulfate anhydride, and the ethyl acetate was distilled off under reduced pressure. The residue was dissolved in 1 L of toluene, and 5 g ion exchange resin (Amberlist) was added thereto, and the mixture was heated for 5 hours under reflux. The ion exchange resin was filtered off, and the filtrate was washed with saturated aqueous sodium bicarbonate and then with water and dried over magnesium sulfate anhydride, and the toluene was distilled off under reduced pressure. The residue was purified by column chromatography with a mixed solvent of heptane/ethyl acetate = 3/1 as the eluent. The purified material thus obtained was dissolved in 500 ml of a mixed solvent of ethanol/ethyl acetate (1 : 1) and hydrogenated by adding 20 g of 10 % Raney catalyst (Raney ND HT-90, Kawaken Fine Chemical K. K.). After the catalyst was filtered off, the filtrate was concentrated. This concentrate was dissolved in 500 ml toluene, and 50 ml formic acid was added thereto and heated for 5 hours under reflux. The reaction solution was washed with water, saturated aqueous sodium bicarbonate and a saline solution in this order and dried over magnesium anhydride, and then the toluene was distilled off under reduced pressure whereby 58.3 g (190 mmol) of 3-(4-(4-pentylphenyl)phenyl)cyclopentanone was obtained. Its yield from 6,10-dioxaspiro[4.5]decane-3-one was 38.0 %.

### (Second step)

81 g (570 mmol) of sodium dihydrogen phosphate and 39.2 g (227 mmol) of m-chloroperbenzoic acid (abbreviated hereinafter to mCPBA) were added to 1.5 L solution of 58.3 g (190 mmol) of 3-(4-(4-pentylphenyl)phenyl)cyclopentanone in dichloroethane, and the mixture was stirred for 24 hours. The reaction solution was filtered through Celite, and the filtrate was washed with sodium thiosulfate, saturated aqueous sodium bicarbonate and a saline solution in this order and dried over magnesium sulfate. The solvent was distilled off under reduced pressure, and the resulting residue was purified by column chromatography with a mixed solvent of heptane/ethyl acetate = 3/1. The purified material thus obtained was recrystallized from a mixed solvent of heptane/ethyl acetate = 5/1 to give 5.5 g (17.1 mmol) of 4-(4-pentylphenyl)phenyl-5-pentanolide. Its yield from 3-(4-(4-pentylphenyl)phenyl)cyclopentanone was 9.0 %.

### (Third step)

6.9 g (17.1 mmol) of a commercial Lawesson's reagent was added to 50 ml solution of 5.5 g (17.1 mmol) of the above 4-(4-pentylphenyl)phenyl-5-pentanolide in toluene, and the mixture was refluxed for 48 hours. The reaction product was filtered and the filtrate was concentrated, and the residue was purified by column chromatography with a mixed solvent of heptane/ethyl acetate = 3/1, and the purified material thus obtained was recrystallized from a mixed solvent of heptane/ethyl acetate = 5/1 to give 0.75 g (2.2 mmol) of 4-(4-pentylphenyl)phenyl thionolactone. Its yield from 4-(4-pentylphenyl)phenyl-5-pentanolide was 12.9 %.

### (Fourth step)

3 ml solution of 0.71 g (4.4 mmol) of DAST in dichloromethane was added dropwise to 5 ml solution of 0.75 g (2.2 mmol) of the above 4-(4-pentylphenyl)phenyl thionolactone in dichloromethane, and the mixture was stirred at the same temperature for 10 hours. The reaction solution was cooled to 0 °C, and after 10 ml saturated aqueous sodium bicarbonate was added dropwise thereto, the purified material was extracted with dichloromethane. After the extract was dried over sodium sulfate, the solvent was distilled off under reduced pressure, and the residue was purified by column chromatography with a mixed solvent of heptane/ethyl acetate = 3/1 and the resulting purified material was recrystallized from a mixed solvent of heptane/ethyl acetate = 5/1 to give 0.10 g (0.29 mmol) of 5-(4-(4-pentylphenyl)phenyl)-2,2-difluoroxane. Its yield from 4-(4-pentylphenyl)phenyl thionolactone was 13.2 %.
1H-NMR data supported its structure well.
MS: 344 (M+)

### Example 3

Production of 3-(4-pentylcyclohexyl)-6-cyano-4H,5H-dihydropyrane (the compound (No. 181) of formula (1) wherein R = pentyl group, m1 = m2 = m3 = 0, n1 = n2 = n3 = n4 = 0, ring A4 = 1,4-cyclohexylene, G = G2, Q₃ = Q₄ = -CH₂-, Q₅ = =CH-, Q₂ = oxygen atom, Y₁ = cyano group, Y₂ = hydrogen atom).

### (First step)

211 g (1.2 mol) of chloroiodomethane and 120 g (554 mmol) of 4-pentylcyclohexyl carboxylate chloride were added to 700 ml THF in an argon atmosphere and cooled to -78 °C, and 104 g (1.2 mol) of lithium bromide was added thereto, and the mixture was stirred for 5 minutes. 1.2 L solution of 1 M methyl lithium in diethyl ether was added dropwise thereto, stirred at the same temperature for 30 minutes, heated to room temperature with stirring, and further stirred for 30 minutes. The reaction solution was warmed to 50 °C, and the pressure in the system was reduced to and kept at 0.1 mmHg, and 2 hours later, the residue was dissolved in heptane, and this solution was mixed with 1 L of saturated ammonium chloride. After this mixture was stirred vigorously, the product was extracted with diethyl ether and the organic layer was washed with saturated aqueous sodium sulfate and dried over sodium sulfate. The solvent was distilled off, and the residue was distilled off to 65.9 g (332 mmol) of 2-(4-pentylcyclohexyl)-2-propene-1-ol. Its yield from 4-pentylcyclohexyl carboxylate chloride was 60.0 %.

### (Second step)

After 500 ml of 65.9 g (332 mmol) of the above 2-(4-pentylcyclohexyl)-2-propene-1-ol in dichloromethane was added to 500 ml of absolute dichloromethane and 97.0 g (450 mmol) of pyridium chlorochromate (abbreviated hereinafter to PCC) under stirring, the mixture was stirred at room temperature for 2 hours. 800 ml diethyl ether was added to the reaction solution, and the supernatant was separated by decantation, and the organic layer was distilled under reduced pressure to give 39.3 g (200 mmol) of 2-(4-pentylcyclohexyl)-2-propene-1-al. Its yield from 2-(4-pentylcyclohexyl)-2-propene-1-ol was 60.2 %.

### (Third step)

39.3 g (200 mmol) of the above 2-(4-pentylcyclohexyl)-2-propene-1-al and 30.0 g (563 mmol) of acrylonitrile were dissolved in 1 L methanol and stirred for 3 hours at room temperature and then refluxed for 2 hours. After the reaction was finished, the solvent and acrylonitrile were distilled off under reduced pressure. The residue was purified by column chromatography with a mixed solvent of heptane/ethyl acetate = 3/1, and the purified material thus obtained was recrystallized from a mixed solvent of heptane/ethyl acetate = 5/1 to give 5.2 g (19.9 mmol) of 3-(4-pentylcyclohexyl)-6-cyano-4H,5H,6H-dihydropyrane. Its yield from 2-(4-pentylcyclohexyl)-2-propene-1-al was 10.0 %.
1H-NMR data supported its structure well.
MS: 261 (M+)

### Example 4

Production of 3-(4-pentylcyclohexyl)-6-cyanotetrahydropyrane (the compound (No. 221) of formula (1) wherein R = pentyl group, m1 = m2 = m3 = 0, n1 = n2 = n3 = n4 = 0, ring A4 = 1,4-cyclohexylene, G = G1, Q₁ = Q₃ = Q₄ = -CH₂-, Q₂ = oxygen atom, Y₁ = cyano group, Y₂ = hydrogen atom).

### (First step)

0.19 g (5.0 mmol) of sodium borohydride was added little by little for 10 minutes to 0.44 g (2.5 mmol) of palladium chloride in 40 ml methanol under stirring, and the mixture was stirred for 20 minutes. 20 ml solution of 4.0 g (15.3 mmol) of the above 3-(4-pentylcyclohexyl)-6-cyano-4H,5H,6H-dihydropyrane in methanol was added to the resulting suspension, about 16 ml, followed by hydrogenation. The catalyst was removed, and after the solvent was distilled off under reduced pressure, the residue was purified by column chromatography with a mixed solvent of heptane/ethyl acetate = 3/1, and the purified material thus obtained was recrystallized from a mixed solvent of heptane/ethyl acetate = 5/1 to give 0.7 g (2.7 mmol) of 3-(4-pentylcyclohexyl)-6-cyanotetrahydropyrane. Its yield was 17.9 %.
1H-NMR data supported its structure well.
MS: 263 (M+)

### Example 5

Production of 5-(4-(4-pentylcyclohexyl)cyclohexyl)-2,2-difluoro-1,3-dithioxane (the compound (No. 111) of formula (1) wherein R = pentyl group, m1 = m2 = 0, m3 = 1, n1 = n2 = n3 = n4 = 0, ring A3 = ring A4 = 1,4-cyclohexylene group, G = G1, Q₁ = Q₄ = -CH₂-, Q₂ = Q₃ = sulfur atom, Y₁ = Y₂ = fluorine atom).

### (First step)

While 10.0 g (32.3 mmol) of 2-(4-(4-pentylcyclohexyl)cyclohexyl)-1,3-propanediol obtained in the same manner as in Example 1 (third step) was stirred in 100 ml toluene, 1.7 g (17.3 mmol) of conc. sulfuric acid and 500 ml of 48 % hydrobromic acid were added thereto, and the mixture was refluxed for 20 hours. The product was extracted with toluene, and the extract was washed with water, saturated aqueous sodium bicarbonate and water in this order and dried over magnesium sulfate. The solvent was distilled off under reduced pressure, and the residue was purified by column chromatography with a mixed solvent of heptane/ethyl acetate = 3/1 to give 9.8 g (22.5 mmol) of 2-(4-(4-pentylcyclohexyl)cyclohexyl)-1,3-dibromopropane. Its yield from 2-(4-(4-pentylcyclohexyl)cyclohexyl)-1,3-propanediol was 69.7 %.

### (Second step)

5 ml water was added to 5.3 g (69.7 mmol) of thiourea, and the solution was warmed to 50 °C and stirred for 5 minutes. Then, 9.8 g (22.5 mmol) of the above 2-(4-(4-pentylcyclohexyl)cyclohexyl)-1,3-dibromopropane was added little by little thereto, then warmed to 70 °C and stirred for 20 hours, and 15 ml of 2 N sodium hydroxide was added dropwise thereto, and the product was extracted with ethyl acetate. The solvent was distilled off, and the residue was purified by column chromatography with a mixed solvent of heptane/ethyl acetate = 1/2 whereby 4.3 g (12.5 mmol) of 2-(4-(4-pentylcyclohexyl)cyclohexyl)-1,3-propanediol was obtained. Its yield from 2-(4-(4-pentylcyclohexyl)cyclohexyl)-1,3-dibromopropane was 55.6 %.

### (Third step)

4.3 g (12.5 mmol) of the above 2-(4-(4-pentylcyclohexyl)cyclohexyl)-1,3-propanediol and 2.88 g (25.0 mmol) of thiophosgene were added to 200 ml benzene, and the mixture was stirred at 0 °C for 5 minutes, and then 4 ml pyridine was added dropwise to this suspension at the same temperature, and the mixture was stirred at room temperature for 2 hours. The reaction solution was washed with water, and the organic layer was dried over magnesium sulfate, and the solvent and thiophosgene were distilled off under reduced pressure. The residue was purified by column chromatography with heptane/ethyl acetate = 1/1 as the eluent and then recrystallized from ethyl acetate whereby 2.10 g (5.46 mmol) of 5-(4-(4-pentylcyclohexyl)cyclohexyl)-1,3-dithioxane-2-thione was obtained. Its yield was 43.7 %.

### (Fourth step)

0.4 mol/l dihydrotrifluorotetrabutyl ammonium (abbreviated hereinafter to TBAH₂F₃) in 1,2-dichloroethane was prepared, and 2.10 g (5.46 mmol) of the above 5-(4-(4-pentylcyclohexyl)cyclohexyl)-1,3-dithioxane-2-thione was added thereto at room temperature, and the mixture was stirred. 2.33 g (10.4 mmol) of N-iodinated succinic acid imide was added thereto, and the mixture was stirred at the same temperature for 45 minutes and then subjected 3 times to decantation with 100 ml of a mixed solvent of heptane/diethyl ether = 1/1. The solvent was distilled off under reduced pressure, and the residue was purified by column chromatography with heptane/ethyl acetate = 8/1 as the eluent whereby 0.10 g (0.26 mmol) of 5-(4-(4-pentylcyclohexyl)cyclohexyl)-2,2-difluoro-1,3-dithioxane was obtained. Its yield from 5-(4-(4-pentylcyclohexyl)cyclohexyl)-1,3-dithioxane-2-thione was 4.8 %.

1H-NMR data supported its structure well.

### Example 6

Production at 5-propyl-2-(2-(4-(4-cyanophenyl)cyclohexyl)ethenyl)-1,3-dioxane (the compound (No. 383) of formula (1) wherein R = cyano group, m1 = m2 = 0, m3 = 1, n1 = n2 = n3 = 0, n4 = 1, ring A3 = 1, 4-phenylene group, ring A4 = 1,4-cyclohexylene group, B₄ = -CH=CH-, G = G1, Q₁ = Q₄ = oxygen atom, Q₂ = Q₃ = -CH₂-, Y₁ = propyl group, Y₂ = hydrogen atom).

### (First step)

2 L THF was added to 357 g (781 mmol) of dry 1,3-dioxanone-2-yl ethyl triphenyl phosphonium bromide, and the mixture was cooled to 5 °C under stirring. 87.5 g (780 mmol) of potassium t-butoxide was added thereto and stirred at the same temperature for 2 hours. 700 ml solution of 129 g (647 mmol) in THF of 4-(4-cyanophenyl)cyclohexane was added dropwise to this reaction solution, heated to room temperature, and stirred for 10 hours. 1.5 L water was added to the reaction solution, and the product was extracted with ethyl acetate, and the extract was washed with a saline solution and then dried over magnesium sulfate, and the solvent was distilled off. The residue was purified by column chromatography with heptane/ethyl acetate = 4/1 as the eluent to give 60.0 (202 mmol) of 4-(4-cyanophenyl)cyclohexylidene (1,3-dioxane-2-yl) methyl. Its yield from 4-(4-cyanophenyl)cylohexanone was 31.2 %.

### (Second step)

300 ml acetone was added to 30.0 g (101 mmol) of the above 4-(4-cyanophenyl)cyclohexylidene (1,3-dioxane-2-yl) methyl, then 300 ml of 2 N hydrochloric acid was added thereto, and the mixture was refluxed for 3 hours. The product was extracted with toluene, and the extract was washed with saturated aqueous sodium bicarbonate and a saline solution, and a part of the solvent was distilled off. Since the residue was separated into 2 layers, the supernatant was recovered, and the solvent was distilled off therefrom whereby 15 g (63 mmol) of 3-(4-(4-cyanophenyl)cyclohexyl-2-propenal was obtained. Its yield from 4-(4-cyanophenyl)cyclohexylidene (1,3-dioxane-2-yl) methyl was 62 %.

### (Third step)

150 ml toluene, 10 g (84.6 mmol) of 2-propyl-1,3-propanediol, and 0.6 g of p-toluenesulfonic acid were added to 15 g (62 mmol) of the above 3-(4-(4-cyanophenyl)cyclohexyl-2-propenal and refluxed for 3 hours. The reaction solution was washed with saturated aqueous sodium bicarbonate and water and dried over magnesium sulfate, and the solvent was distilled off. The residue, about 22 g, was purified by column chromatography with heptane/ethyl acetate = 3/1 as the eluent and then recrystallized from heptane/ethanol = 1/1 to give 0.44 g (1.3 mmol) of 5-propyl-2-(2-(4-(4-cyanophenyl)cyclohexyl)ethenyl)-1,3-dioxane. Its yield from 3-(4-(4-cyanophenyl)cyclohexyl-2-propenal was 2.1 %. 1H-NMR (CDCl3) δ (ppm): 7.57 (d, 2H), 7.29 (d, 2H), 6.06-5.40 (m, 2H), 4.86 (d, 1H), 4.22-4.04 (m, 2H), 3.51-3.26 (m, 2H), 2.6-0.82 (m, 18H).
C-SB 121.8 °C
SB-N 124.8 °C
N-I 229.5 °C

### Example 7

Production of 4-(2-(4-propylcyclohexyl)ethyl)-2,2-difluoro-1,3-dioxane (the compound (No. 86) of formula (1) wherein R = propyl group, m1 = m2 = m3 = 0, n1 = n2 = n3 = 0, n4 = 1, ring A4 = 1,4-cyclohexylene group, B₄ = -CH₂CH₂-, G = G1, Q₁ = -CH₂-, Q₂ = Q₃ = oxygen atom, Q₄ = covalent bond, Y₁ = Y₂ = fluorine atom).

### (First step)

30 ml of 30 % aqueous hydrogen peroxide (280 mmol) was added to 120 ml of 88 % formic acid, and while the reaction temperature was kept at 40 °C, 36.1 g (200 mmol) of 4-(4-propylcyclohexyl)butene was added dropwise thereto for 30 minutes, and the mixture was stirred at 40 °C for 1 hour and left at room temperature for 12 hours. The formic acid and water were distilled off under reduced pressure, and 30 ml of 35 % aqueous sodium hydroxide cooled on ice was added dropwise to the residue at 45 °C or less. The product was extracted with diethyl ether, and the extract was distilled off under reduced pressure. The residue was recrystallized from ethyl acetate to give 18.7 g (87.2 mmol) of 4-(4-propylcyclohexyl)-1,2-butanediol. Its yield from 4-(4-propylcyclohexyl)butene was 43.6 %.

### (Second step)

18.7 g (87.2 mmol) of the above 4-(4-propylcyclohexyl)-1,2-butanediol and 14.4 g (125.0 mmol) of thiophosgene were added to 500 ml benzene and stirred at 0 °C for 5 minutes, then 20 ml pyridine was added dropwise to this solution at the same temperature and stirred at room temperature for 2 hours. The reaction solution was washed with water, and the organic layer was dried over magnesium sulfate, and the solvent and thiophosgene were distilled off under reduced pressure. The residue was purified by column chromatography with heptane/ethyl acetate = 1/1 as the eluent and then recrystallized from ethyl acetate to give 8.2 g (32.0 mmol) of 4-(2-(4-propylcyclohexyl)ethyl)-1,3-dioxolane-2-thione. Its yield from 4-(4-propylcyclohexyl)-1,2-butanediol was 36.7 %.

### (Third step)

0.4 mol/l dihydrotrifluorotetrabutyl ammonium (abbreviated hereinafter to TBAH₂F₃) in 1,2-dichloroethane was prepared, and to 40.0 ml (16.0 mmol) of this solution was added 1.44 g (5.49 mmol) of the above 4-(2-(4-propylcyclohexyl)ethyl)-1,3-dioxolane-2-thione at room temperature, and the mixture was stirred. 2.33 g (10.4 mmol) of N-iodinated succinic acid imide was added thereto, and the mixture was stirred at the same temperature for 45 minutes and then subjected 3 times to decantation with 100 ml of a mixed solvent of heptane/diethyl ether = 1/1. The solvent was distilled off under reduced pressure, and the residue was purified by column chromatography with heptane/ethyl acetate = 8/1 as the eluent whereby 0.05 g (0.19 mmol) of 4-(2-(4-propylcyclohexyl)ethyl)-2,2-difluoro-1,3-dioxolane was obtained. Its yield from 4-(2-(4-propylcyclohexyl)ethyl)-1,3-dioxolane-2-thione was 3.5 %.

1H-NMR data supported its structure well.

### Example 8

Production of 5-(4-pentylcyclohexyl)-2-ethenyl-1,3-dioxane (the compound (No. 371) of formula (1) wherein R = pentyl group, m1 = m2 = m3 = 0, n1 = n2 = n3 = n4 = 0, ring A4 = 1,4-cyclohexylene group, G = G1, Q₁ = Q₄ = -CH₂-, Q₂ = Q₃ = oxygen atom, Y₁ = ethenyl group, Y₂ = hydrogen atom).

### (First step)

10.0 g (178 mmol) of acrolein and 1 g of p-toluenesulfonic acid were added to 20.0 g (87.6 mol) of 2-(4-pentylcyclohexyl)-1,3-propanediol, and the mixture was refluxed for 2 hours. The reaction solution was washed with saturated aqueous sodium bicarbonate and then with water and dried over magnesium sulfate, and the solvent was distilled off. The residue was purified by column chromatography with toluene as the eluent and recrystallized from ethyl acetate whereby 1.0 g (3.8 mmol) of 5-(4-pentylcyclohexyl)-2-ethenyl-1,3-dioxane was obtained. Its yield from 2-(4-pentylcyclohexyl)-1,3-propanediol was 4.3 %. 1H-NMR (CDCl3) δ (ppm): 6.08-5.21 (m, 3H), 4.84 (d, 1H), 4.30-4.12 (m, 2H), 3.62-3.36 (m, 2H), 1.80-0.81 (m, 22H).
C-N 39.5 °C
N-I 52.0 °C

On the basis of Examples 1 to 8 and the detailed description of the invention, the following Compound Nos. 1 to 390 can be produced.

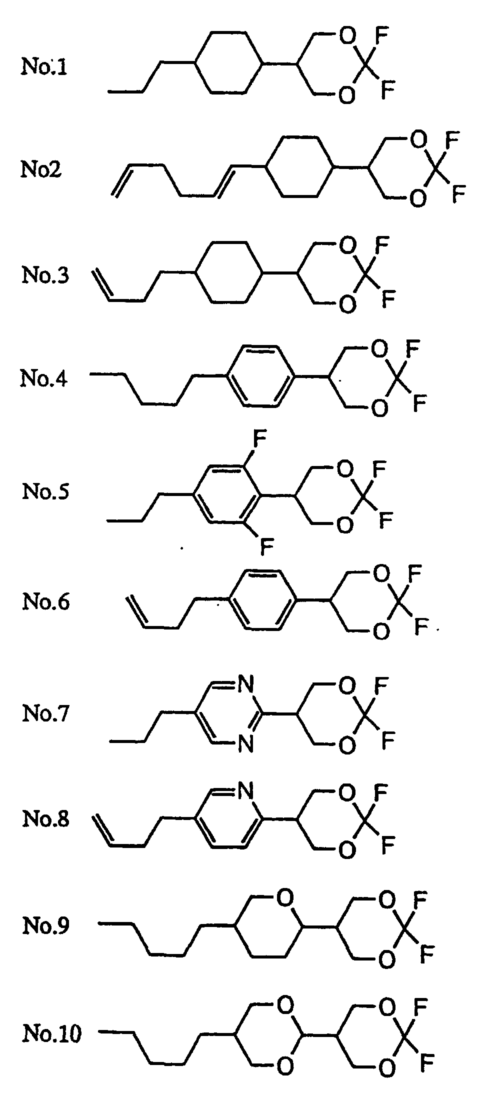

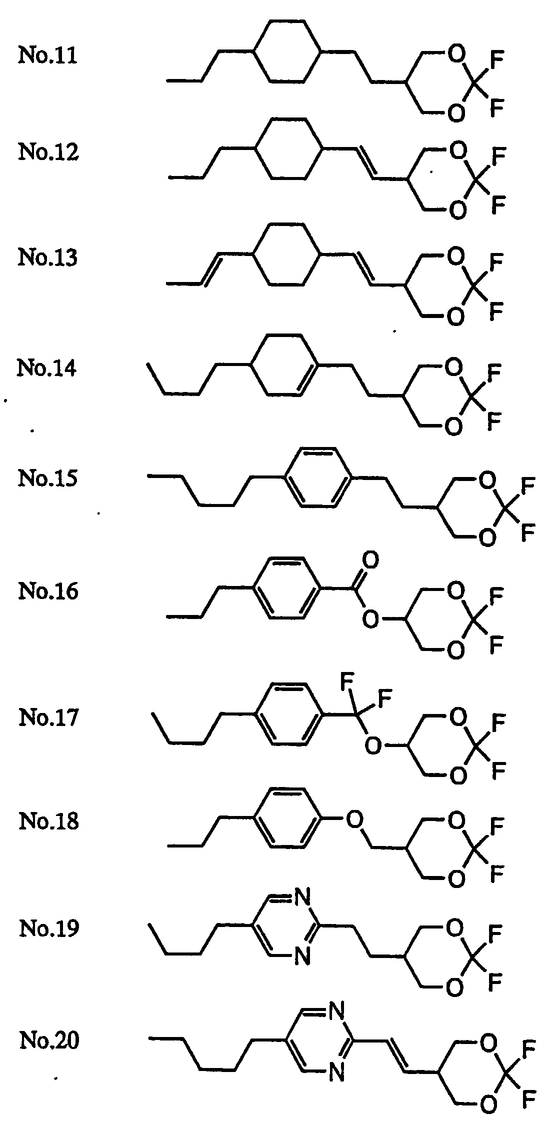

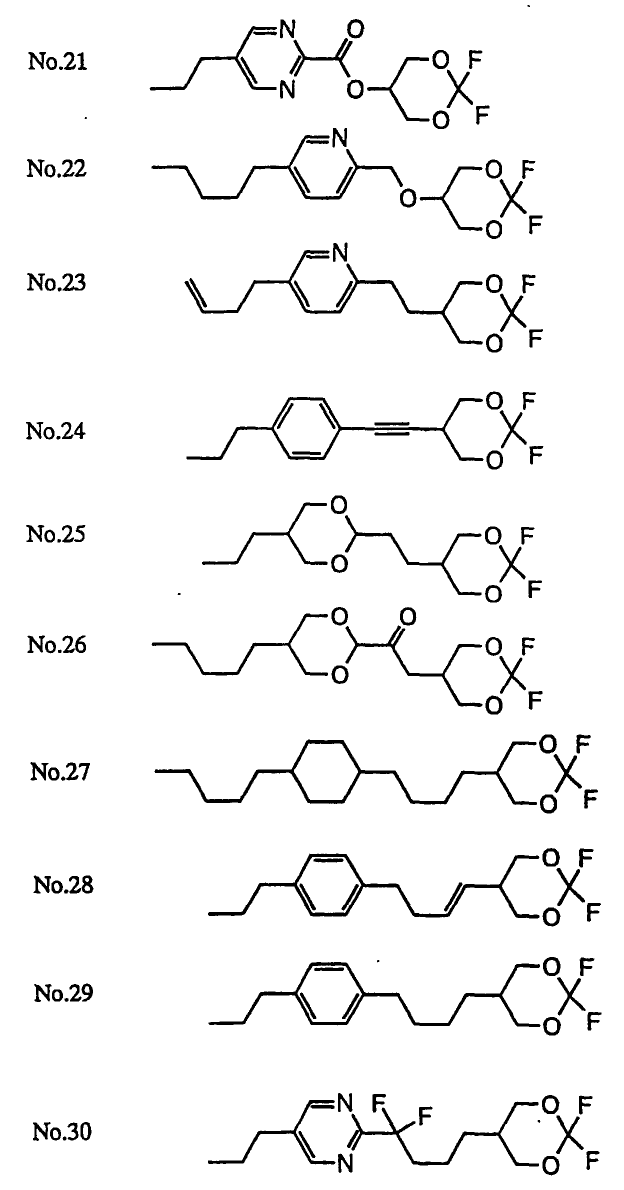

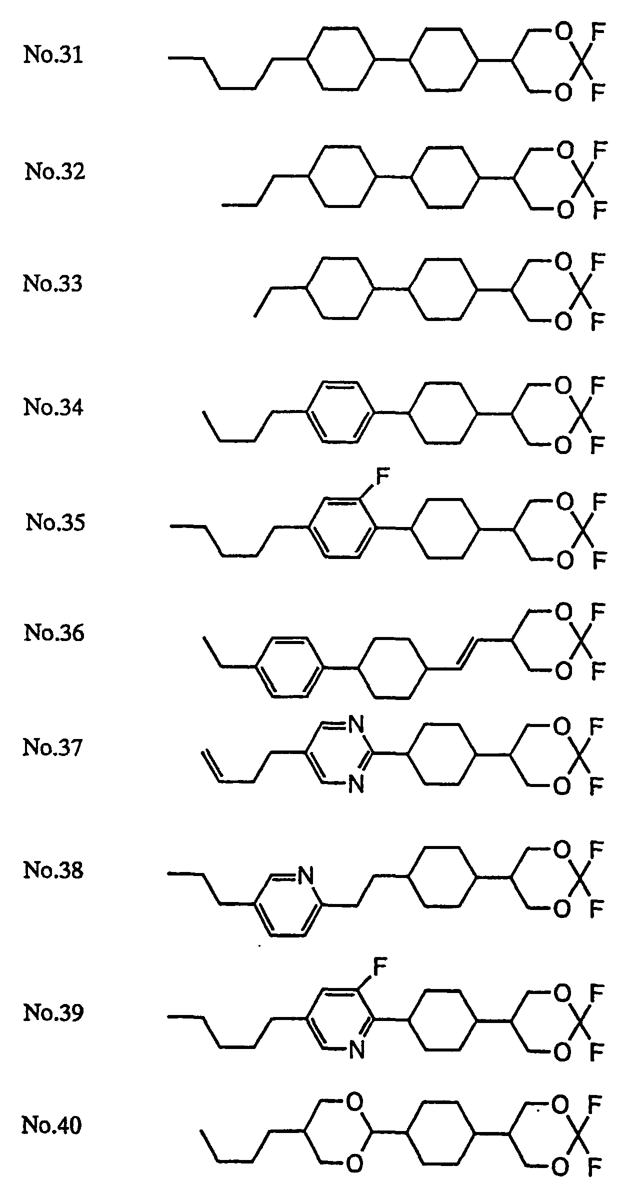

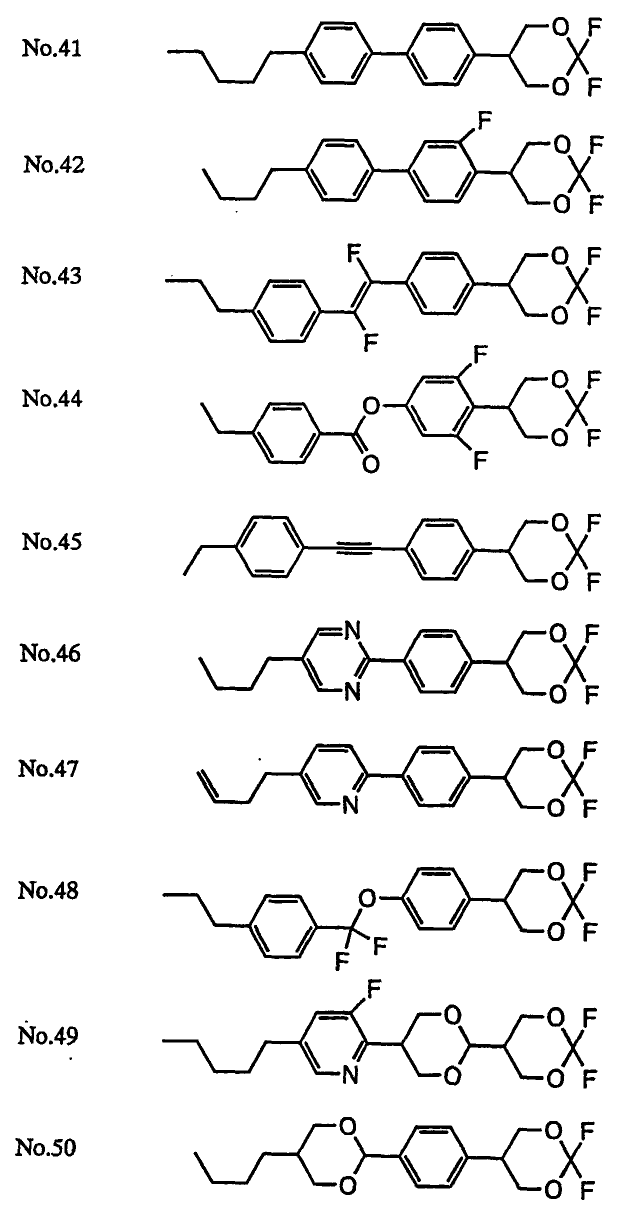

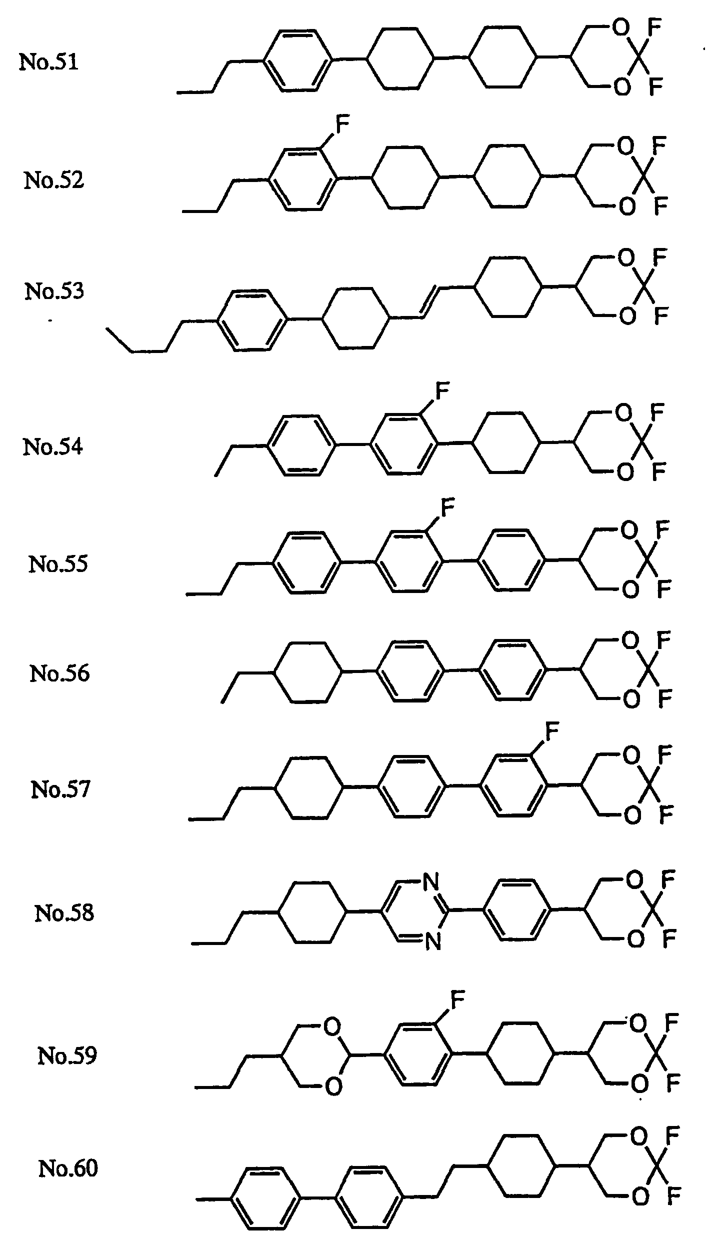

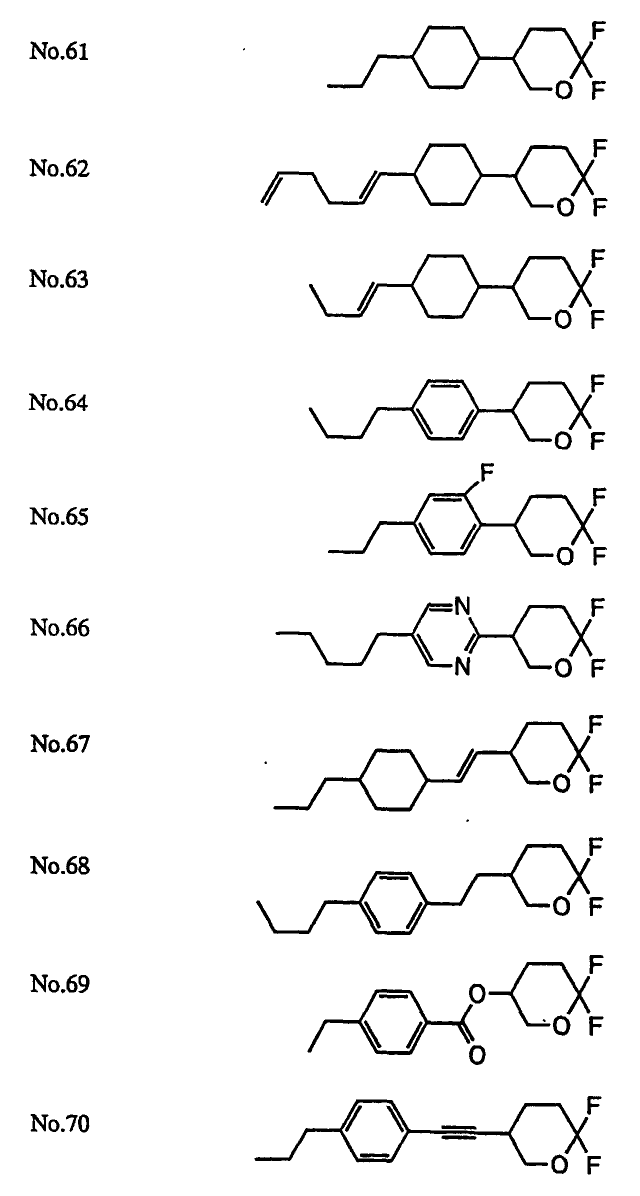

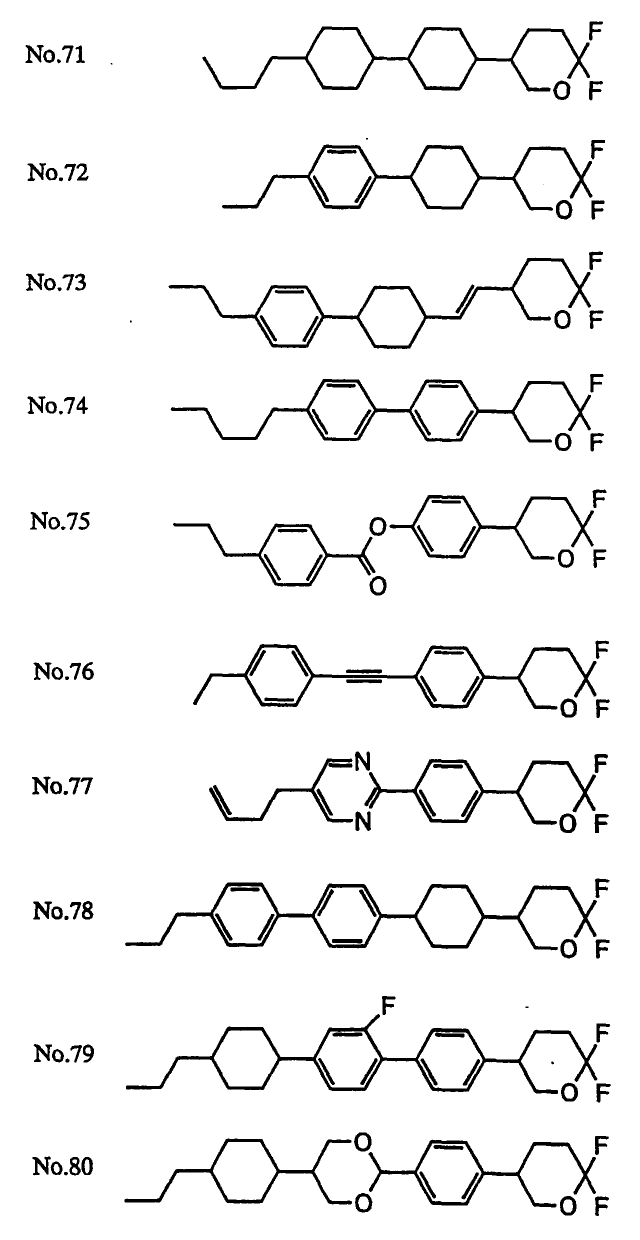

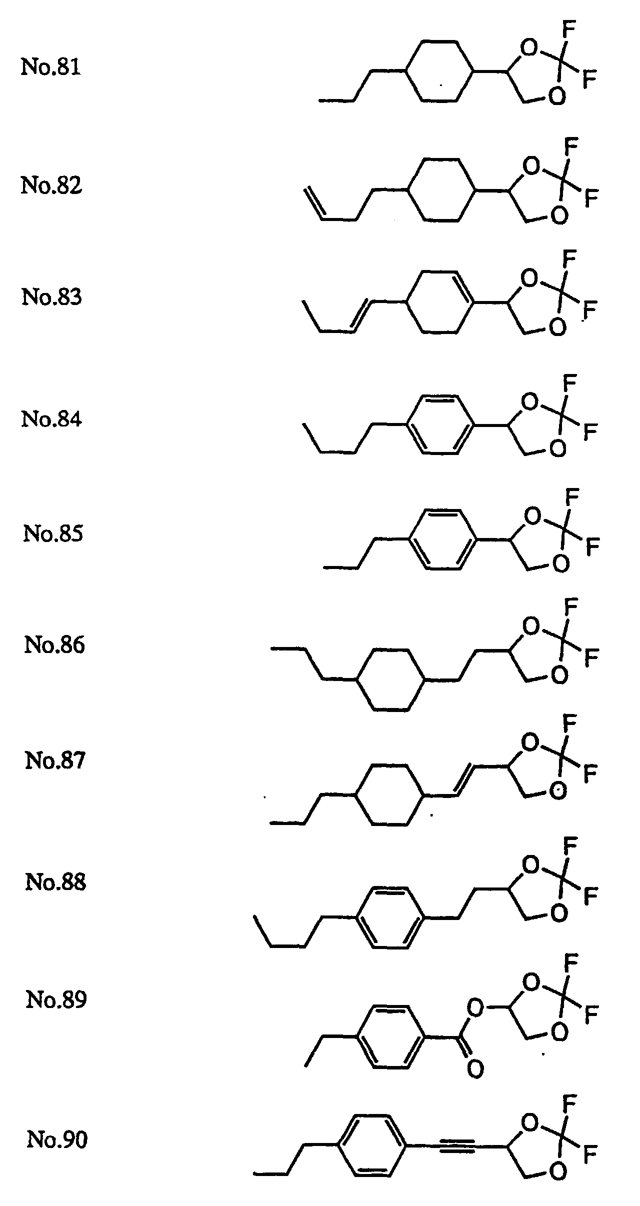

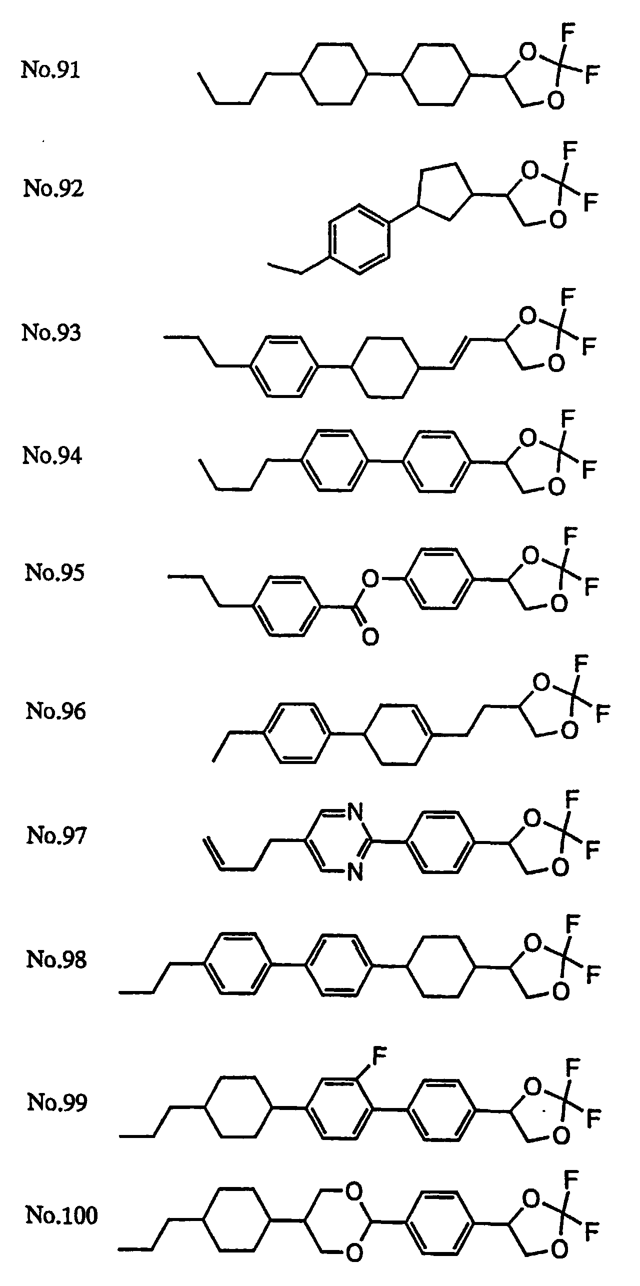

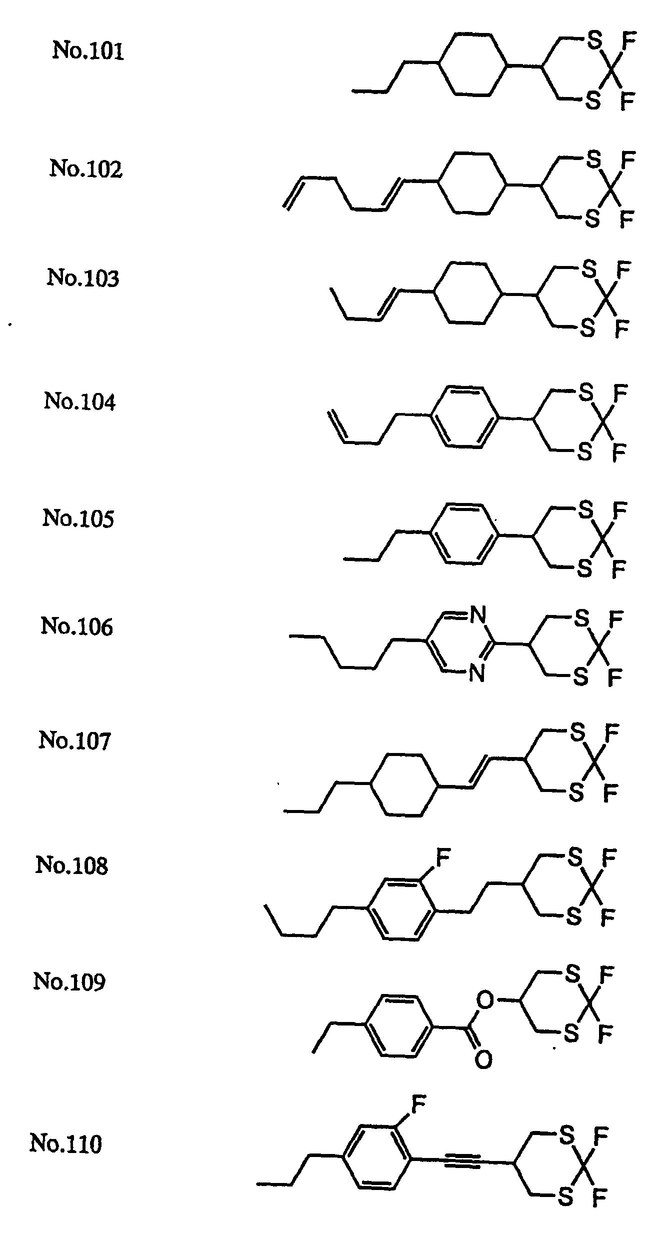

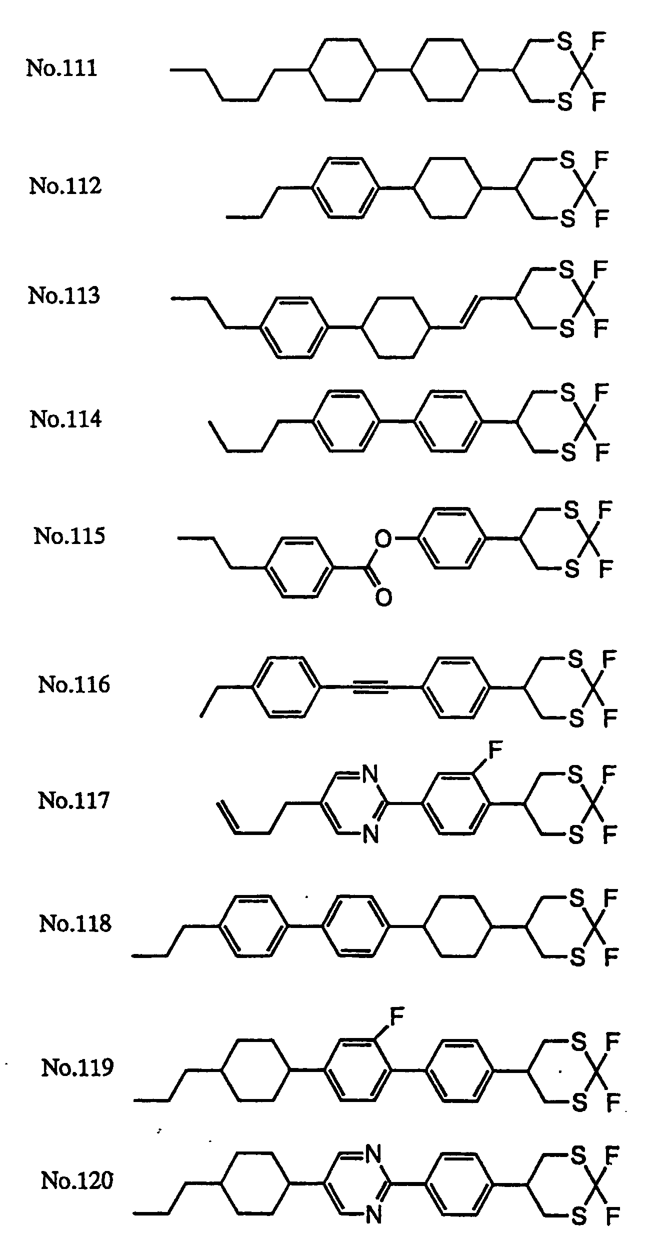

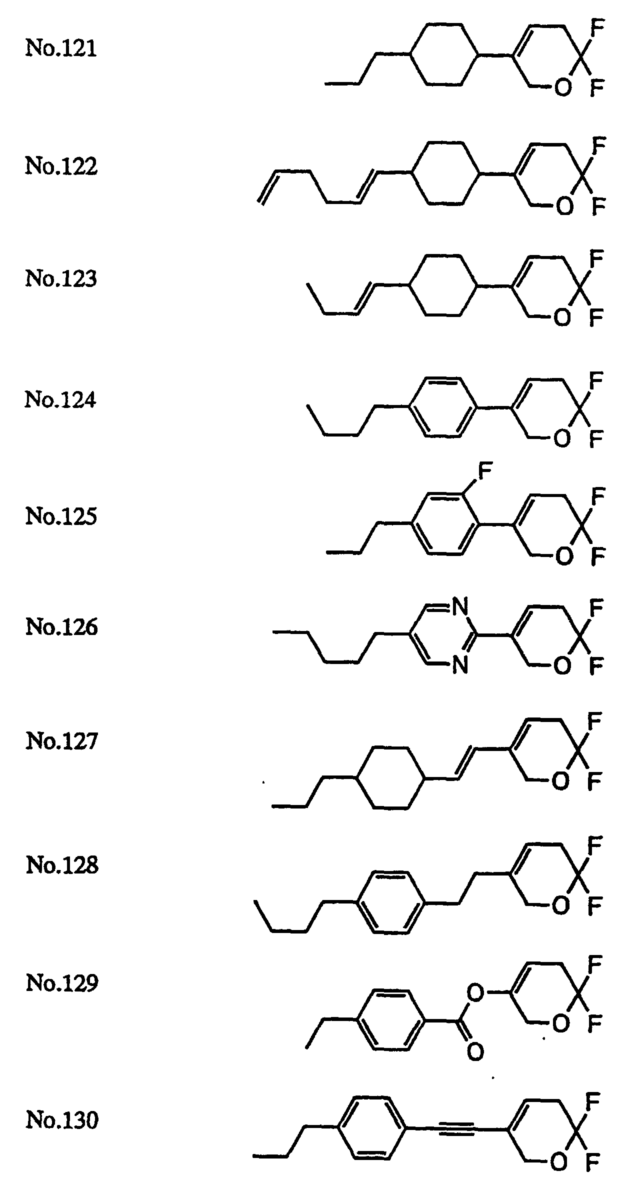

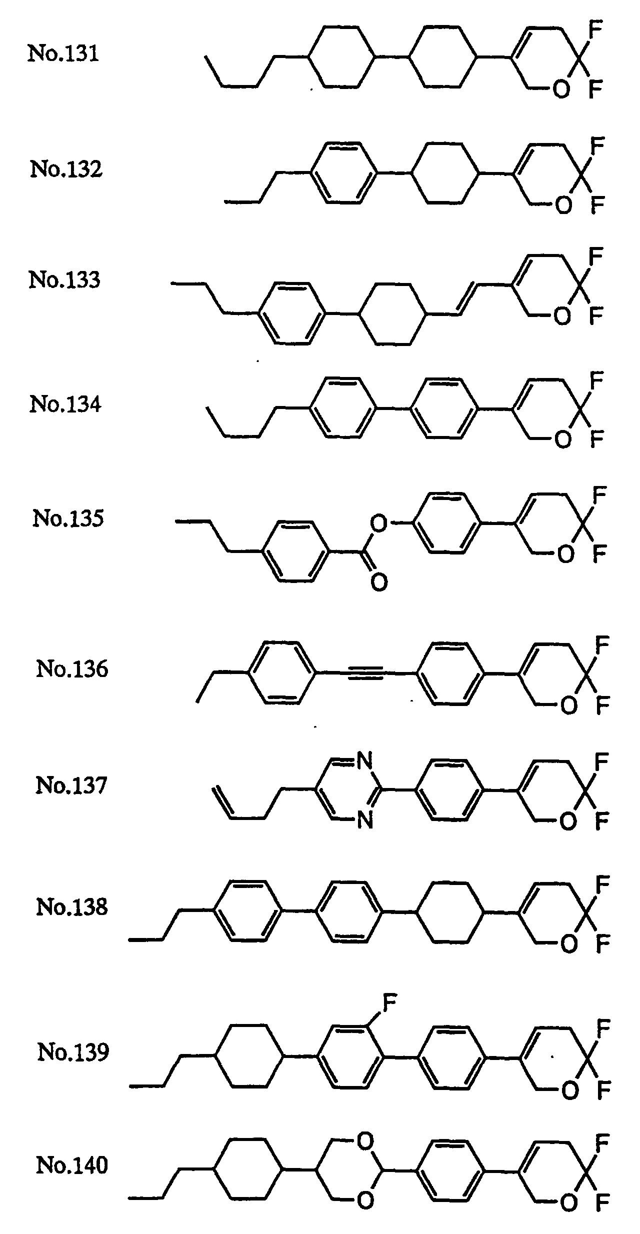

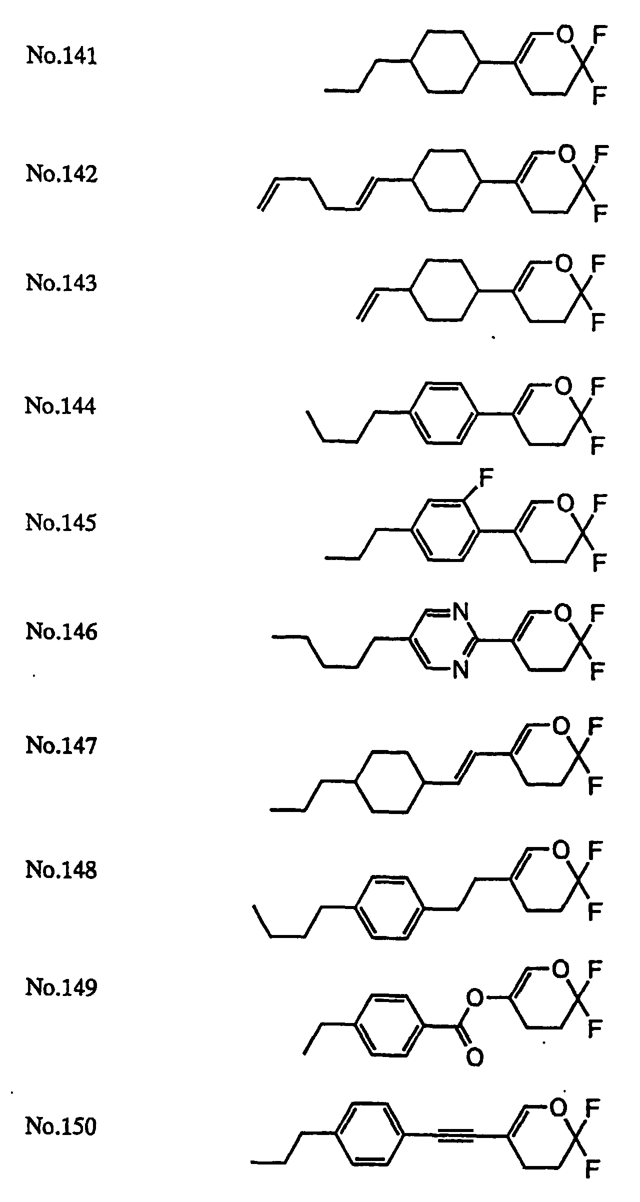

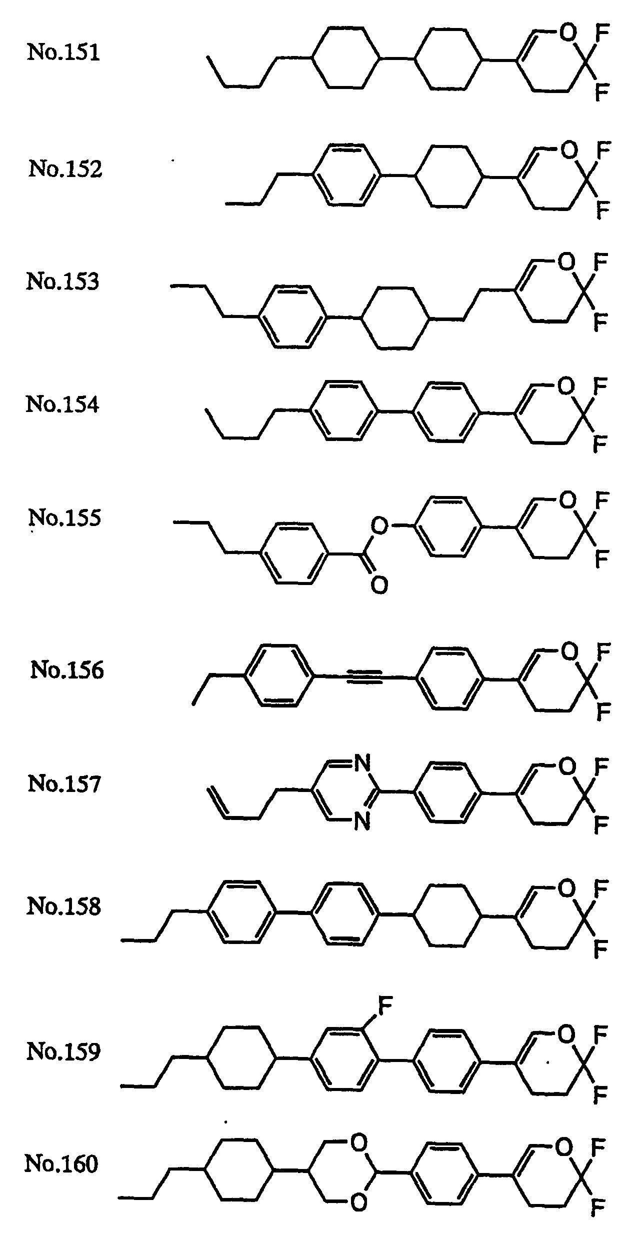

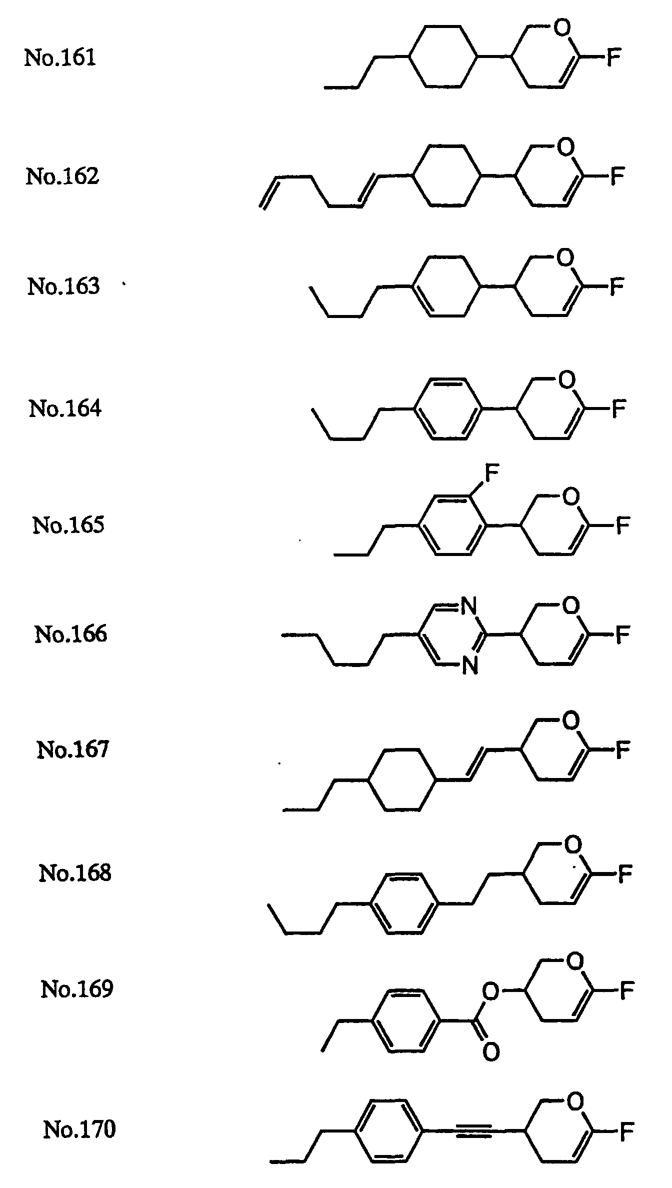

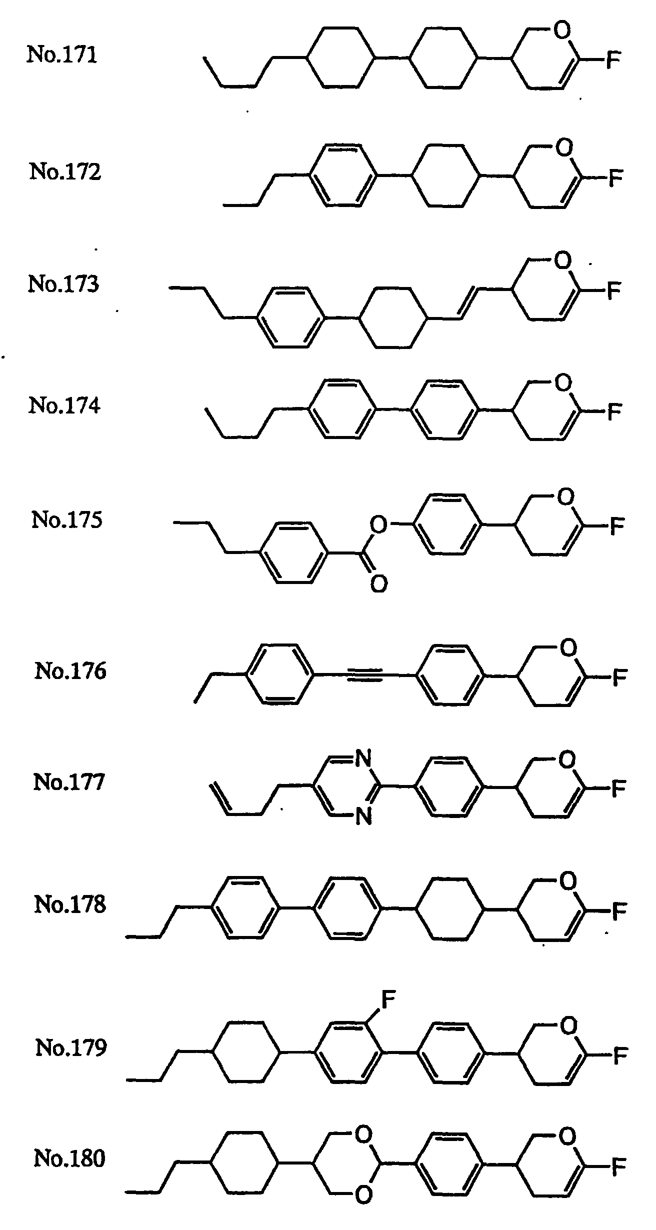

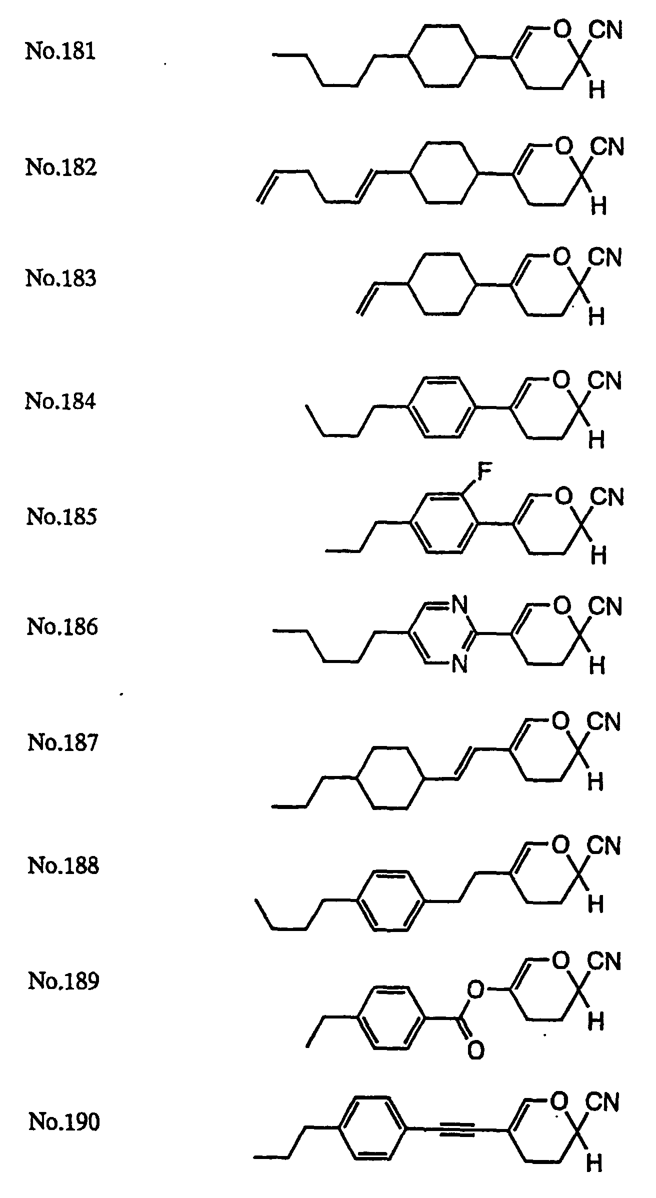

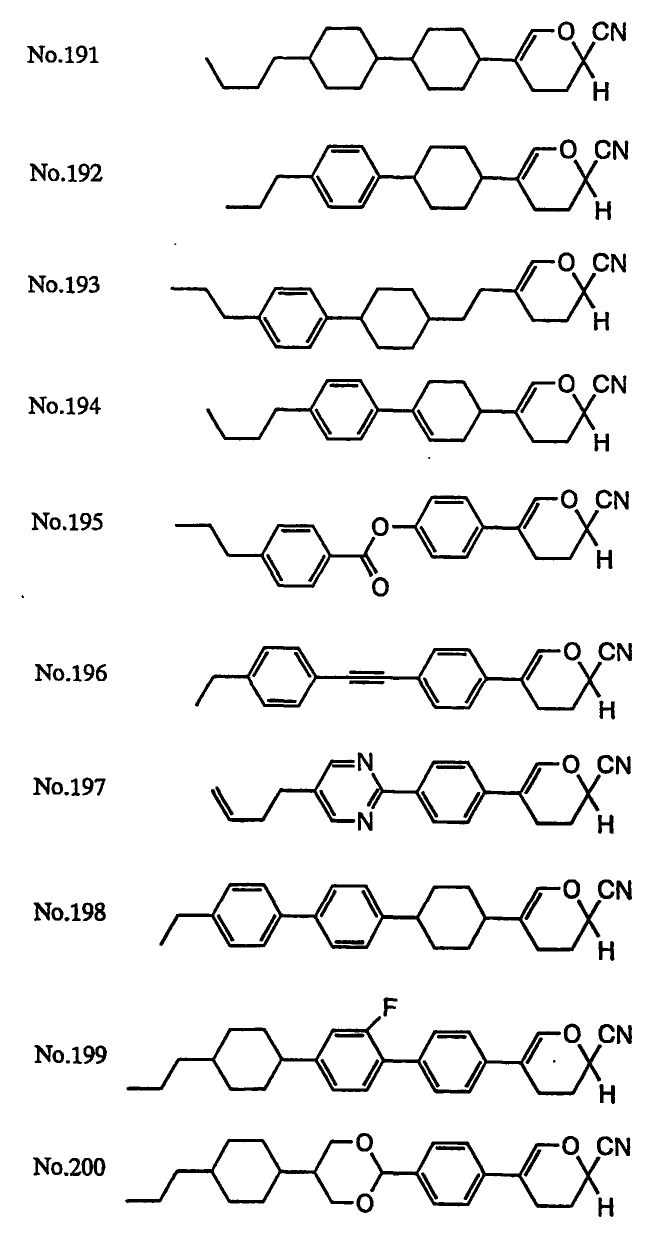

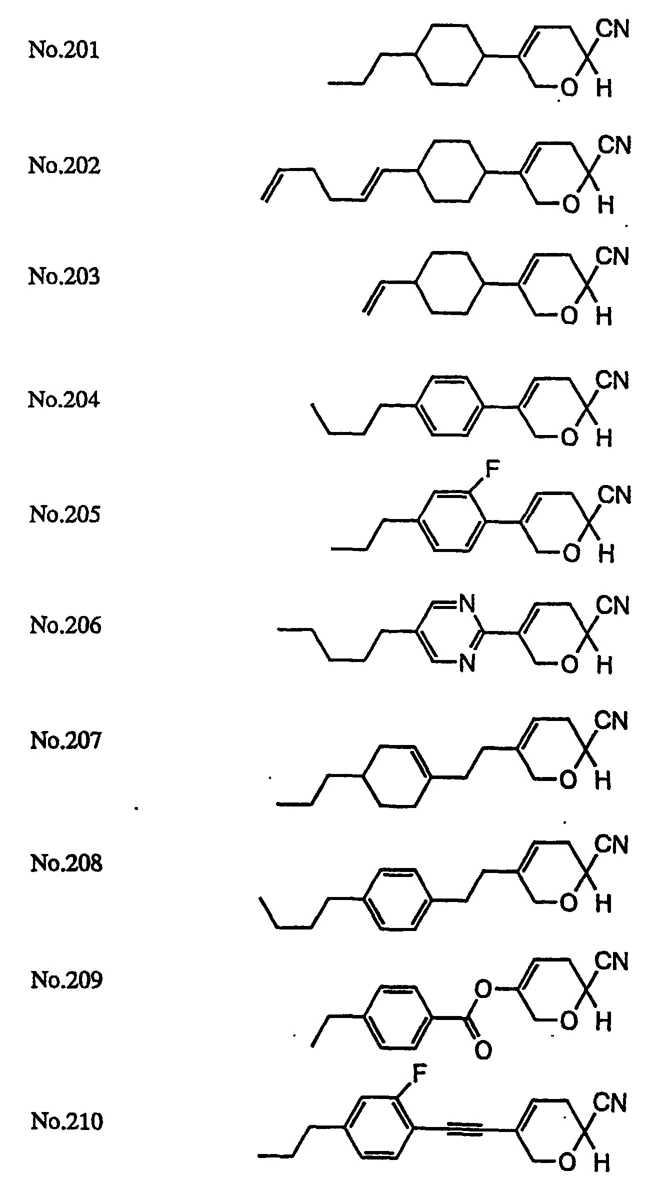

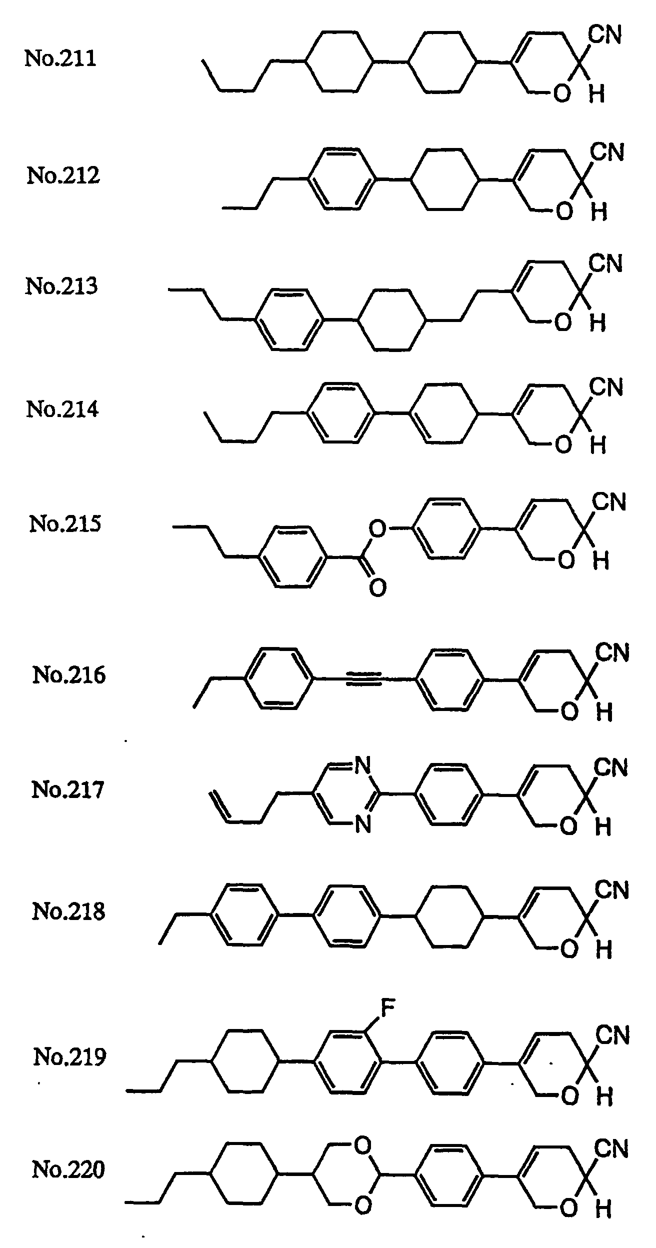

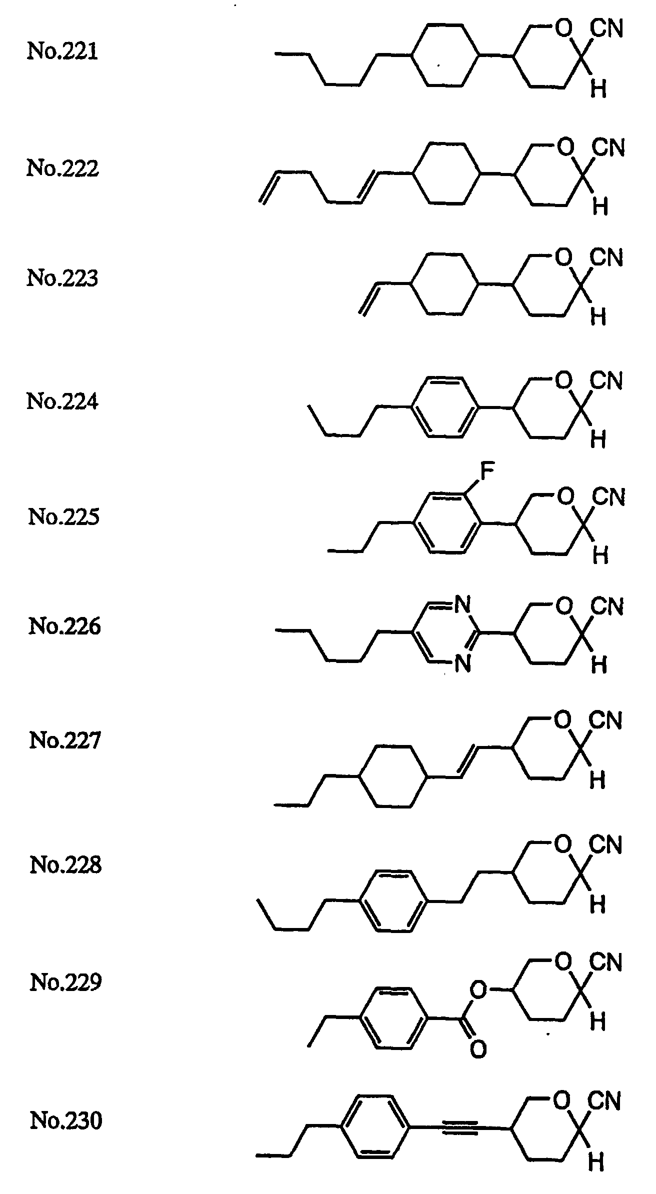

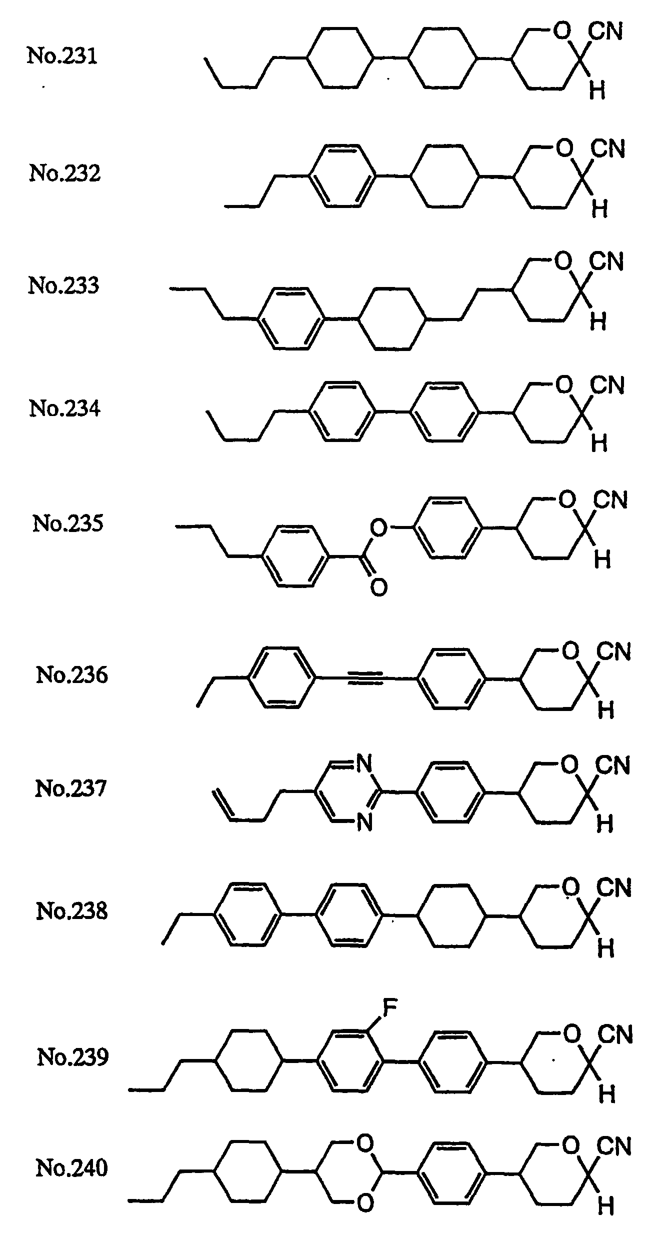

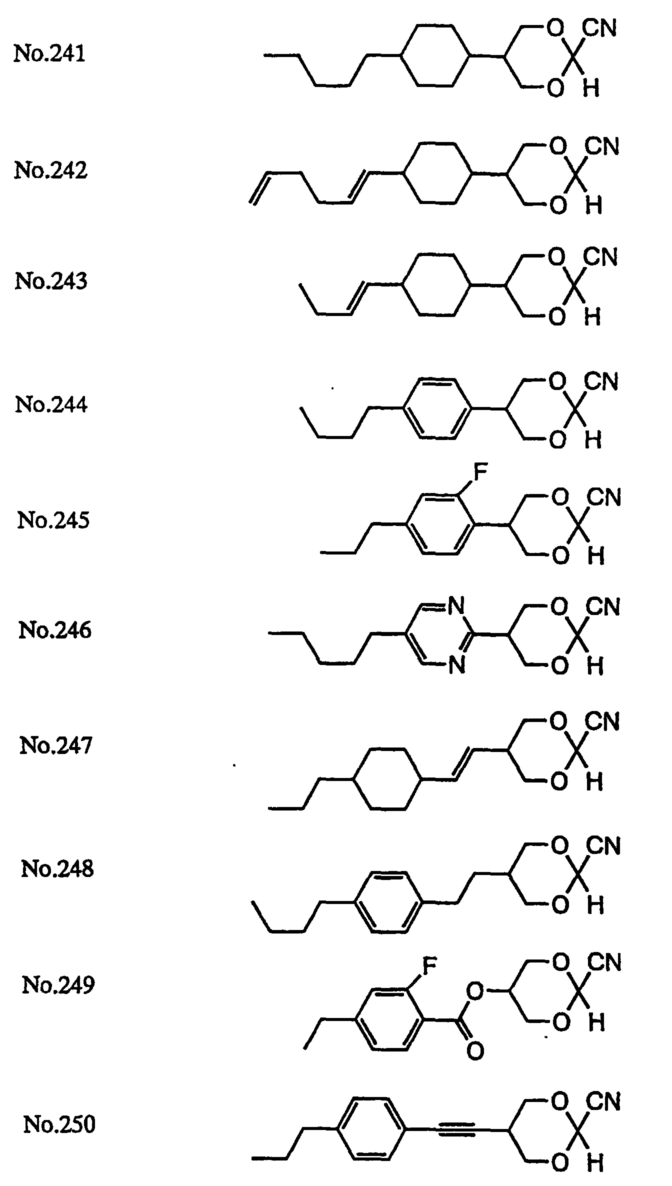

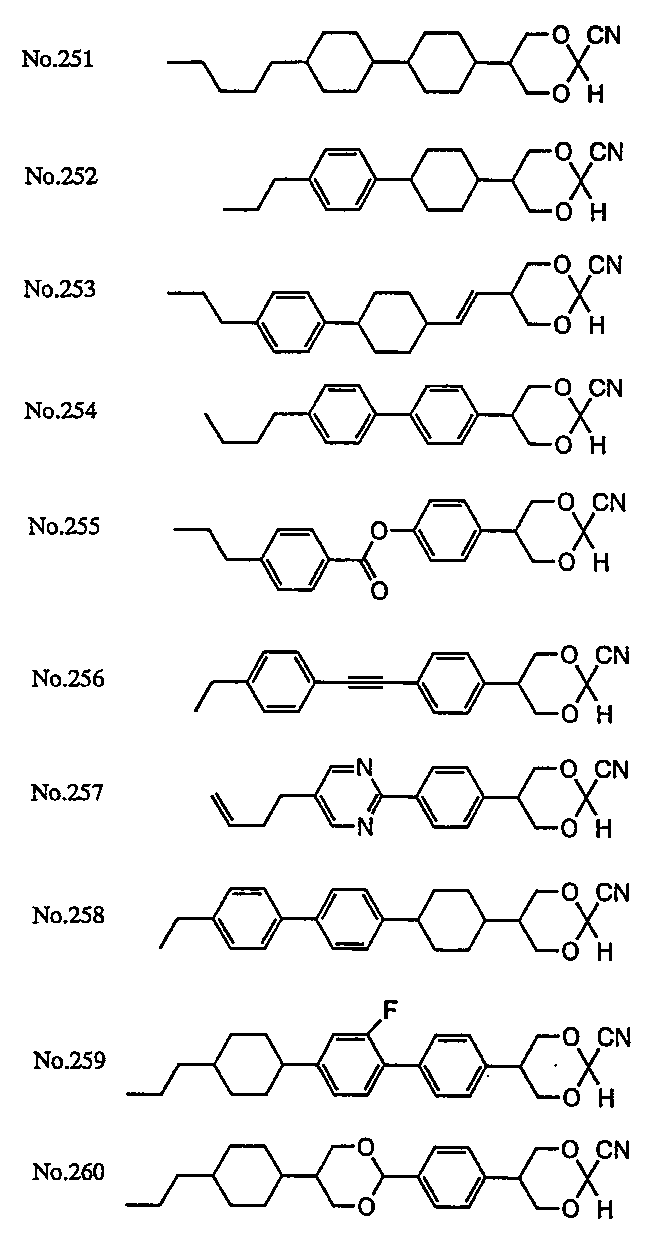

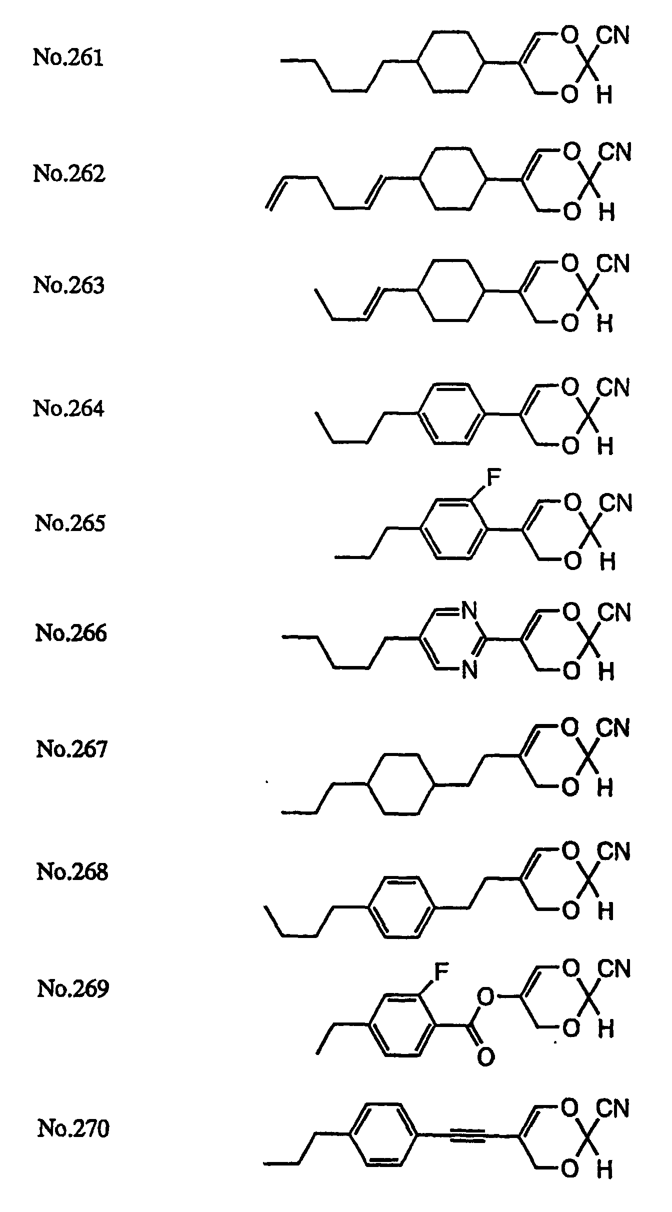

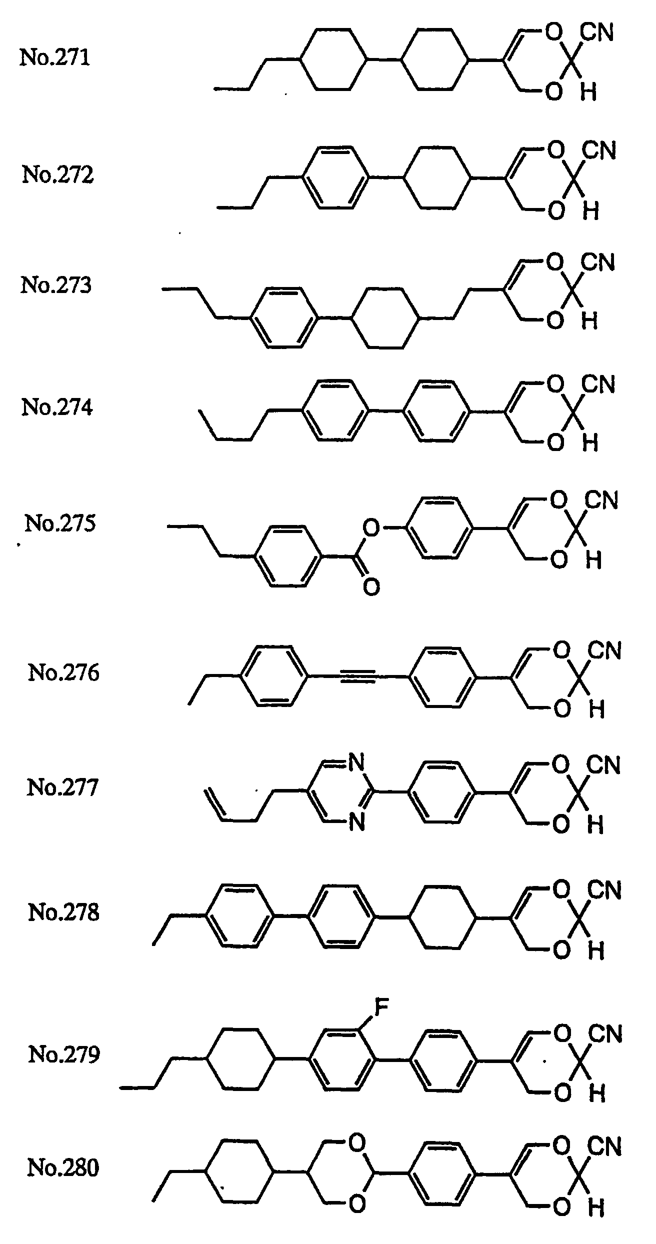

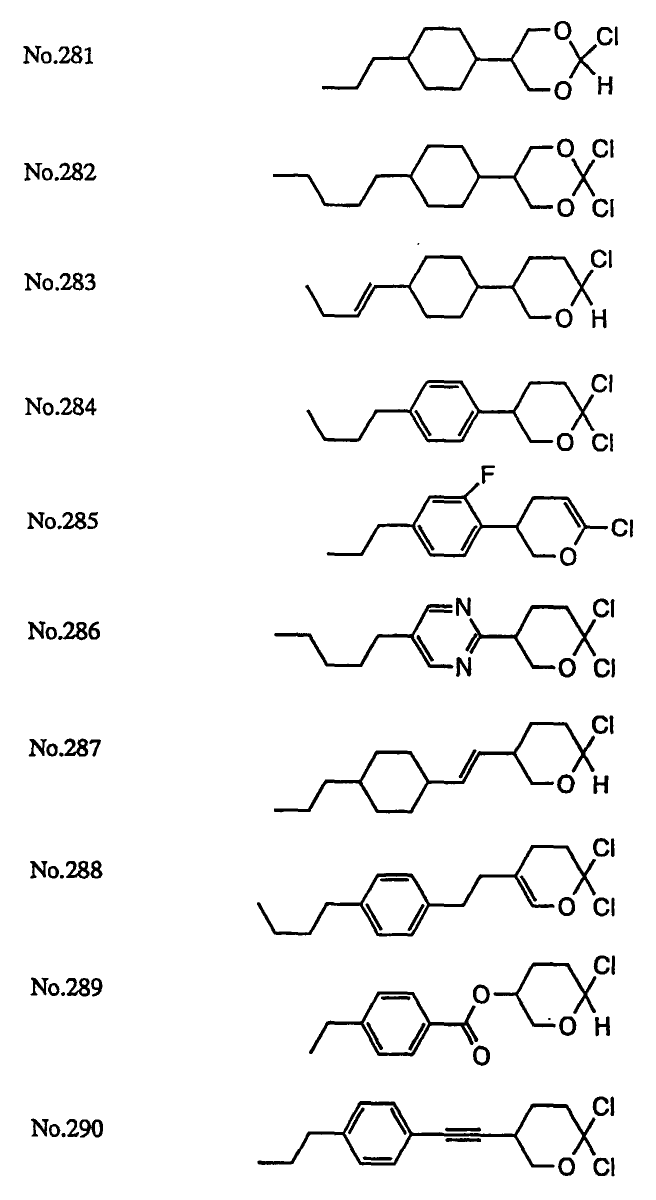

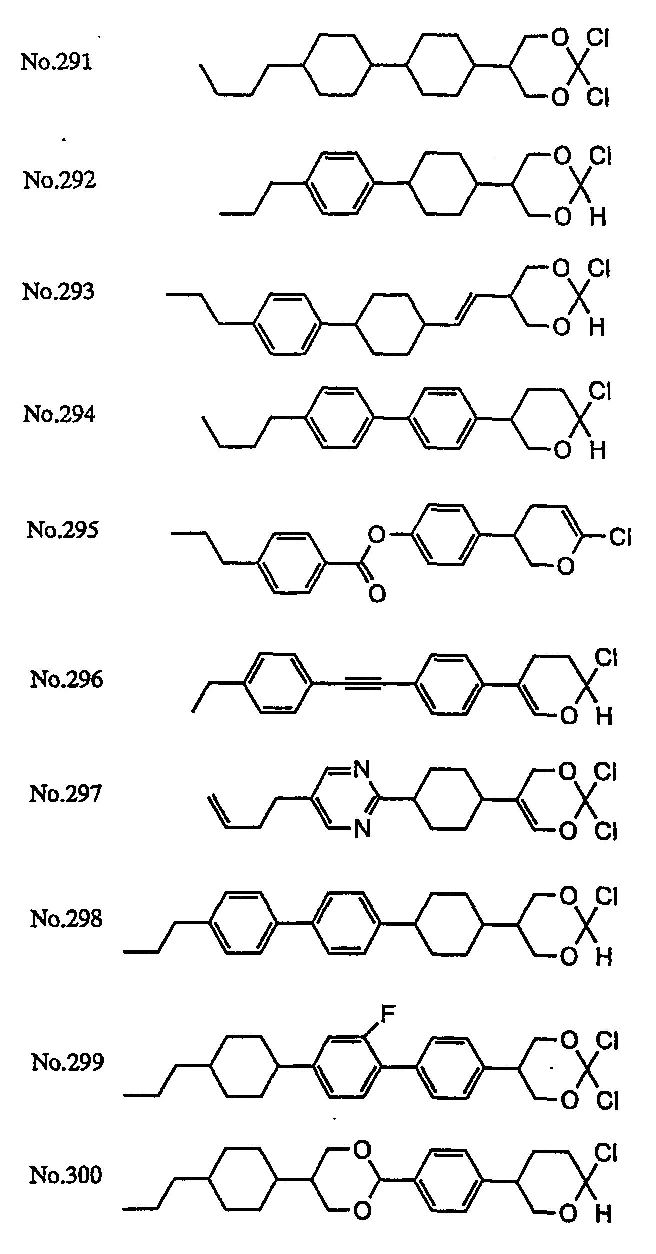

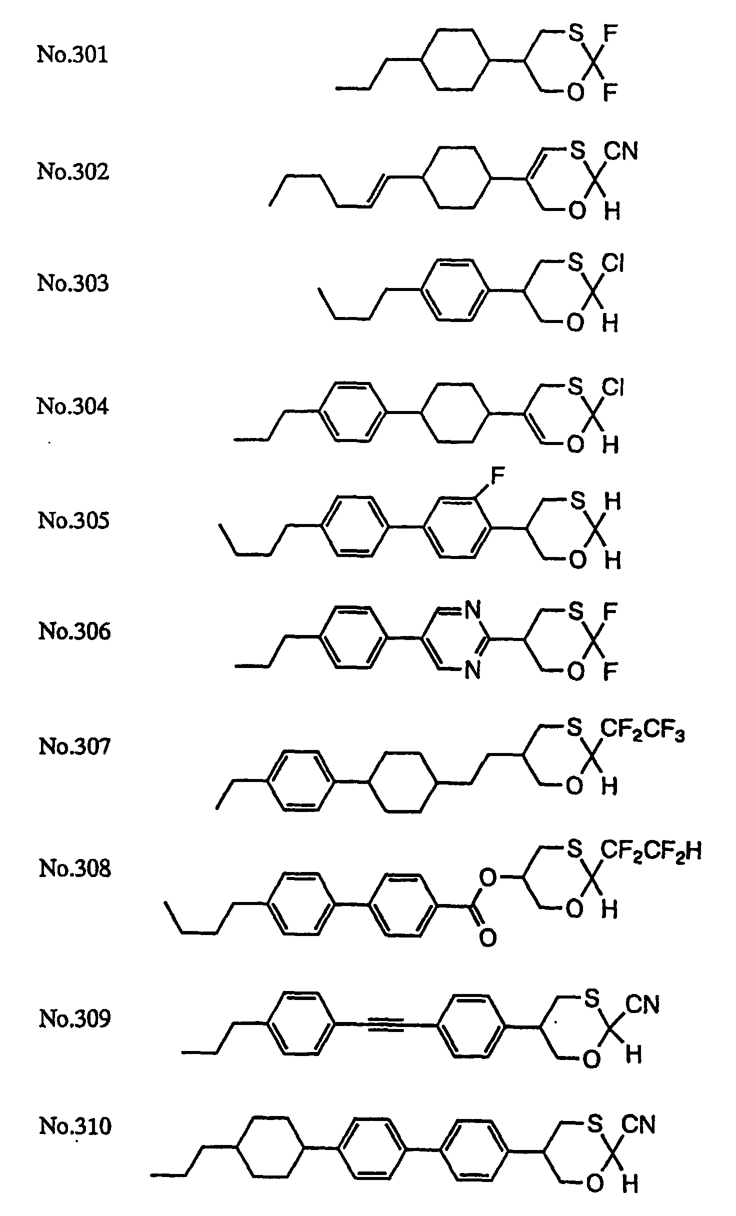

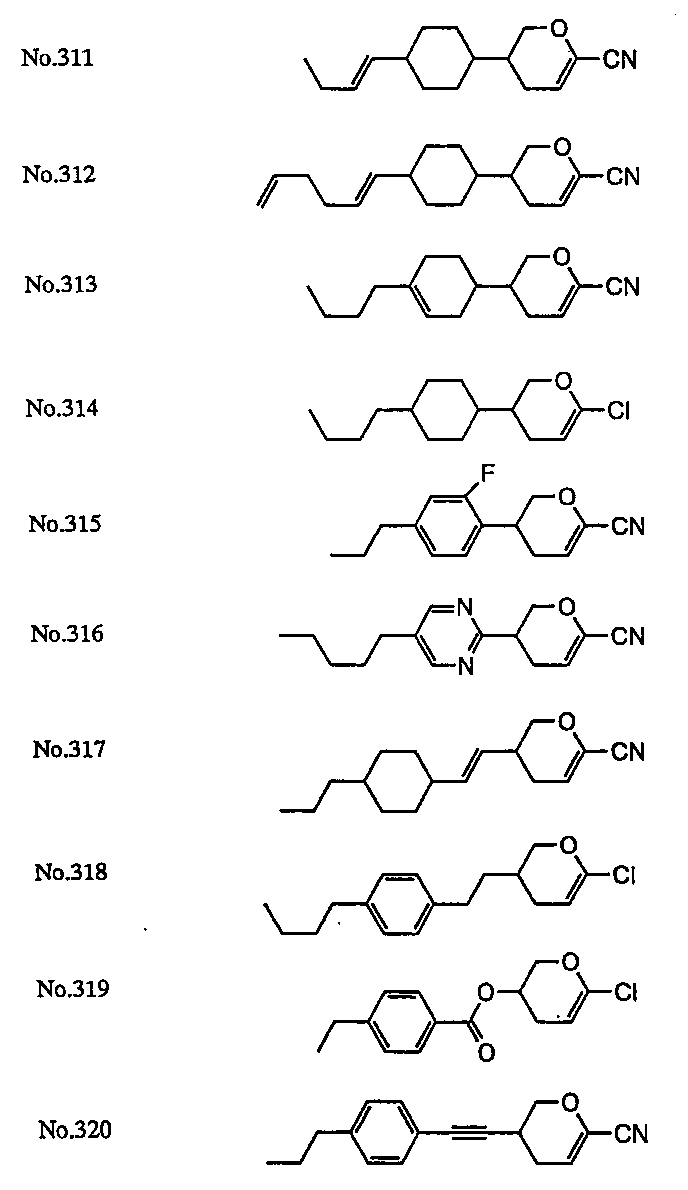

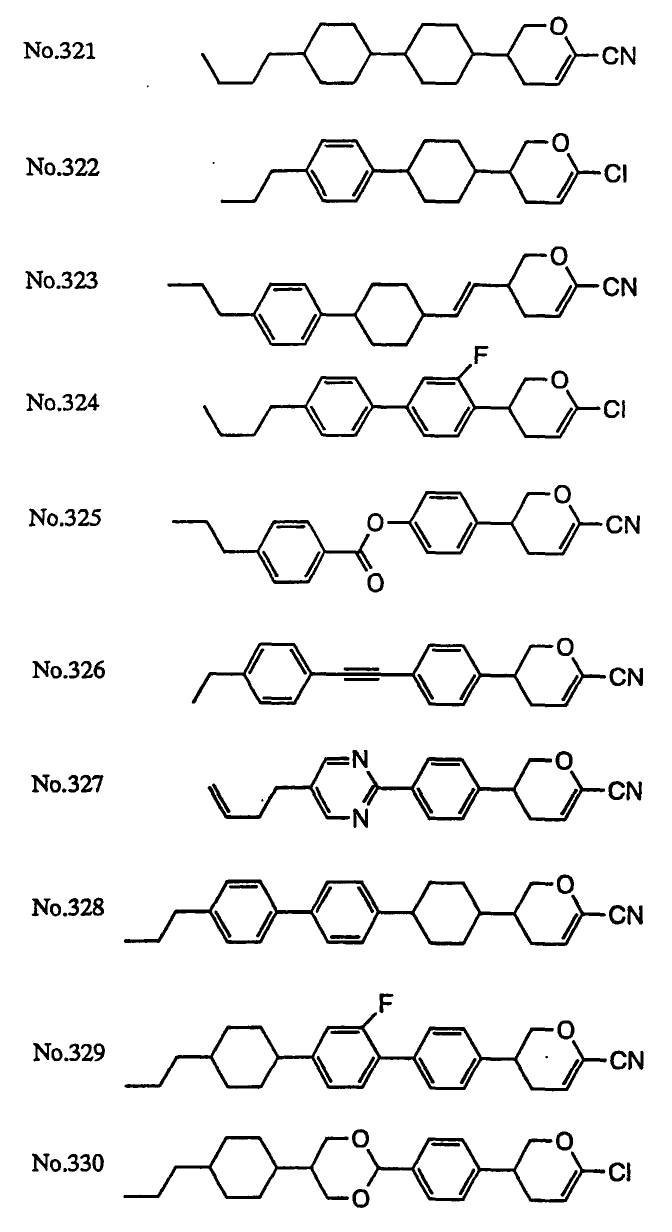

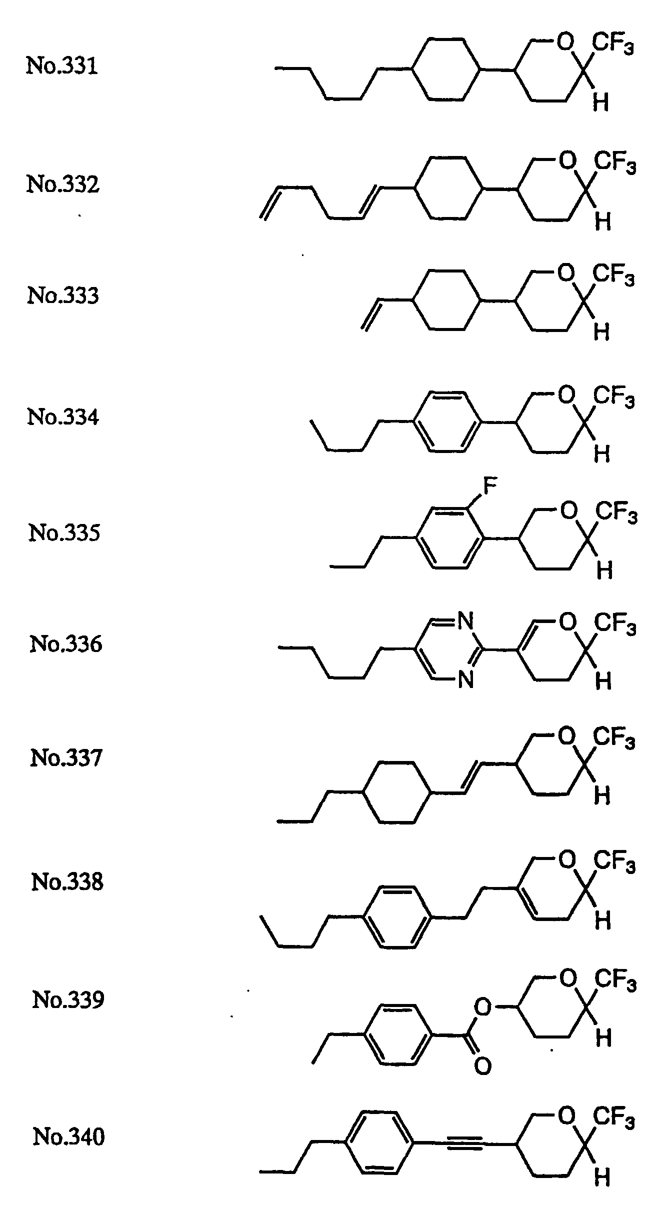

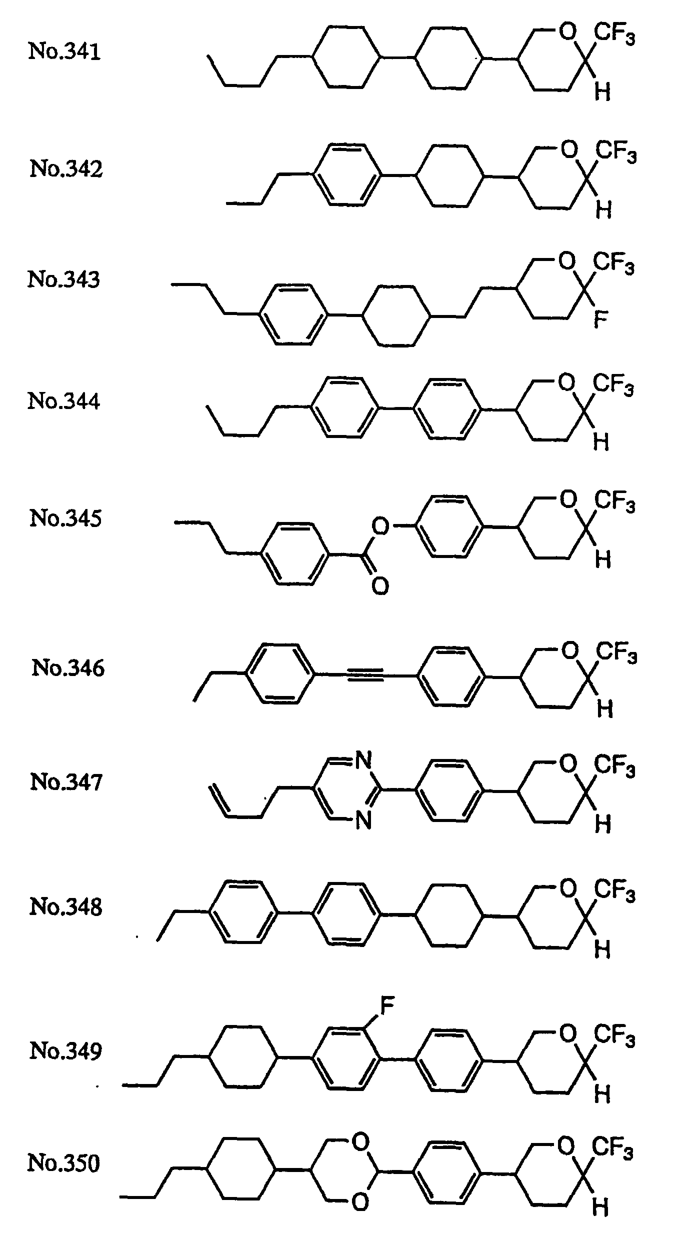

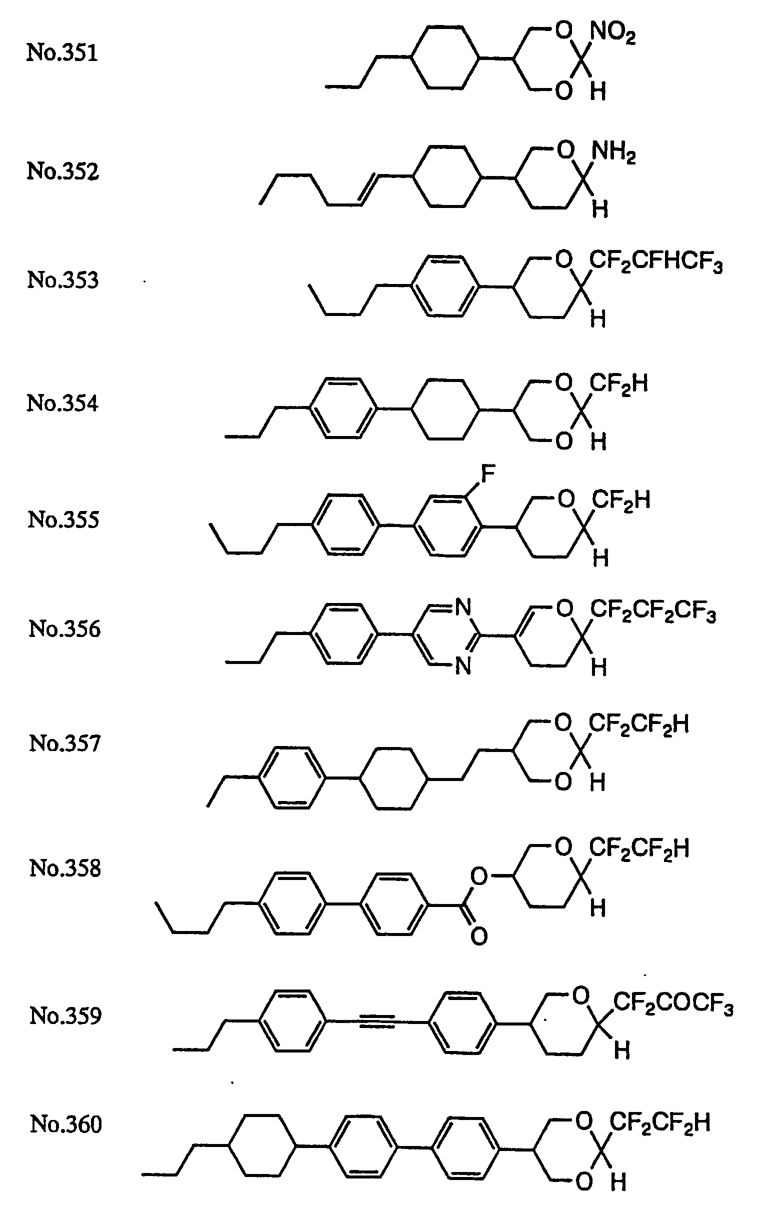

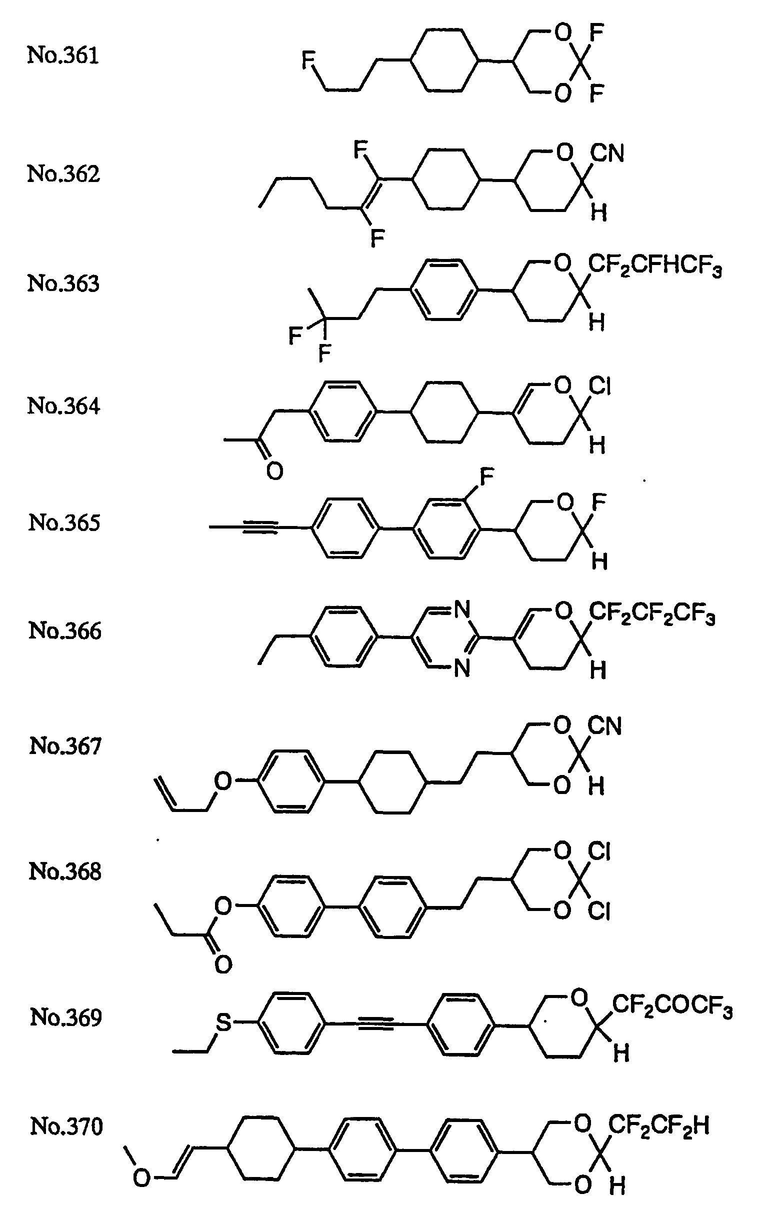

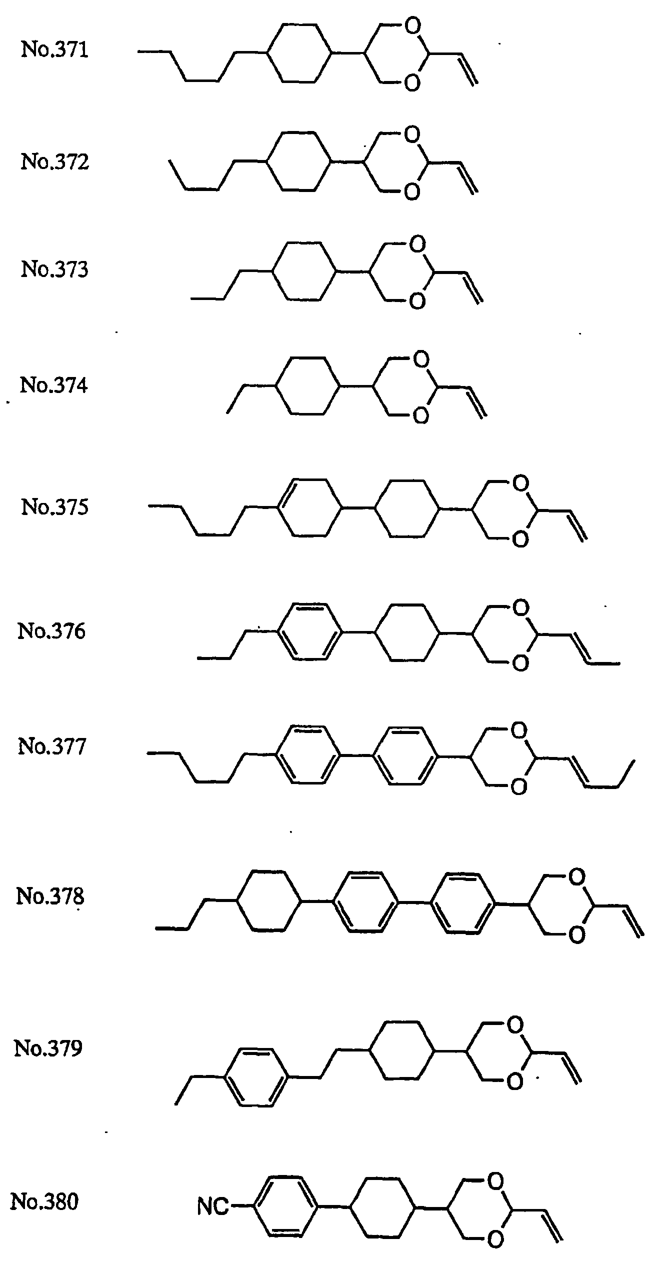

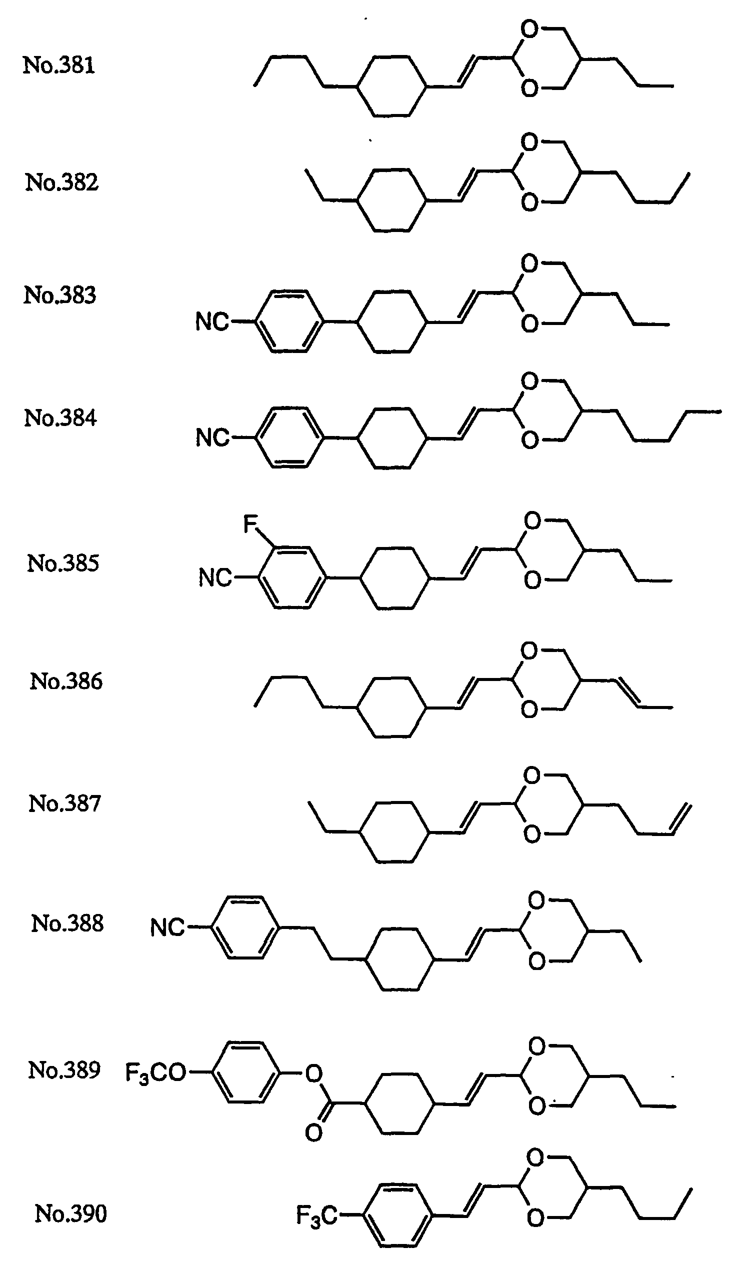

### Example 9 (Application Example 1)

A liquid crystal composition (A1) with the following composition was prepared:

| | |
|---|---|
| 4-(4-Propylcyclohexyl)benzonitrile | 24 % |
| 4-(4-Pentylcyclohexyl)benzonitrile | 36 % |
| 4-(4-Heptylcyclohexyl)benzonitrile | 25 % |
| 4-(4-(4-Pentylcyclohexyl)phenylbenzonitrile | 15 % |

The clearing point of this nematic liquid crystal composition was 71.7 °C, the threshold voltage at a cell thickness of 9 µm was 1.78 V, the Δε was 11.0, and the Δn was 0.137. A liquid crystal composition (B1) comprising 85 % of this liquid crystal composition and 15 % of the compound of the present invention in Example 1 i.e. 5-(4-(4-pentylcyclohexyl)cyclohexyl)-2,2-difluoro-1,3-dioxane (compound No. 31). The clearing point of this liquid crystal composition was 74.2 °C, the threshold voltage at a cell thickness of 8.8 µm was 1.61 V, the Δε was 8.3, and the Δn was 0.125. The physical values of the compound in Example 1, calculated by extrapolation from the mixing ratio of the above liquid crystal composition, indicated that the clearing point was 88.4 °C, the Δε was 8.3, and the Δn was 0.057.

### Example 10 (Application Example 2)

A liquid crystal composition (A1) with the following composition was prepared:

| | |
|---|---|
| 4-(4-Propylcyclohexyl)benzonitrile | 24 % |
| 4-(4-Pentylcyclohexyl)benzonitrile | 36 % |
| 4-(4-Heptylcyclohexyl)benzonitrile | 25 % |
| 4-(4-(4-Pentylcyclohexyl)phenylbenzonitrile | 15 % |

The clearing point of this nematic liquid crystal composition was 71.7 °C, the threshold voltage at a cell thickness of 9.2 µm was 1.78 V, the Δε was 11.0, and the Δn was 0.137. A liquid crystal composition (B2) comprising 90 % of this liquid crystal composition (A1) and 10 % of the compound of the present invention in Example 6 i.e. 5-propyl-2-(2-(4-(4-cyanophenyl)cyclohexyl)ethenyl)-1,3-dioxane (compound No. 383). The clearing point of this liquid crystal composition was 80.7 °C, the threshold voltage at a cell thickness of 9.2 µm was 1.71 V, the Δε was 12.3, and the Δn was 0.139. The physical values of the compound in Example 6, calculated by extrapolation from the mixing ratio of the above liquid crystal composition, indicated that the clearing point was 161.7 °C, the Δε was 24.0, and the Δn was 0.157.

### Example 11 (Application Example 3)

A liquid crystal composition (B4) comprising 85 % of the liquid crystal composition (A1) and 15 % of the compound of the present invention in Example 8 i.e. 5-(4-pentylcyclohexyl)-2-ethenyl-1,3-dioxane (compound No. 371). The clearing point of this liquid crystal composition was 69.5 °C, the threshold voltage at a cell thickness of 9.0 µm was 1.79 V, the Δε was 9.8, and the Δn was 0.125. The physical values of the compound in Example 9, calculated by extrapolation from the mixing ratio of the above liquid crystal composition, indicated that the clearing point was 57.0 °C, the Δε was 3.0, and the Δn was 0.057.

The compounds of the present invention are those having small Δn and large Δε, those having small Δn and high clearing points, those having small Δn and showing the nematic phase even at low temperature, or those having middle Δn and large Δε. For example, as shown in Example 9 (Application Example 1), Compound No. 31, while having very small Δn, has large Δε and a high clearing point. As shown in Example 10 (Application Example 2), Compound No. 383 has middle Δn and very large Δε. As shown in Example 11, Compound No. 371 has a wide temperature range of nematic phase at low temperature and is thus superior in miscibility at low temperature. Because the compounds of the present invention possess the preferable characteristics described above, these can be used as components in liquid crystal compositions, thus achieving characteristics not attainable in the prior art or providing new liquid crystal composition having the preferable characteristics.

### INDUSTRIAL APPLICABILITY

According to the present invention, there can be provided a liquid crystalline compound having small optical anisotropy (Δn) and large dielectric anisotropy (Δε), a liquid crystalline compound having small Δn and a high clearing point, a liquid crystalline compound having small Δn and exhibiting the nematic phase even at low temperature, or a liquid crystalline compound having middle Δn and large Δε, as well as a liquid crystal composition comprising the same, and a liquid crystal display device fabricated by using the same, and these can be used in various liquid crystal display units including TFT type.

## Claims

1. A liquid crystalline compound expressedd by the general formula (1): 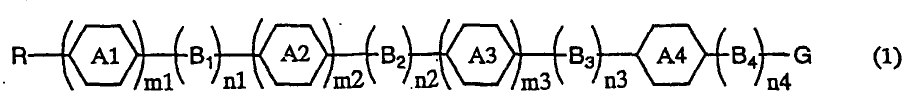 wherein R represents a hydrogen atom, a cyano group, a halogen atom, a C₁ to C₂₀ alkyl group or halogenated alkyl group, in which one or more -CH₂- groups may be replaced by an oxygen atom, a sulfur atom, -CH=CH-, -C≡C-, -CO-, -CF=CF-, -CF₂-, a C₃ to C₅ cycloalkane ring and cycloalkene ring, provided that the oxygen atom and the sulfur atom are not adjacent to each other,
B1, B2, B3 and B4 independently represent a covalent bond or a C₁ to C₄ alkylene group, in which one or more -CH₂- groups may be replaced by an oxygen atom, a sulfur atom, -CH=CH-, -C≡C-, -CO-, -CF=CF-, -CF₂-, a C₃ to C₅ cycloalkane ring and cycloalkene ring, provided that the oxygen atom and the sulfur atom are not adjacent to each other,
n1, n2, n3, n4, m1, m2 and m3 are independently 0 or 1,
rings A1, A2, A3 and A4 independently represent a benzene ring in which one to four hydrogen atoms may be replaced by halogen atoms, bicyclo[1.1.1]pentane ring, bicyclo[2.1.1]-hexane ring, bicyclo[2.2.1]heptane ring, bicyclo[2.2.2]octane ring, naphthalene ring, 1,2,3,4-tetrahydronaphthalene ring, perhydronaphthalene ring, fluorene ring, phenanthrene ring, 9,10-dihydrophenanthrene ring, indan ring, indene ring, pyridine ring, pyrimidine ring, C₄ to C₂₀ cycloalkane ring or cycloalkene ring, and -CH₂- in the rings may be replaced by an oxygen atom(s) or a sulfur atom(s),
G represents any of the groups expressed by formulae (G1) to (G3): 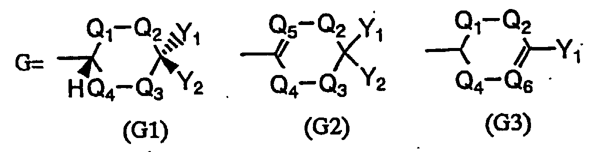
Q₁, Q₂, Q₃ and Q₄ are independently a covalent bond, an oxygen atom, a sulfur atom, -CH₂-, -CH₂CH₂-, -CH=CH-, -CO-, -CF=CF- or -CF₂-, whereupon the number of the covalent bonds is 2 or less and Q₁ and Q₄ are preferential, the oxygen atom and the sulfur atom are not adjacent to each other, and Q₅ and Q₆ are independently =CH-, =CF- or =CCl-,
Y₁ and Y₂ are independently a hydrogen atom, a halogen atom, a cyano group, a nitro group, an amino group, or a C₁ to C₂₀ alkyl group or halogenated alkyl group, in which one or more methylene groups may be replaced by an oxygen atom, a sulfur atom, -CH=CH-, -C≡C-, -CO-, -CF=CF-, -CF₂-, a C₃ to C₅ cycloalkane ring and cycloalkene ring, provided that the oxygen atom and the sulfur atom are not adjacent to each other,
when G is a group of the formula (G1), Y₁ and Y₂ are not simultaneously hydrogen atoms; when both Q₁ and Q₄ are oxygen atoms, both Q₂ and Q₃ are -CH₂-, and Y₂ is a hydrogen atom, then n4 is 1 and B4 is -CH=CH-; it is not a case that Q₂ and Q₃ are independently an oxygen atom or a sulfur atom, and Y₁ and Y₂ are a combination of a hydrogen atom and a saturated alkyl group, of a hydrogen atom and a trifluoromethyl group, or of a trifluoromethyl group and a trifluoromethyl group; and it is not a case that all Q₁ to Q₄ are -CH₂-,
when G is a group of the formula (G2), Y₂ is not a hydrogen atom insofar as all Q₂ to Q₄ are -CH₂- and Q₅ is =CH-,
when G is a group of the formula (G3), it is not a case that all Q₁, Q₂ and Q₄ are -CH₂-, and Q₆ is =CH-.

2. A liquid crystalline compound according to claim 1, wherein G is a group represented by the formula (G1).

3. A liquid crystalline compound according to claim 1, wherein G is a group represented by the formula (G2).

4. A liquid crystalline compound according to claim 1, wherein G is a group represented by the formula (G3).

5. A liquid crystalline compound according to claim 2, wherein Q₂ and Q₃ are independently an oxygen atom or a sulfur atom.

6. A liquid crystalline compound according to claim 5, wherein both Q₁ and Q₄ are -CH₂-.

7. A liquid crystalline compound according to claim 5, wherein Y₁ is a halogen atom or a cyano group.

8. A liquid crystalline compound according to claim 5, wherein Y₁ and Y₂ are independently a fluorine atom or a chlorine atom.

9. A liquid crystalline compound according to claim 5, wherein Y₁ is a C₁ to C₂₀ alkyl group in which one or more -CH₂- groups are replaced by -CH=CH-, -C≡C-, -CO-, -CF=CF- or CF₂, Y₂ is a hydrogen atom, and it is not a case that m3 is 1 and rings A3 and A4 are simultaneously cyclohexane rings.

10. A liquid crystalline compound according to claim 9, wherein Y₁ is a C₁ to C₂₀ alkyl group in which one or more -CH₂- groups are replaced by -CH=CH-.

11. A liquid crystalline compound according to claim 10, wherein Y₁ is -CH=CH₂.

12. A liquid crystal composition comprising at least one liquid crystalline compound according to any of claims 1 to 11.

13. A liquid crystal composition comprising as a first component at least one liquid crystalline compound according to any of claims 1 to 11 and as a second component at least one compound selected from the group of compounds expressed by the general formulae (2), (3) or (4): 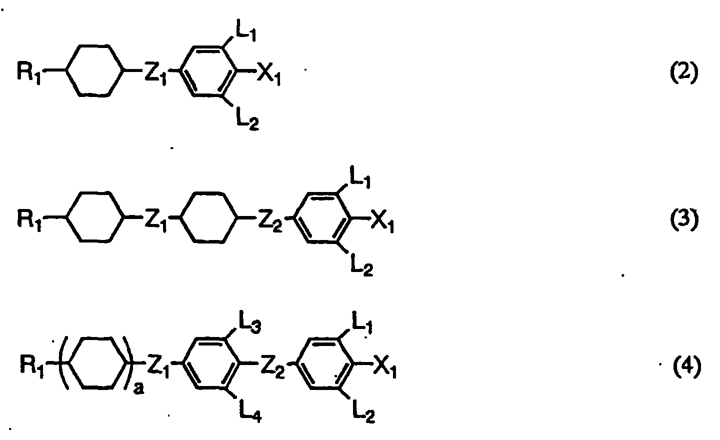 wherein R₁ represents a C₁ to C₁₀ alkyl group, X₁ represents F, Cl, -OCF₃, -OCF₂H, -CF₃, -CF₂H or -CFH₂, L₁, L₂, L₃ and L₄ independently one another represent H or F, Z₁ and Z₂ independently one another represent -(CH₂)₂-, -CH=CH- or a single bond, and a represents 1 or 2.

14. A liquid crystal composition comprising as a first component at least one liquid crystalline compound according to any of claims 1 to 11 and as a second component at least one compound selected from the group of compounds expressed by the general formulae (5), (6), (7), (8) or (9): 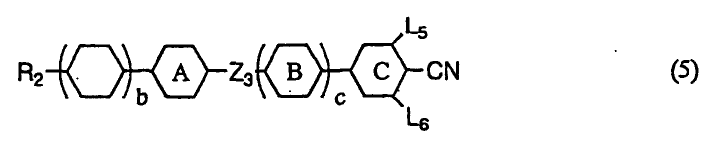 wherein R₂ represents F or a C₁ to C₁₀ alkyl group or C₂ to C₁₀ alkenyl group a nonadjacent and arbitrary methylene group(s) in the groups may be replaced by an oxygen atom(s), ring A represents a trans-1,4-cyclohexylene group, 1,4-phenylene group, or 1,3-dioxane-2,5-diyl group, ring B represents a trans-1,4-cyclohexylene group, 1,4-phenylene group, or pyrimidine-2,5-diyl group, ring C represents a trans-1,4-cyclohexylene group or 1,4-phenylene group, Z₃ represents - (CH₂)₂-, -COO- or a single bond, L₅ and L₆ independently represent H or F, and b and c independently represent 0 or 1. 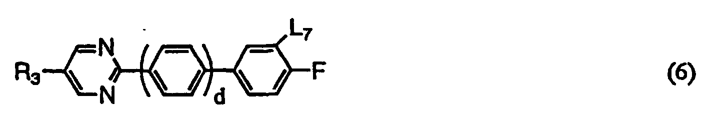 wherein R₃ represents a C₁ to C₁₀ alkyl group, L₇ represents H or F, and d represents 0 or 1. 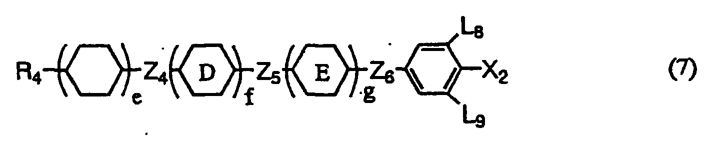 wherein R₄ represents a C₁ to C₁₀ alkyl group, rings D and E independently one another represent a trans-1,4-cyclohexylene group or 1,4-phenylene group, Z₄ and Z₅ independently one another represent -COO- or a single bond, Z₆ represents -COO-or -C≡C-, X₂ represents F, -OCF₃, -OCF₂H, -CF₃, -CF₂H or -CFH₂, and L₈ and L₉ independently one another represent H or F, and e, f and g independently one another represent 0 or 1. 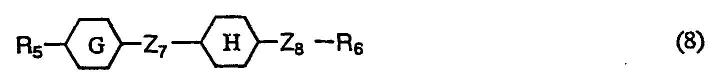 wherein R₅ and R₆ independently one another represent a C₁ to C₁₀ alkyl group or C₂ to C₁₀ alkenyl group a nonadjacent and arbitrary methylene group(s) in the groups may be replaced by an oxygen atom(s), ring G represents a trans-1,4-cyclohexylene group, 1,4-phenylene group, or pyrimidine-2,5-diyl group, ring H represents a trans-1,4-cyclohexylene group or 1,4-phenylene group, Z₇ represents -C≡C-, -COO-, -(CH₂)₂-, -CH=CH-C≡C- or a single bond, and Z₈ represents -COO- or a single bond, 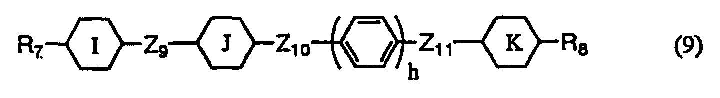 wherein R₇ and R₈ independently one another represent a C₁ to C₁₀ alkyl group or C₂ to C₁₀ alkenyl group a nonadjacent arbitrary methylene group(s) in the groups may be replaced by an oxygen atom(s), ring I represents a trans-1,4-cyclohexylene group, 1,4-phenylene group, or pyrimidine-2,5-diyl group, ring J represents a trans-1,4-cyclohexylene group, a 1,4-phenylene group in which one or more hydrogen atoms may be replaced by F, or a pyrimidine-2,5-diyl group, ring K represents a trans-1,4-cyclohexylene group or 1,4-phenylene group, Z₉ and Z₁₁ independently represent -COO-, -(CH₂)₂- or a single bond, Z₁₀ represents -CH=CH-, -C≡C-, -COO- or a single bond, and h represents 0 or 1.

15. A liquid crystal composition comprising as a first component at least one liquid crystalline compound according to any of claims 1 to 11 and as a part of a second component at least one compound selected from the group consisting of the general formulae (2), (3) and (4) and as the other part of the second component at least one compound selected from the group consisting of the general formulae (5), (6), (7), (8) and (9).

16. A liquid crystal display device fabricated by using a liquid crystal composition according to any of claims 12 to 15.
